# EUROPEAN PATENT APPLICATION

(11) **EP 4 559 911 A1**
(43) Date of publication of application: **28.05.2025**
(21) Application number: 23843443.5
(22) Date of filing: 21.07.2023
(51) Int. Cl.: C07D 413/14, A61K 31/506, A61P 29/00, A61P 37/00, A61P 35/00, A61P 7/02, C07D 403/14, A61K 31/4439, A61K 31/397, A61K 31/497

(54) **NOVEL HETEROCYCLIC COMPOUND AND PHARMACEUTICAL COMPOSITION FOR INHIBITING AUTOTAXIN COMPRISING THE SAME**

(30) Priority: 22.07.2022 KR 20220091039; 10.03.2023 KR 20230031868
(71) Applicant: Nextgen Bioscience Co., Ltd., Seongnam-si, Gyeonggi-do 13487 (KR)
(72) Inventor: LEE, Bong Yong, Seoul 06625 (KR); SHIN, Young Ah, Seongnam-si, Gyeonggi-do 13639 (KR); KIM, Shin Ae, Suwon-si, Gyeonggi-do 16507 (KR); HAN, Na Ra, Yongin-si, Gyeonggi-do 17064 (KR); NOH, Hyeon Seok, Seongnam-si, Gyeonggi-do 13292 (KR); SHIN, Soo Jeong, Yongin-si, Gyeonggi-do 16944 (KR); LEE, Jae Sang, Yongin-si, Gyeonggi-do 16839 (KR); LEE, Jae Un, Seongnam-si, Gyeonggi-do 13505 (KR); CHO, Young Kyoung, Suwon-si, Gyeonggi-do 16544 (KR)
(74) Representative: Graf von Stosch Patentanwaltsgesellschaft mbH
(86) International application number: PCT/KR2023/010621
(87) International publication number: WO 2024/019597

(57) **Abstract**

The present invention provides a novel heterocyclic compound, a hydrate thereof, a solvate thereof or a pharmaceutically acceptable salt thereof, and a pharmaceutical composition comprising the same as an active ingredient for preventing or treating a disease associated with autotaxin activity.

The heterocyclic compound of the present invention exhibits excellent inhibitory activity against autotaxin, and can be usefully used in the treatment and prevention of diseases associated with the inhibition of autotaxin, such as fibrotic diseases, inflammatory diseases, autoimmune diseases, respiratory diseases, cardiovascular diseases, metabolic diseases, cancer and cancer metastasis, ocular diseases, cholestatic forms and other forms of chronic pruritus, and acute or chronic organ transplant rejection.

## Description

### FIELD

The present invention relates to heterocyclic compounds, and more specifically, to novel heterocyclic compounds and pharmaceutical compositions comprising the same for autotaxin inhibition.

### BACKGROUND

Autotaxin (ATX), also known as ectonucleotide pyrophosphatase/phosphodiesterase family member 2 (ENPP2), is a secreted enzyme essential for the production of the lipid signaling molecule lysophosphatidic acid (LPA). Autotaxin exhibits lysophospholipase D activity, which converts lysophosphatidylcholine (LPC) to LPA. Therefore, LPA levels in plasma and ascites are correlated with ATX activity.

Plasma LPA is a bioactive lipid that influences the migration, proliferation, and survival of various cell types. In addition, the ATX-LPA signaling process is involved in the physiological and pathophysiological functions of various diseases, including nervous system function, vascular development, cardiovascular physiology, tissue regeneration, immune system function, chronic inflammation, tumor metastasis and progression, organ fibrosis, and obesity and/or other metabolic diseases (e.g., diabetes mellitus).

Therefore, increased ATX activity and increased LPA levels, altered LPA receptor expressions, and altered responses to LPA may be associated with the initiation, progression, and/or outcome of various pathophysiological diseases involving the ATX/LPA signaling pathway. In particular, it is known to be associated with cancer, lymphocyte homing, chronic inflammation, neuropathic pain, fibrotic diseases (e.g., idiopathic pulmonary fibrosis, IPF), and thrombosis. Accordingly, lowering the levels of LPA and/or autotaxin (ATX), which induces it, is necessary to treat these diseases.

### OBJECT OF THE INVENTION

The problem to be addressed by the present invention is provide novel structured autotaxin inhibitory compounds that exhibit excellent inhibitory activity against autotaxin.

Further, the problem to be addressed by the present invention is to provide a pharmaceutical composition for inhibiting autotaxin comprising an autotaxin inhibitory compound having the novel structure.

Further, the problem to be addressed by the present invention is to provide a method for inhibiting autotaxin and treating and preventing diseases caused by it using the autotaxin inhibitory compound having the novel structure.

Further, the problem to be addressed by the present invention is to provide a use of the novel structure of the autotaxin inhibitory compound for inhibiting autotaxin and treating diseases accordingly.

The problems to be addressed by the present invention are not limited to the problems mentioned above, and other technical problems not mentioned can be clearly understood by a person having ordinary skill in the technical field to which the present invention belongs from the description below.

### SUMMARY

In order to address the above problem, according to one aspect of the present invention, there is provided a heterocyclic compound represented by the following Formula 1, a hydrate thereof, a solvate thereof or a pharmaceutically acceptable salt thereof, wherein: 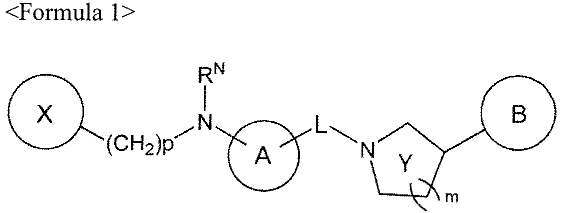
X is aryl; a fused bicyclic ring in which an aryl ring is fused with a non-aromatic cycloalkyl ring; a fused bicyclic ring in which an aryl ring is fused with a non-aromatic heterocyclic ring having 1 to 3 of O; a fused bicyclic ring in which a heteroaryl ring having 1 to 3 of N is fused with a non-aromatic cycloalkyl ring, wherein X is substituted with one or more independent R^{x} or not,
R^{x} is C₁₋₄ alkoxy or halogen,
p is an integer from 0 to 2,
R^{N} is hydrogen or C₁₋₄ alkyl,
A is 5- or 6-membered heteroaryl ring having 1 to 3 heteroatom(s) selected from the group consisting of N and O, wherein A is substituted with R^{a} or not,
R^{a} is C₁₋₄ alkyl,
L is -(CH₂)₁₋₅CO-; -(CHCH)₁₋₂CO-; or a 5-membered aromatic or non-aromatic heterocycle having 1 to 3 heteroatoms selected from the group consisting of N and O,
Y ring is substituted or unsubstituted with R^{y},
R^{y} is C₁₋₄ alkyl or halogen,
m is 0 or 1,
B is cyano; OH; COOH; CH₂COOH; sulfonyl; sulfonate(-O-SO₂-); or a 5- to 6-membered aromatic or non-aromatic heterocycle ring having 1 to 4 heteroatoms selected from the group consisting of N and O, wherein B is substituted or unsubstituted with R^{b} or oxo(O),
wherein R^{b} is C₁₋₄ alkyl, C₁₋₄ haloalkyl, amino, or C₁₋₄ alkyl pivalate.

According to another aspect of the present invention, there is provided a pharmaceutical composition for preventing or treating a disease associated with autotaxin activity, comprising the heterocyclic compound, a hydrate thereof, a solvate thereof or a pharmaceutically acceptable salt thereof as an active ingredient.

According to another aspect of the present invention, there is provided a method for inhibiting autotaxin and preventing or treating a disease resulting therefrom, using the heterocyclic compound, a hydrate thereof, a solvate thereof or a pharmaceutically acceptable salt thereof.

According to another aspect of the present invention, there is provided a use of the heterocyclic compound, a hydrate thereof, a solvate thereof or a pharmaceutically acceptable salt thereof for inhibiting autotaxin and preventing or treating a disease resulting therefrom.

According to another aspect of the present invention, there is provided a pharmaceutical composition comprising the heterocyclic compound, a hydrate thereof, a solvate thereof, or a pharmaceutically acceptable salt thereof, and a pharmaceutically acceptable additive.

It was found that the novel heterocyclic compound of the present invention exhibits excellent inhibitory activity against autotaxin.

Therefore, the novel heterocyclic compound of the present invention can be usefully used in the treatment and prevention of diseases associated with the inhibition of autotaxin, such as fibrotic diseases, inflammatory diseases, autoimmune diseases, respiratory diseases, cardiovascular diseases, metabolic diseases, cancer and cancer metastasis, ocular diseases, cholestatic forms and other forms of chronic pruritus, and acute or chronic organ transplant rejection.

The effects of the present invention are not limited to the above effects, but should be understood to include all effects that can be inferred from the description of the invention or the composition of the invention as recited in the patent claims.

### DETAILED DESCRIPTION OF THE INVENTION

In this specification, autotaxin (ATX) is a secreted enzyme that plays an important role in the production of lysophosphatidic acid (LPA), also referred to as ectonucleotide pyrophosphatase/phosphodiesterase family member 2 (ENPP2). Autotaxin exhibits lysophospholipase D activity, which converts lysophosphatidylcholine (LPC) to LPA. Therefore, LPA levels in plasma and ascites are associated with ATX activity.

The present invention provides a heterocyclic compound represented by the following Formula 1, a hydrate thereof, a solvate thereof or a pharmaceutically acceptable salt thereof, wherein: 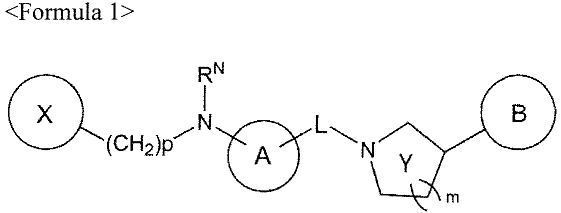
X is aryl; a fused bicyclic ring in which an aryl ring is fused with a non-aromatic cycloalkyl ring; a fused bicyclic ring in which an aryl ring is fused with a non-aromatic heterocyclic ring having 1 to 3 of O; a fused bicyclic ring in which a heteroaryl ring having 1 to 3 of N is fused with a non-aromatic cycloalkyl ring, wherein X is substituted with one or more independent R^{x} or not,
R^{x} is C₁₋₄ alkoxy or halogen,
p is an integer from 0 to 2,
R^{N} is hydrogen or C₁₋₄ alkyl,
A is 5- or 6-membered heteroaryl ring having 1 to 3 heteroatom(s) selected from the group consisting of N and O, wherein A is substituted with R^{a} or not,
R^{a} is C₁₋₄ alkyl,
L is -(CH₂)₁₋₅CO-; -(CHCH)₁₋₂CO-; or a 5-membered aromatic or non-aromatic heterocycle having 1 to 3 heteroatoms selected from the group consisting of N and O,
Y ring is substituted or unsubstituted with R^{y},
R^{y} is C₁₋₄ alkyl or halogen,
m is 0 or 1,
B is cyano; OH; COOH; CH₂COOH; sulfonyl; sulfonate(-O-SO₂-); or a 5- to 6-membered aromatic or non-aromatic heterocycle ring having 1 to 4 heteroatoms selected from the group consisting of N and O, wherein B is substituted or unsubstituted with R^{b} or oxo(O),
wherein R^{b} is C₁₋₄ alkyl, C₁₋₄ haloalkyl, amino, or C₁₋₄ alkyl pivalate.

In one embodiment, X may be one selected from the group consisting of phenyl, dihydroindenyl, benzodioxolyl, dihydro-cyclopentapyrazinyl, and dihydro-cyclopentapyridinyl.

In one embodiment, R^{x} may be one selected from the group consisting of methoxy, F, Cl, and Br.

In one embodiment, R^{N} may be hydrogen or methyl.

In one embodiment, A may be one selected from the group consisting of pyridine, pyrimidine, pyrazine, and oxadiazole.

In one embodiment, R^{a} may be methyl.

In one embodiment, L may be one selected from the group consisting of -(CH₂)₂CO-, -(CH₂)₃CO-, -(CH)₂CO-, dihydroisoxazolyl, isoxazolyl, and oxadiazolyl.

In one embodiment, R^{y} may be methyl or F.

In one embodiment, B may be one selected from the group consisting of OH, cyano, carboxyl, carboxymethyl, sulfonyl, sulfonate, imidazolyl, triazolyl, tetrazolyl, oxadiazolyl, oxadiazolone, and morpholino.

In one embodiment, R^{b} may be one selected from the group consisting of methyl, difluoromethyl, trifluoromethyl, amino, and methylpivalate.

Representative examples of heterocyclic compounds according to the present invention are as follows:
[1] 5-(5-(3-(1H-1,2,3-triazol-4-yl)pyrrolidin-1-yl)-1,3,4-oxadiazol-2-yl)-N-(2,3-dihydro-1H-inden-2-yl)pyrimidin-2-amine,
[2] (4-(1-(5-(2-((5,6-difluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate,
[3] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrimidin-2-amine,
[4] (4-(1-(5-(2-((6,7-dihydro-5H-cyclopenta[b]pyridin-6-yl)amino)pyrimidin-5-yl)-1,3,4-oxadiazol-2-yl)pyrrolidin-3-yl)-1H-1,2,3-triazol-1-yl)methyl pivalate,
*[5]* (4-(1-(5-(2-((benzo[d][1,3]dioxol-5-ylmethyl)amino)pyrimidin-5-yl)-1,3,4-oxadiazol-2-yl)pyrrolidin-3-yl)-1H-1,2,3-triazol-1-yl)methyl pivalate,
[6] 5-(5-(3-(1H-1,2,3-triazol-4-yl)pyrrolidin-1-yl)-1,3,4-oxadiazol-2-yl)-N-(benzo[d][1,3]dioxol-5-ylmethyl)pyrimidin-2-amine,
[7] N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyrazin-6-amine,
[8] N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyridin-6-amine,
[9] (E)-1-(3-(1H-1,2,3-triazol-4-yl)pyrrolidin-1-yl)-3-(2-((5,6-difluoro-2,3-dihydro-1H-inden-2-yl)amino)pyrimidin-5-yl)prop-2-en-1-one,
[10] 1-(3-(1H-1,2,3-triazol-4-yl)pyrrolidin-1-yl)-3-(2-((5,6-difluoro-2,3-dihydro-1H-inden-2-yl)amino)pyrimidin-5-yl)propan-1-one,
[11] 5-(5-(3-(1H-1,2,3-triazol-4-yl)pyrrolidin-1-yl)-1,3,4-oxadiazol-2-yl)-N-(3,5-dichlorophenethyl)pyrimidin-2-amine,
[12] (4-(1-(5-(2-((5-methoxy-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate,
[13] 5-(5-(3-(1H-1,2,3-triazol-4-yl)pyrrolidin-1-yl)-1,3,4-oxadiazol-2-yl)-N-(5-methoxy-2,3-dihydro-1H-inden-2-yl)pyrimidin-2-amine,
[14] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrazin-2-amine,
[15] methyl 1-(5-(2-((5,6-difluoro-2,3-dihydro-1H-inden-2-yl)amino)pyrimidin-5-yl)-1,3,4-oxadiazol-2-yl)pyrrolidine-3-carboxylate,
[16] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)-4-methylpyrimidin-2-amine,
[17] 5-(3-(3-(1H-1,2,3-triazol-4-yl)pyrrolidin-1-yl)-4,5-dihydroisoxazol-5-yl)-N-(5,6-difluoro-2,3-dihydro-1H-inden-2-yl)pyrimidin-2-amine,
[18] 5-(3-(3-(1H-1,2,3-triazol-4-yl)pyrrolidin-1-yl)isoxazol-5-yl)-N-(5,6-difluoro-2,3-dihydro-1H-inden-2-yl)pyrimidin-2-amine,
[19] 5-(5-(3-(1H-1,2,3-triazol-4-yl)pyrrolidin-1-yl)-1,3,4-oxadiazol-2-yl)-N-(5-bromo-2,3-dihydro-1H-inden-2-yl)pyrimidin-2-amine,
[20] (E)-1-(3-(1H-1,2,3-triazol-4-yl)pyrrolidin-1-yl)-3-(2-((2,3-dihydro-1H-inden-2-yl)amino)pyrimidin-5-yl)prop-2-en-1-one,
[21] 5-(5-(3-(1H-1,2,3-triazol-4-yl)pyrrolidin-1-yl)-1,3,4-oxadiazol-2-yl)-N-(3,5-difluorobenzyl)pyrimidin-2-amine,
[22] 1-(5-(2-((5,6-difluoro-2,3-dihydro-1H-inden-2-yl)amino)pyrimidin-5-yl)-1,3,4-oxadiazol-2-yl)pyrrolidine-3-carboxylic acid,
[23] 5-(1-(5-(2-((5,6-difluoro-2,3-dihydro-1H-inden-2-yl)amino)pyrimidin-5-yl)-1,3,4-oxadiazol-2-yl)pyrrolidin-3-yl)-1,3,4-oxadiazol-2(3H)-one,
[24] methyl 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetate,
[25] 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetic acid,
[26] 1-(5-(2-((5,6-difluoro-2,3-dihydro-1H-inden-2-yl)amino)pyrimidin-5-yl)-1,3,4-oxadiazol-2-yl)pyrrolidin-3-ol,
[27] 1-(5-(2-((5,6-difluoro-2,3-dihydro-1H-inden-2-yl)amino)pyrimidin-5-yl)-1,3,4-oxadiazol-2-yl)pyrrolidin-3-yl methanesulfonate,
[28] 5-(5-(3-(1H-1,2,4-triazol-1-yl)pyrrolidin-1-yl)-1,3,4-oxadiazol-2-yl)-N-(5,6-difluoro-2,3-dihydro-1H-inden-2-yl)pyrimidin-2-amine,
[29] N-(5,6-difluoro-2,3-dihydro-1H-inden-2-yl)-5-(5-(3-(3-methyl-1H-1,2,4-triazol-1-yl)pyrrolidin-1-yl)-1,3,4-oxadiazol-2-yl)pyrimidin-2-amine,
[30] N-(5,6-difluoro-2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine,
[31] N-(2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine,
[32] N-(3,5-dichlorophenethyl)-5-(5-(3-methyl-3-(1H-1,2,3-triazol-4-yl)pyrrolidin-1-yl)-1,3,4-oxadiazol-2-yl)pyrimidin-2-amine,
[33] N-(3,5-difluorobenzyl)-5-(5-(3-methyl-3-(1H-1,2,3-triazol-4-yl)pyrrolidin-1-yl)-1,3,4-oxadiazol-2-yl)pyrimidin-2-amine,
[34] (E)-3-(2-((2,3-dihydro-1H-inden-2-yl)amino)pyrimidin-5-yl)-1-(3-methyl-3-(1H-1,2,3-triazol-4-yl)pyrrolidin-1-yl)prop-2-en-1-one,
[35] N-(5,6-difluoro-2,3-dihydro-1H-inden-2-yl)-5-(5-(3-(1-methyl-1H-1,2,3-triazol-4-yl)pyrrolidin-1-yl)-1,3,4-oxadiazol-2-yl)pyrimidin-2-amine,
[36] 5-(5-(3-(1H-1,2,3-triazol-4-yl)pyrrolidin-1-yl)-1,3,4-oxadiazol-2-yl)-N-(5-fluoro-2,3-dihydro-1H-inden-2-yl)pyrimidin-2-amine,
[37] 5-(5-(3-(1H-1,2,3-triazol-4-yl)pyrrolidin-1-yl)-1,3,4-oxadiazol-2-yl)-N-(5-chloro-2,3-dihydro-1H-inden-2-yl)pyrimidin-2-amine,
[38] 5-(5-(3-(1H-imidazol-4-yl)pyrrolidin-1-yl)-1,3,4-oxadiazol-2-yl)-N-(5,6-difluoro-2,3-dihydro-1H-inden-2-yl)pyrimidin-2-amine,
[39] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrimidin-2-amine,
[40] (E)-1-(3-(1H-1,2,3-triazol-4-yl)azetidin-1-yl)-3-(2-((5,6-difluoro-2,3-dihydro-1H-inden-2-yl)amino)pyrimidin-5-yl)prop-2-en-1-one,
[41] N-(5,6-difluoro-2,3-dihydro-1H-inden-2-yl)-5-(5-(3-(1-methyl-1H-1,2,3-triazol-5-yl)azetidin-1-yl)-1,3,4-oxadiazol-2-yl)pyrimidin-2-amine,
[42] 5-(5-(3-(1H-1,2,3-triazol-4-yl)azetidin-1-yl)-1,3,4-oxadiazol-2-yl)-N-(2,3-dihydro-1H-inden-2-yl)pyrimidin-2-amine,
[43] 5-(3-(3-(1H-1,2,3-triazol-4-yl)azetidin-1-yl)-4,5-dihydroisoxazol-5-yl)-N-(5,6-difluoro-2,3-dihydro-1H-inden-2-yl)pyrimidin-2-amine,
[44] 1-(3-(1H-1,2,3-triazol-4-yl)azetidin-1-yl)-3-(2-((5,6-difluoro-2,3-dihydro-1H-inden-2-yl)amino)pyrimidin-5-yl)propan-1-one,
[45] 5-(3-(3-(1H-1,2,3-triazol-4-yl)azetidin-1-yl)isoxazol-5-yl)-N-(5,6-difluoro-2,3-dihydro-1H-inden-2-yl)pyrimidin-2-amine,
[46] 1-(5-(2-((5,6-difluoro-2,3-dihydro-1H-inden-2-yl)amino)pyrimidin-5-yl)-1,3,4-oxadiazol-2-yl)azetidine-3-sulfonamide,
[47] methyl 1-(5-(2-((5,6-difluoro-2,3-dihydro-1H-inden-2-yl)amino)pyrimidin-5-yl)-1,3,4-oxadiazol-2-yl)azetidine-3-carboxylate,
[48] 5-(5-(3-(1H-1,2,3-triazol-4-yl)azetidin-1-yl)-1,3,4-oxadiazol-2-yl)-N-(3,5-difluorobenzyl)pyrimidin-2-amine,
[49] 1-(5-(2-((5,6-difluoro-2,3-dihydro-1H-inden-2-yl)amino)pyrimidin-5-yl)-1,3,4-oxadiazol-2-yl)azetidin-3-ol,
[50] 1-(5-(2-((5,6-difluoro-2,3-dihydro-1H-inden-2-yl)amino)pyrimidin-5-yl)-1,3,4-oxadiazol-2-yl)azetidine-3-carboxylic acid,
[51] N-(5,6-difluoro-2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine,
[52] methyl 2-(1-(5-(2-((5,6-difluoro-2,3-dihydro-1H-inden-2-yl)amino)pyrimidin-5-yl)-1,3,4-oxadiazol-2-yl)azetidin-3-yl)acetate,
[53] 2-(1-(5-(2-((5,6-difluoro-2,3-dihydro-1H-inden-2-yl)amino)pyrimidin-5-yl)-1,3,4-oxadiazol-2-yl)azetidin-3-yl)acetic acid,
[54] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyridin-2-amine,
[55] 1-(5-(2-((5,6-difluoro-2,3-dihydro-1H-inden-2-yl)amino)pyrimidin-5-yl)-1,3,4-oxadiazol-2-yl)azetidin-3-yl methanesulfonate,
[56] N-(5,6-difluoro-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[57] 1-(5-(2-((5,6-difluoro-2,3-dihydro-1H-inden-2-yl)amino)pyrimidin-5-yl)-1,3,4-oxadiazol-2-yl)azetidine-3-carbonitrile,
[58] N-(5,6-difluoro-2,3-dihydro-1H-inden-2-yl)-5-(5-(3-(5-(trifluoromethyl)-1,3,4-oxadiazol-2-yl)azetidin-1-yl)-1,3,4-oxadiazol-2-yl)pyrimidin-2-amine,
[59] 5-(5-(3-(1H-1,2,3-triazol-4-yl)azetidin-1-yl)-1,3,4-oxadiazol-2-yl)-N-(3,5-dichlorophenethyl)pyrimidin-2-amine,
[60] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrazin-2-amine,
[61] N-(5,6-difluoro-2,3-dihydro-1H-inden-2-yl)-5-(5-(3-(5-(difluoromethyl)-1,3,4-oxadiazol-2-yl)azetidin-1-yl)-1,3,4-oxadiazol-2-yl)pyrimidin-2-amine,
[62] (4-(1-(5-(2-((5-bromo-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[63] (4-(1-(5-(2-((5-fluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[64] 5-(5-(3-(1H-1,2,3-triazol-4-yl)azetidin-1-yl)-1,3,4-oxadiazol-2-yl)-N-(5-bromo-2,3-dihydro-1H-inden-2-yl)pyrimidin-2-amine,
[65] 5-(5-(3-(1H-1,2,3-triazol-4-yl)azetidin-1-yl)-1,3,4-oxadiazol-2-yl)-N-(5-fluoro-2,3-dihydro-1H-inden-2-yl)pyrimidin-2-amine,
[66] 5-(1-(5-(2-((5,6-difluoro-2,3-dihydro-1H-inden-2-yl)amino)pyrimidin-5-yl)-1,3,4-oxadiazol-2-yl)azetidin-3-yl)-1,3,4-oxadiazol-2(3H)-one,
[67] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-4-methylpyrimidin-2-amine,
[68] 5-(5-(3-(1H-tetrazol-5-yl)azetidin-1-yl)-1,3,4-oxadiazol-2-yl)-N-(5,6-difluoro-2,3-dihydro-1H-inden-2-yl)pyrimidin-2-amine,
[69] N-(2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine,
[70] (4-(1-(5-(2-((5-methoxy-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[71] 5-(5-(3-(1H-1,2,3-triazol-4-yl)azetidin-1-yl)-1,3,4-oxadiazol-2-yl)-N-(5-methoxy-2,3-dihydro-1H-inden-2-yl)pyrimidin-2-amine,
[72] (4-(1-(5-(2-((6,7-dihydro-5H-cyclopenta[b]pyrazin-6-yl)amino)pyrimidin-5-yl)-1,3,4-oxadiazol-2-yl)azetidin-3-yl)-1H-1,2,3-triazol-1-yl)methyl pivalate,
[73] (4-(1-(5-(2-((6,7-dihydro-5H-cyclopenta[b]pyridin-6-yl)amino)pyrimidin-5-yl)-1,3,4-oxadiazol-2-yl)azetidin-3-yl)-1H-1,2,3-triazol-1-yl)methyl pivalate,
[74] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyrazin-6-amine,
[75] N-(5,6-difluoro-2,3-dihydro-1H-inden-2-yl)-5-(5-(3-(5-methyl-1H-1,2,3-triazol-4-yl)azetidin-1-yl)-1,3,4-oxadiazol-2-yl)pyrimidin-2-amine,
[76] 5-(5-(3-(1H-imidazol-4-yl)azetidin-1-yl)-1,3,4-oxadiazol-2-yl)-N-(5,6-difluoro-2,3-dihydro-1H-inden-2-yl)pyrimidin-2-amine,
[77] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyridin-6-amine,
[78] (E)-3-(2-((5,6-difluoro-2,3-dihydro-1H-inden-2-yl)amino)pyrimidin-5-yl)-1-(3-methyl-3-(1H-1,2,3-triazol-4-yl)azetidin-1-yl)prop-2-en-1-one,
[79] 5-(5-(3-(1H-1,2,4-triazol-1-yl)azetidin-1-yl)-1,3,4-oxadiazol-2-yl)-N-(5,6-difluoro-2,3-dihydro-1H-inden-2-yl)pyrimidin-2-amine,
[80] N-(5,6-difluoro-2,3-dihydro-1H-inden-2-yl)-5-(5-(3-(3-methyl-1H-1,2,4-triazol-1-yl)azetidin-1-yl)-1,3,4-oxadiazol-2-yl)pyrimidin-2-amine,
[81] N-(5,6-difluoro-2,3-dihydro-1H-inden-2-yl)-5-(5-(3-morpholinoazetidin-1-yl)-1,3,4-oxadiazol-2-yl)pyrimidin-2-amine,
[82] (E)-3-(2-((2,3-dihydro-1H-inden-2-yl)amino)pyrimidin-5-yl)-1-(3-methyl-3-(1H-1,2,3-triazol-4-yl)azetidin-1-yl)prop-2-en-1-one,
[83] N-(2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[84] (4-(1-(5-(2-((6,7-dihydro-5H-cyclopenta[c]pyridin-6-yl)amino)pyrimidin-5-yl)-1,3,4-oxadiazol-2-yl)azetidin-3-yl)-1H-1,2,3-triazol-1-yl)methyl pivalate,
[85] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[c]pyridin-6-amine,
[86] (4-(1-(5-(2-((benzo[d][1,3]dioxol-5-ylmethyl)amino)pyrimidin-5-yl)-1,3,4-oxadiazol-2-yl)azetidin-3-yl)-1H-1,2,3-triazol-1-yl)methyl pivalate,
[87] 5-(5-(3-(1H-1,2,3-triazol-4-yl)azetidin-1-yl)-1,3,4-oxadiazol-2-yl)-N-(benzo[d][1,3]dioxol-5-ylmethyl)pyrimidin-2-amine,
[88] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-N-methylpyrimidin-2-amine,
[89] N-(5-bromo-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[90] 1-(3-(1H-1,2,3-triazol-4-yl)azetidin-1-yl)-4-(5-((5,6-difluoro-2,3-dihydro-1H-inden-2-yl)amino)-1,3,4-oxadiazol-2-yl)butan-1-one,
[91] 5-(5-(3-(1H-1,2,3-triazol-4-yl)azetidin-1-yl)-1,3,4-oxadiazol-2-yl)-N-(5-chloro-2,3-dihydro-1H-inden-2-yl)pyrimidin-2-amine,
[92] 5-(5-(3-(1H-1,2,3-triazol-4-yl)pyrrolidin-1-yl)-1,3,4-oxadiazol-2-yl)-N-(4-chlorophenethyl)pyrimidin-2-amine, and
[93] 5-(5-(3-(1H-1,2,3-triazol-4-yl)azetidin-1-yl)-1,3,4-oxadiazol-2-yl)-N-(4-chlorophenethyl)pyrimidin-2-amine.

This specification uses the following definitions when defining the compound of Formula 1 unless specifically defined.

The term "alkyl" refers to a straight-chain or branched-chain saturated hydrocarbon, preferably C ₁₋₁₀ alkyl. Examples of alkyl include, but are not limited to, methyl, ethyl, *n*-propyl, *iso*-propyl, *n*-butyl, *iso*-butyl, *tert*-butyl, *n*-pentyl, *iso*-pentyl, *n*-hexyl, 3-methylhexyl, 2,2-dimethylpentyl, 2,3-dimethylpentyl, *n*-heptyl, *n*-octyl, *n*-nonyl, *n*-decyl, *n*-undecyl and *n-*dodecyl, etc..

The term "alkylene" refers to a divalent functional group derived from an alkyl group, preferably having from 1 to 10 carbon atoms, but is not limited thereto. Examples of alkylene include, but are not limited to -CH₂-, -CH₂CH₂-, -CH₂CH₂CH₂-, -CH₂CH₂CH₂CH₂- and - CH₂CH₂CH₂CH₂CH₂-.

The term "cycloalkyl" refers to a partially or fully saturated single or fused ring hydrocarbon, preferably C₃₋₁₀ cycloalkyl. Examples include, but are not limited to, cyclopropyl, cyclobutyl, cyclopentyl, cyclohexyl, cyclohexynyl, and the like.

The term "hydroxy" or "hydroxyl" is defined as -OH, and the term "alkoxy", unless otherwise defined, means alkyloxy, a radical in which the hydrogen atoms of the hydroxy group are replaced by 1 to 10 alkyl atoms.

The term "halogen" or "halo" refers to fluorine (F), chlorine (Cl), bromine (Br), or iodine (I).

The terms "haloalkyl" and "haloalkoxy" mean alkyl or alkoxy substituted with one or more halogen atoms.

The term "heteroatom" means N, O, or S.

The term "aryl" means an aromatic hydrocarbon, including a polycyclic aromatic ring system in which a carbocyclic aromatic ring or a heteroaryl ring is fused with one or more other rings. Preferably it is C₅₋₁₂ aryl, more preferably C₅₋₁₀ aryl. For example, the aryl includes, but is not limited to, phenyl, naphthyl, tetrahydronaphthyl, and the like.

The term "heteroaryl" or "aromatic heterocycle" means a 3- to 12-membered, more preferably 5- to 10-membered aromatic hydrocarbon forming a single or fused ring that contains one or more heteroatoms selected from N, O and S as ring atoms, and that can be fused with a benzo or C₃-₈ cycloalkyl. For example, heteroaryl includes, but are not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiazolyl, benzooxazolyl, benzimidazolyl, quinolinyl, isoquinolinyl, etc.. Additionally, the heteroaryl includes groups in which the heteroaryl ring is fused to a cycloalkyl or a non-aromatic heterocyclic ring, for example, dihydrocyclopentapyrazinyl, and the like.

A non-aromatic heterocyclic ring is a non-aromatic carbocyclic ring containing one or more heteroatoms, such as nitrogen, oxygen or sulfur, within the ring. The ring may be 5, 6, 7 or 8-membered and/or may be fused to another ring, such as a cycloalkyl or an aromatic ring. Examples of such compounds include 3-1H-benzimidazol-2-one, 3-1-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoic acid, benzotriazol-1-yl, benzopyrrolidine, benzopiperidine, benzoxolane, benzothiolane and benzothiane, etc..

Arylalkyl, alkylaryl and heteroarylalkyl refer to groups formed by combining aryl and alkyl or heteroaryl and alkyl as defined above, and examples thereof include, but are not limited to, benzyl, thiophenemethyl, and pyrimidinemethyl.

The compound represented by the chemical formula 1 according to the present invention can be made and used in the form of a prodrug, a hydrate, a solvate, and a pharmaceutically acceptable salt in order to promote *in vivo* absorption or increase solubility, and therefore the prodrug, hydrate, solvate, and pharmaceutically acceptable salt also fall within the scope of the present invention. Furthermore, the compound represented by the chemical formula 1 has a chiral carbon, and thus its stereoisomers exist, and such stereoisomers are also included within the scope of the present invention.

The term "prodrug" refers to a substance that is transformed *in vivo* into the parent drug. Prodrugs are often used because, in some cases, they are easier to administer than the parent drug. For example, they may be bioactive by oral administration, whereas the parent drug may not be. Prodrugs may also have improved solubility in pharmaceutical compositions than the parent drug. For example, prodrugs may be *in vivo* hydrolysable esters of compounds according to the invention and pharmaceutically acceptable salts thereof. Another example of a prodrug may be a short peptide (polyamino acid) that is linked to an acid group that is metabolized to reveal the active site of the peptide.

The term "hydrate" refers to a compound of the present invention or a salt thereof having a stoichiometric or non-stoichiometric amount of water bound by non-covalent intermolecular forces.

The term "solvate" refers to a compound of the present invention or a salt thereof having a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof having the same chemical formula or molecular formula but structurally or sterically different. Such isomers include structural isomers such as tautomers, and stereoisomers such as R or S isomers having an asymmetric carbon center, and geometric isomers (trans, cis). All of these isomers and mixtures thereof are also included in the scope of the present invention.

The term "pharmaceutically acceptable salt" means a salt form of a compound which does not cause serious irritation to the organism to which the compound is administered and does not impair the biological activity and physical properties of the compound. The pharmaceutical salts include acid addition salts formed by acids which form non-toxic acid addition salts containing pharmaceutically acceptable anions, for example, inorganic acids such as hydrochloric acid, sulfuric acid, nitric acid, phosphoric acid, hydrobromic acid, hydroiodic acid, etc., organic carboxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl)methylamine, diethanolamine, choline, and triethylamine. The compound of formula 1 according to the present invention can be converted into its salt by a conventional method.

In addition, the present invention provides a method for preparing the compound of formula 1. The synthetic methods of Examples 1 to 93 are exemplified as a method for preparing the compound of formula 1 of the present invention, and the synthetic methods of Examples 1 to 93 do not limit the method for preparing the compound of formula 1 according to the present invention. The synthetic methods of Examples 1 to 93 are merely examples, and it is obvious that they can be easily modified by those skilled in the art depending on a specific substituent.

The present invention also provides a pharmaceutical composition for preventing or treating a disease associated with autotaxin activity, comprising the heterocyclic compound, a hydrate thereof, a solvate thereof or a pharmaceutically acceptable salt thereof as an active ingredient.

The present invention also provides a method for inhibiting autotaxin and preventing or treating a disease resulting therefrom by administering the heterocyclic compound, a hydrate thereof, a solvate thereof, or a pharmaceutically acceptable salt thereof to a patient in need thereof.

The present invention also provides a use of the heterocyclic compound, a hydrate thereof, a solvate thereof or a pharmaceutically acceptable salt thereof for inhibiting autotaxin and preventing or treating diseases thereby.

As a result of measuring the autotaxin protein inhibitory activity of the heterocyclic compound of the present invention, it was confirmed that excellent autotaxin inhibitory activity was exhibited even at a very low compound concentration (nM level), and thus it can be used for the treatment and prevention of diseases related to autotaxin activity.

In one embodiment, the disease associated with said autotaxin activity may be selected from the group consisting of fibrotic diseases, inflammatory diseases, autoimmune diseases, respiratory diseases, cardiovascular diseases, metabolic diseases, cancer and cancer metastases, ocular diseases, cholestatic forms and other forms of chronic pruritus, and acute or chronic organ transplant rejection.

The above fibrotic diseases include, but are not limited to, idiopathic pulmonary fibrosis (idiopathic pulmonary fibrosis, IPF), interstitial lung disease, liver fibrosis, liver cirrhosis, non-alcoholic steatohepatitis, radiation-induced fibrosis, renal fibrosis, cutaneous fibrosis, glomerulosclerosis, myocardial and vascular fibrosis.

The above inflammatory diseases include, but are not limited to, rheumatoid arthritis, osteoarthritis, atopic dermatitis, inflammatory bowel disease, inflammatory airway disease, chronic obstructive pulmonary disease (COPD) and asthma.

The above autoimmune diseases include, but are not limited to, multiple sclerosis and scleroderma.

The above respiratory diseases include, but are not limited to, asbestos-induced pulmonary fibrosis and acute respiratory distress syndrome (ARDS).

The above cardiovascular diseases include, but are not limited to, arteriosclerosis, myocardial infarction, arterial and pulmonary hypertension, cardiac arrhythmias, stroke and other vascular damage.

The above metabolic diseases include, but are not limited to, obesity and diabetes.

The above cancers and cancer metastases include, but are not limited to, breast cancer, ovarian cancer, lung cancer, prostate cancer, mesothelioma, glioma, hepatocarcinoma, gastrointestinal cancer, pancreatic cancer, and their progression and metastatic invasion.

The above ocular diseases include, but are not limited to, proliferative and non-proliferative retinopathy, diabetic retinopathy, dry and wet age-related macular degeneration (AMD), macular edema, central artery/vein occlusion, traumatic injury, and glaucoma.

The present invention also provides a pharmaceutical composition or formulation comprising the heterocyclic compound, a hydrate thereof, a solvate thereof, or a pharmaceutically acceptable salt thereof, and a pharmaceutically acceptable additive.

The above additives may include pharmaceutically acceptable carriers such as commonly used excipients, disintegrants, sweeteners, lubricants or flavoring agents, and may be formulated into oral preparations such as tablets, capsules, powders, granules and suspensions, emulsions or syrups according to conventional methods; or parenteral preparations such as external solutions, external suspensions, external emulsions, gels (ointments, etc.), inhalants, sprays and injections. The preparations may be formulated into various forms, for example, single-dose or multiple-dose dosage forms.

The pharmaceutical composition of the present invention may include excipients such as lactose and corn starch, lubricants such as magnesium stearate, emulsifiers, suspending agents, stabilizers, and isotonic agents. If necessary, a sweetener and/or flavoring agent may be added.

The pharmaceutical composition of the present invention can be administered to mammals such as livestock and humans by various routes, for example, oral, dermal, subcutaneous, intramuscular, intravenous, intraperitoneal, intrarectal, intrauterine, intradural or intracerebrovascular injection, and topical administration. Accordingly, the composition of the present invention can be formulated in various forms such as tablets, capsules, aqueous solutions or suspensions. In the case of tablets for oral administration, carriers such as lactose and corn starch and lubricants such as magnesium stearate can usually be added. In the case of capsules for oral administration, lactose and/or dried corn starch can be used as diluents. When an oral aqueous suspension is required, the active ingredient can be combined with an emulsifier and/or a suspending agent. If necessary, a specific sweetener and/or flavoring agent can be added. In the case of intramuscular, intraperitoneal, subcutaneous and intravenous administration, a sterile solution of the active ingredient is usually prepared, and the pH of the solution should be suitably adjusted and buffered. In the case of intravenous administration, the total concentration of the solute should be adjusted so as to impart isotonicity to the formulation. The composition according to the present invention may be in the form of an aqueous solution containing a pharmaceutically acceptable carrier, such as saline having a pH of 7.4. The solution may be introduced into the intramuscular bloodstream of the patient by local injection.

The dosage of the active ingredient contained in the pharmaceutical composition of the present invention varies depending on the patient's condition and weight, the degree of the disease, the form of the active ingredient, the route and period of administration, and can be appropriately adjusted depending on the patient. For example, the active ingredient can be administered at a dosage of 0.0001 to 1000 mg/kg per day, preferably 0.01 to 100 mg/kg, and the administration can be administered once a day or divided into several times. In addition, the pharmaceutical composition of the present invention can contain the active ingredient at a weight percentage of 0.001 to 90% based on the total weight of the composition.

The pharmaceutical composition of the present invention can be administered to mammals such as rats, mice, livestock, and humans by various routes, for example, orally, cutaneously, intraperitoneally, rectally, or by intravenous, intramuscular, subcutaneous, intrauterine, or intracerebroventricular injection.

Hereinafter, the present invention will be described in more detail through manufacturing examples, examples, and test examples. However, the following Preparation examples, Examples, and Tst examples are intended to illustrate the present invention, and the scope of the present invention is not limited thereto.

### Preparation example 1. Preparation of (4-(pyrrolidin-3-yl)-1H-1,2,3-triazol-1-yl)methyl pivalate, 2,2,2-trifluoroacetate

### Step 1: Preparation of tert-butyl 3-(1-((pivaloyloxy)methyl)-1H-1,2,3-triazol-4-yl)pyrrolidine-1-carboxylate

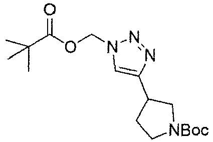

Azidomethyl pivalate (0.2 g, 1.27 mmol) and tert-butyl 3-ethynyl-1-pyrrolidinecarboxylate (0.249 g, 1.27 mmol) were dissolved in THF (2.5 mL)/DW (2.5 mL), and CuOAc (15.6 mg, 0.127 mmol) and NaOAc (0.313 g, 3.82 mmol) were sequentially added, and the mixture was stirred at room temperature overnight. DW was added to the reaction mixture, and extracted with EtOAc. The organic layer was dried over Na₂SO₄, filtered and concentrated, and the residue was purified by column chromatography (MeOH/DCM) to give the title compound as a colorless oil (0.23 g, 50.4%).

¹H NMR (400MHz, DMSO-d₆) *δ* 7.58 (s, 1H), 6.20 (s, 2H), 3.84 - 3.78 (m, 1H), 3.52 (dd, *J* = 15.6, 7.8 Hz, 2H), 3.46 - 3.37 (m, 2H), 2.31 (s, 2H), 1.46 (s, 9H), 1.19 (s, 9H)

### Step 2: Preparation of (4-(pyrrolidin-3-yl)-1H-1,2,3-triazol-1-yl)methyl pivalate, 2,2,2-trifluoroacetate

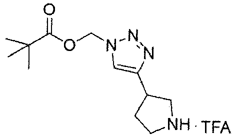

To a solution of tert-Butyl 3-(1-((pivaloyloxy)methyl)-1H-1,2,3-triazol-4-yl)pyrrolidine-1-carboxylate (0.222 g, 0.630 mmol) in DCM (6.3 mL) was added TFA (2.1 mL) at 0 °C and stirred at room temperature for 2 h. The reaction mixture was concentrated under reduced pressure to obtain the title compound as a yellow oil (quantitatively), which was used in the next reaction without purification. (180 mg).

¹H NMR (400 MHz, CDCl3) *δ* 7.76 (s, 1H), 6.20 (s, 2H), 3.75 (s, 2H), 2.52 (s, 5H), 1.19 (s, 9H)

### Preparation example 2. Preparation of 4-(pyrrolidin-3-yl)-1H-1,2,3-triazole trifluoroacetic acid

### Step 1: tert-Butyl 3-(1H-1,2,3-triazol-4-yl)pyrrolidin-1-carboxylate

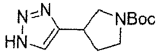

The mixture of tert-Butyl 3-ethynyl-1-pyrrolidinecarboxylate (0.3 g, 1.54 mmol), TMSN₃ (221 µL, 1.69 mmol), CuI (29.3 mg, 0.154 mmol) and DMF/MeOH (5.1 mL/0.67 mL) was stirred at 80° C overnight. After concentrating the reaction mixture, DW was added and extracted with EtOAc. The organic layer was dried over Na₂SO₄, filtered and concentrated, and the obtained residue was purified by column chromatography (MeOH/DCM) to obtain the title compound as a light green oil (0.154 g, 42.0%).

¹H NMR (400 MHz, CDCl₃) *δ* 7.54 (s, 1H), 3.91 - 3.77 (m, 1H), 3.65 - 3.35 (m, 4H), 2.37 - 2.04 (m, 2H), 1.47 (s, 9H)

### Step 2: Preparation of 4-(pyrrolidin-3-yl)-1H-1,2,3-triazole, 2,2,2-trifluoroacetate

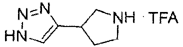

To a solution of tert-butyl 3-(1H-1,2,3-triazol-4-yl)pyrrolidine-1-carboxylate (0.150 g, 0.629 mmol) in DCM (6.3 mL) was added TFA (2.1 mL) at 0 °C and the mixture was stirred at room temperature for 2 h. The reaction mixture was concentrated to give the title compound as a yellow oil (quantitative) which was used for the next reaction without purification (148 mg).

### Preparation example 3. Preparation of 5-(2-((2,3-dihydro-1H-inden-2-yl)amino)pyrimidin-5-yl)-1,3,4-oxadiazol-2(3H)-one

### Step 1: Preparation of ethyl 2-((2,3-dihydro-1H-inden-2-yl)amino)pyrimidine-5-carboxylate

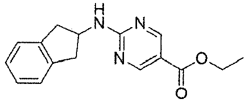

2,3-Dihydro-1H-inden-2-amine (1 g, 7.5 mmol) was dissolved in anhydrous 1,4-dioxane (25 mL), and N,N-Diisopropylethylamine (DIPEA) (2.6 mL, 15 mmol) and ethyl 2-chloropyrimidine-5-carboxylate (1.26 g, 6.76 mmol) were sequentially added. The mixture was stirred at 100 °C for 3 hours. The reaction mixture was cooled to room temperature, DW (20 mL) was added, and the mixture was stirred for 2 hours. The resulting solid was filtered, washed with DW and dioxane, and dried in vacuo to obtain the title compound (1.5 g, yield 70.5%) as a white solid.

¹H NMR (400 MHz, DMSO-d₆) *δ* 8.80 (s, 1H), 8.72 (s, 1H), 8.46 (d, J = 6.9 Hz, 1H), 7.32 - 7.04 (m, 4H), 4.75 - 4.67 (m, 1H), 4.27 (q, J = 7.1 Hz, 2H), 3.27 (dd, J = 15.8, 7.6 Hz, 2H), 2.92 (dd, J = 15.8, 6.8 Hz, 2H), 1.29 (t, J = 7.1 Hz, 3H); LCMS m/z 284 [M+H]⁺

### Step 2: Preparation of 2-((2,3-dihydro-1H-inden-2-yl)amino)pyrimidine-5-carbohydrazide

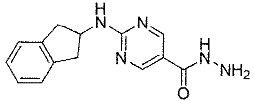

The reaction mixture of ethyl 2-((2,3-dihydro-1H-inden-2-yl)amino)pyrimidine-5-carboxylate (0.5 g, 1.77 mmol), hydrazine monohydrate (2.8 mL, 35.3 mmol), and EtOH (20 mL) was stirred overnight at 80°C. After cooling to room temperature, DW was added and stirred at 0°C for 2 hours. The resulting solid was filtered and dried under vacuum to obtain the title compound as a white solid. (0.4 g, 84.6%).

LCMS m/z 270 [M+H]⁺

### Step 3: Preparation of 5-(2-((2,3-dihydro-1H-inden-2-yl)amino)pyrimidin-5-yl)-1,3,4-oxadiazol-2(3H)-one

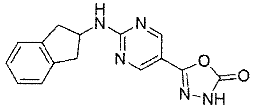

2-((2,3-Dihydro-1H-inden-2-yl)amino)pyrimidine-5-carbohydrazide (0.35 g, 1.31 mmol) was dissolved in THF (7 mL) and cooled to 0 °C. TEA (0.18 mL, 1.31 mmol) and CDI (0.26 g, 1.57 mmol) were slowly added, and the mixture was stirred at 0 °C for 20 min and at room temperature for 20 min. The reaction mixture was concentrated, EtOAc was added, and the mixture was stirred at room temperature for 1 h. The resulting solid was filtered, washed once with cold EtOAc, and dried in vacuo to obtain the title compound as a white solid (0.33 g, 86%).

¹H NMR (400 MHz, DMSO-d₆) *δ* 12.45 (broad, 1H), 8.64 (d, J = 13.2 Hz, 2H), 8.31 (s, 1H), 7.24 - 7.12 (m, 4H), 4.73 - 4.64 (m, 1H), 3.30 - 3.23 (m, 2H), 2.92 (dd, J = 15.7, 6.8 Hz, 2H); LCMS m/z 296 [M+H]⁺

### Preparation example 4. Preparation of 5-(2-((6,7-dihydro-5H-cyclopenta[b]pyridin-6-yl)amino)pyrimidin-5-yl)-1,3,4-oxadiazol-2(3H)-one

### Step 1: Ethyl 2-((6,7-dihydro-5H-cyclopenta[b]pyridin-6-yl)amino)pyrimidine-5-carboxylate

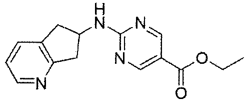

6,7-Dihydro-5H-cyclopenta[b]pyridin-6-amine dihydrochloride (346 mg, 1.67 mmol) was dissolved in anhydrous dioxane (5.6 mL), DIPEA (0.85 mL, 5.01 mmol) and ethyl 2-chloropyrimidine-5-carboxylate (280 mg, 1.50 mmol) were added, and the mixture was stirred at 100 °C for 4 h. DW (20 mL) was added to the reaction mixture, and extracted with EtOAc. The organic layer was washed with brine, dried over MgSO₄, filtered and concentrated. The obtained residue was purified by column chromatography (0-33% EtOAc/Hex->3% MeOH/DCM) to give the title compound as a brown solid (125 mg, 29%).

¹H NMR (400 MHz, CDCl₃) *δ* 8.85 (d, J = 32.9 Hz, 2H), 8.39 (d, J = 4.9 Hz, 1H), 7.52 (d, J = 7.4 Hz, 1H), 7.09 (dd, J = 7.2, 5.3 Hz, 1H), 5.89 (d, J = 7.1 Hz, 1H), 4.98 - 4.85 (m, 1H), 4.35 (q, J = 7.0 Hz, 2H), 3.58 - 3.39 (m, 2H), 2.98 (ddd, J = 36.1, 16.5, 5.1 Hz, 2H), 1.37 (t, J = 7.1 Hz, 3H)

### Step 2: 2-((6,7-dihydro-5H-cyclopenta[b]pyridin-6-yl)amino)pyrimidine-5-carbohydrazide

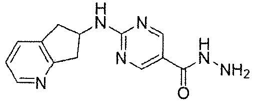

Ethyl 2-((6,7-dihydro-5H-cyclopenta[b]pyridin-6-yl)amino)pyrimidine-5-carboxylate (120 mg, 0.42 mmol) and hydrazine monohydrate (0.45 mL, 9.28 mmol) were dissolved in EtOH (4.6 mL) and stirred at 100 °C for 22 h. The reaction mixture was concentrated and solidified by adding DCM. The resulting solid was filtered and dried to obtain the title compound as a pale orange solid. (81 mg, 71%)

LCMS m/z 272 [M+H]⁺

### Step 3: 5-(2-((6,7-dihydro-SH-cyclopenta[b]pyridin-6-yl)amino)pyrimidin-5-yl)-1,3,4-oxadiazol-2(3H)-one

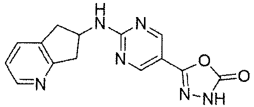

2-((6,7-Dihydro-5H-cyclopenta[b]pyridin-6-yl)amino)pyrimidine-5-carbohydrazide (80 mg, 0.30 mmol) was dissolved in acetonitrile (1.6 mL), and TEA (43.3 uL, 0.31 mmol) and CDI (63 mg, 0.39 mmol) were added at 0 °C. The mixture was stirred at the same temperature for 15 minutes and then at room temperature for 6 hours. After concentrating the reaction mixture, the obtained residue was purified by column chromatography (0-2% MeOH/DCM) to obtain the title compound as an off-white solid (83 mg, 94%).

¹H NMR (400 MHz, DMSO-d₆) δ12.12 (br s, 1H), 8.66 (d, J = 15.9 Hz, 2H), 8.36 (d, J = 6.8 Hz, 1H), 8.30 (d, J = 4.9 Hz, 1H), 7.59 (d, J = 8.1 Hz, 1H), 7.13 (dd, J = 7.4, 5.2 Hz, 1H), 4.76 - 4.65 (m, 1H), 3.28 - 3.22 (m, 2H), 3.03 - 2.88 (m, 2H); LCMS m/z 297 [M+H]⁺

### Preparation example 5. Preparation of (4-(3-methylpyrrolidin-3-yl)-1H-1,2,3-triazol-1-yl)methyl pivalate, 2,2,2-trifluoroacetate

### Step 1: Preparation of tert-butyl 3-methyl-3-(1-((pivaloyloxy)methyl)-1H-1,2,3-triazol-4-yl)pyrrolidine-1-carboxylate

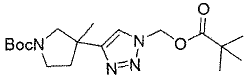

Azidomethyl pivalate (101 mg, 0.65 mmol) and tert-butyl 3-ethynyl-3-methylpyrrolidine-1-carboxylate (135 mg, 0.65 mmol) were dissolved in t-BuOH (1.2 mL)/DW (1.2 mL), and 10% aqueous CuSO₄5H₂O solution (0.5 mL, 0.19 mmol) and sodium ascorbate (38 mg, 0.19 mmol) were added sequentially, and the mixture was stirred at room temperature overnight. DW was added to the reaction mixture, extracted with EtOAc, and the organic layer was washed with brine. The organic layer was dried over MgSO₄, filtered, concentrated, and the resulting residue was purified by column chromatography (0-2% MeOH/DCM) to obtain the title compound as a colorless oil (244 mg, 99%).

¹H NMR (400 MHz, CDCl₃) *δ* 7.57 (s, 1H), 6.20 (s, 2H), 3.68 (t, J = 9.8 Hz, 1H), 3.56 - 3.32 (m, 4H), 2.43 - 2.26 (m, 1H), 2.02 - 1.89 (m, 1H), 1.46 (s, 9H), 1.19 (s, 9H)

### Step 2: Preparation of (4-(3-methylpyrrolidin-3-yl)-1H-1,2,3-triazol-1-yl)methyl pivalate, 2,2,2-trifluoroacetate

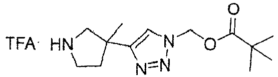

By the same method as in step 2 of Preparation example 1, tert-Butyl 3-methyl-3-(1-((pivaloyloxy)methyl)-1H-1,2,3-triazol-4-yl)pyrrolidine-1-carboxylate (244 mg, 0.67 mmol), TFA (2 mL), and DCM (6.5 mL) were used to obtain the title compound as a light pink oil (quantitative) which was used in the next reaction without purification (326 mg).

LCMS m/z 267 [M+H]⁺

### Preparation example 6. Preparation of 4-(azetidin-3-yl)-1H-1,2,3-triazole, 2,2,2-trifluoroacetate

### Step 1: Preparation of tert-butyl 3-(1H-1,2,3-triazol-4-yl)azetidine-1-carboxylate

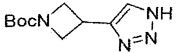

By the same method as in Step 1 of Preparation example 2, tert-butyl 3-ethynyl azetidine-1-carboxylate (40 mg, 0.221 mmol), TMSN₃ (32 µL, 0.243 mmol), CuI (4.2 mg, 0.022 mmol), and DMF (0.7 mL)/MeOH (96 µL) were used to obtain the title compound as a colorless oil (29.2 mg, 59.0%).

¹H NMR (400MHz, DMSO-d₆) *δ* 7.83 (s, 1H), 4.19 (s, 2H), 3.88 (s, 3H), 1.37 (s, 9H)

### Step 2: Preparation of 4-(azetidin-3-yl)-1H-1,2,3-triazole, 2,2,2-trifluoroacetate

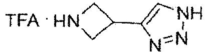

By the same method as in Step 2 of Preparation example 2, tert-butyl 3-(1H-1,2,3-triazol-4-yl)azetidine-1-carboxylate (25 mg, 0.111 mmol), TFA (372 µL), and DCM (1.1 mL) were used to obtain the title compound as a yellow oil (quantitatively), which was used in the next reaction without purification (24 mg).

### Preparation example 7. Preparation of 4-(azetidin-3-yl)-1H-1,2,3-triazole dihydrochloride

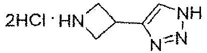

tert-Butyl 3-(1H-1,2,3-triazol-4-yl)azetidine-1-carboxylate (13.3 g, 59.3 mmol) was dissolved in DCM (94 mL) and 4 M HCl dioxane solution (60 mL) was slowly added in a water bath. The mixture was stirred at room temperature for 4 h. The resulting solid was filtered and washed with cold DCM. The solid was dissolved in MeOH and concentrated. The residue was then azeotropically concentrated three times with toluene to obtain the title compound as an off-white solid (11.4 g, 97.5%).

¹H NMR (400 MHz, DMSO-d₆) δ 9.63 (br s, 1H), 9.33 (br s, 1H), 8.29 (br s, 1H), 7.92 (s, 1H), 4.27~3.95 (m, 4H), 3.67~3.64 (m, 1H).

### Preparation example 8. Preparation of (4-(azetidin-3-yl)-1H-1,2,3-triazol-1-yl)methyl pivalate

### Step 1: Preparation of tert-butyl 3-(1-((pivaloyloxy)methyl)-1H-1,2,3-triazol-4-yl)azetidine-1-carboxylate

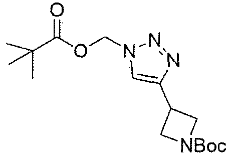

By the same method as in Step 1 of Preparation example 1, azidomethyl pivalate (0.3 g, 1.91 mmol), tert-butyl 3-ethynyl azetidine-1-carboxylate (0.22 g, 1.91 mmol), CuOAc (23.4 mg, 0.191 mmol), NaOAc (0.47 g, 5.73 mmol), and THF (3.8 mL)/DW (3.8 mL) were used to obtain the title compound was obtained as a white solid (0.361 g, 55.9%).

¹H NMR (400 MHz, CDCl₃) *δ* 7.70 (s, 1H), 6.22 (s, 2H), 4.32 (t, J = 8.6 Hz, 2H), 4.13 - 4.02 (m, 2H), 3.94 - 3.87 (m, 1H), 1.45 (s, 9H), 1.20 (s, 9H)

### Step 2: (4-(azetidin-3-yl)-1H-1,2,3-triazol-1-yl)methyl pivalate

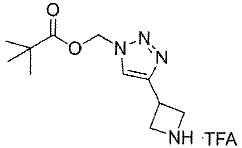

By the same method as in Step 2 of Preparation example 1, tert-butyl 3-(1-((pivaloyloxy)methyl)-1H-1,2,3-triazol-4-yl)azetidine-1-carboxylate (0.355 g, 1.05 mmol), TFA (3.5 mL), and DCM (10.5 mL) were used to obtain the title compound (quantitatively), which was used in the next reaction without purification (282 mg).

### Example 1. 5-(5-(3-(1H-1,2,3-triazol-4-yl)pyrrolidin-1-yl)-1,3,4-oxadiazol-2-yl)-N-(2,3-dihydro-1H-inden-2-yl)pyrimidin-2-amine

### Step 1: (4-(1-(5-(2-((2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

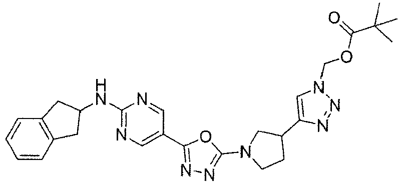

5-(2-((2,3-Dihydro-1H-inden-2-yl)amino)pyrimidin-5-yl)-1,3,4-oxadiazol-2(3H)-one (23.8 mg, 0.08 mmol) and (4-(pyrrolidin-3-yl)-1H-1,2,3-triazol-1-yl)methyl pivalate, 2,2,2-trifluoroacetate (29.5 mg, 0.08 mmol) were dissolved in DMF (2 mL), and DIPEA (69 uL, 0.4 mmol) and BOP reagent (42.7 mg, 0.097 mmol) were added sequentially. After stirring at room temperature overnight, the reaction mixture was washed with DW, extracted with EtOAc, and the organic layer was washed with brine. The organic layer was dried over MgSO₄, filtered and concentrated, and the resulting residue was purified by column chromatography (0-10% MeOH/DCM) to obtain the title compound as a white solid (34.5 mg, 80.9%).

LCMS m/z 530 [M+H]⁺

### Step 2: 5-(5-(3-(1H-1,2,3-triazol-4-yl)pyrrolidin-1-yl)-1,3,4-oxadiazol-2-yl)-N-(2,3-dihydro-1H-inden-2-yl)pyrimidin-2-amine

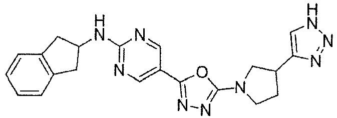

(4-(1-(5-(2-((2,3-Dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (33.2 mg, 0.063 mmol) was dissolved in MeOH (2 mL), K₂CO₃ (17.3 mg, 0.13 mmol) was added, and the mixture was stirred at room temperature for 1 h. DW was added to the reaction mixture, and then extracted with EtOAc. The organic layer was washed with brine, dried over MgSO₄, filtered and concentrated. The obtained residue was purified by column chromatography (0-10% MeOH/DCM) to give the title compound as a white solid (19 mg, 72.9%).

¹H NMR (400 MHz, DMSO-d₆) *δ* 8.74 (d, J = 14.2 Hz, 2H), 8.24 (d, J = 6.8 Hz, 1H), 7.88 - 7.72 (m, 1H), 7.29 - 7.07 (m, 4H), 4.73 - 4.62 (m, 1H), 3.99 - 3.88 (m, 1H), 3.76 - 3.53 (m, 4H), 3.27 (dd, J = 15.9, 7.6 Hz, 2H), 2.45 - 2.33 (dd, J = 15.9, 6.7 Hz, 2H), 2.24 - 2.11 (m, 1H), 2.16 (m, 1H); LCMS m/z 416 [M+H]⁺

### Example 2. (4-(1-(5-(2-((5,6-difluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

### Step 1: 5-(2-((5,6-difluoro-2,3-dihydro-1H-inden-2-yl)amino)pyrimidin-5-yl)-1,3,4-oxadiazol-2(3H)-one

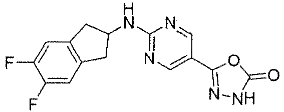

The title compound was prepared by the same method as in Preparation Example 3, except that 5,6-difluoro-2,3-dihydro-1H-inden-2-amine was used instead of 2,3-dihydro-1H-inden-2-amine.

¹H NMR (400 MHz, DMSO-d₆) *δ* 12.45 (s, 1H), 8.65 (d, *J =* 14.6 Hz, 2H), 8.33 (d, *J* = 6.8 Hz, 1H), 7.27 (t, *J* = 9.3 Hz, 2H), 4.68 (dq, *J* = 13.8, 6.9 Hz, 1H), 3.24 (dd, *J* = 16.1, 7.6 Hz, 2H), 2.87 (dd, *J* = 16.0, 6.4 Hz, 2H); LCMS m/z 332 [M+H]⁺

### Step 2: (4-(1-(5-(2-((5,6-difluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

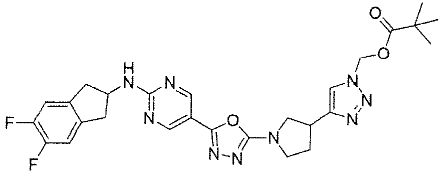

By the same method as in Step 1 of Example 1, (4-(pyrrolidin-3-yl)-1H-1,2,3-triazol-1-yl)methyl pivalate, 2,2,2-trifluoroacetate (0.1 g, 0.302 mmol), 5-(2-((5,6-difluoro-2,3-dihydro-1H-inden-2-yl)amino)pyrimidin-5-yl)-1,3,4-oxadiazol-2(3H)-one (0.127 g, 0.362 mmol), DIPEA (154 µL, 0.906 mmol), BOP reagent (0.160 g, 0.362 mmol), and DMF (1.5 mL) were used to obtain the title compound was obtained as a white solid (69.8 mg, 40.8%).

¹H NMR (400 MHz, DMSO-d₆) *δ* 8.72 (s, 2H), 8.23 (d, J = 6.9 Hz, 1H), 8.18 (s, 1H), 7.27 (t, J = 9.4 Hz, 2H), 6.26 (s, 2H), 4.68 (dd, J = 14.4, 7.4 Hz, 1H), 3.94 - 3.88 (m, 1H), 3.73 - 3.64 (m, 2H), 3.64 - 3.54 (m, 4H), 3.24 (d, J = 8.4 Hz, 2H), 2.88 (dd, J = 16.0, 6.5 Hz, 2H), 1.10 (s, 9H); LCMS m/z 566 [M+H]⁺

### Example 3. 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrimidin-2-amine

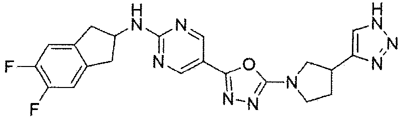

(4-(1-(5-(2-((5,6-Difluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (68 mg, 0.120 mmol) was dissolved in MeOH (0.6 mL), K₂CO₃ (33.2 mg, 0.240 mmol) was added, and the mixture was stirred at room temperature for 2 h. The reaction mixture was filtered and concentrated, and the resulting residue was purified by column chromatography (MeOH/DCM) to obtain the title compound as a white solid (47.2 mg, 87.0%).

¹H NMR (400 MHz, DMSO-d₆) *δ* 8.72 (s, 2H), 8.23 (d, J = 6.7 Hz, 1H), 7.79 (s, 1H), 7.27 (t, J = 9.3 Hz, 2H), 4.68 (dd, J = 13.9, 7.0 Hz, 1H), 3.94 - 3.87 (m, 1H), 3.58 (d, J = 9.0 Hz, 2H), 3.24 (d, J = 8.6 Hz, 2H), 2.87 (dd, J = 16.1, 6.5 Hz, 2H), 2.38 (dd, J = 12.4, 5.6 Hz, 2H), 2.20 - 2.06 (m, 2H); LCMS m/z 452 [M+H]⁺

### Example 4. (4-(1-(5-(2-((6,7-dihydro-5H-cyclopenta[b]pyridin-6-yl)amino)pyrimidin-5-yl)-1,3,4-oxadiazol-2-yl)pyrrolidin-3-yl)-1H-1,2,3-triazol-1-yl)methyl pivalate

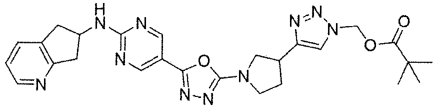

By the same method as in step 1 of Example 1, 5-(2-((6,7-dihydro-5H-cyclopenta[b]pyridin-6-yl)amino)pyrimidin-5-yl)-1,3,4-oxadiazol-2(3H)-one (27.8 mg, 0.094 mmol), (4-(pyrrolidin-3-yl)-1H-1,2,3-triazol-1-yl)methyl pivalate, 2,2,2-trifluoroacetate (56 mg, crude), DIPEA (83 uL, 0.49 mmol), BOP reagent (49.8 mg, 0.113 mmol), and DMF (1.88 mL) were used to obtain the title compound as a yellow solid (28 mg, 56%).

¹H NMR (400 MHz, DMSO-d₆) δ8.75 (s, 2H), 8.29 (dd, J = 17.1, 5.7 Hz, 2H), 8.20 (s, 1H), 7.61 (d, J = 7.4 Hz, 1H), 7.15 (dd, J = 7.3, 5.1 Hz, 1H), 6.28 (s, 2H), 4.79 - 4.66 (m, 1H), 3.93 (dd, J = 9.3, 7.5 Hz, 1H), 3.71 (dt, J = 14.1, 7.0 Hz, 1H), 3.67 - 3.55 (m, 3H), 3.38 - 3.34 (m, 1H), 3.30 - 3.27 (m, 1H), 3.06 - 2.90 (m, 2H), 2.45 - 2.35 (m, 1H), 2.24 - 2.11 (m, 1H), 1.12 (s, 9H); LCMS m/z 531 [M+H]⁺

### Example 5. (4-(1-(5-(2-((benzo[d][1,3]dioxol-5-ylmethyl)amino)pyrimidin-5-yl)-1,3,4-oxadiazol-2-yl)pyrrolidin-3-yl)-1H-1,2,3-triazol-1-yl)methyl pivalate

### Step 1: 5-(2-((benzo[d][1,3]dioxol-5-ylmethyl)amino)pyrimidin-5-yl)-1,3,4-oxadiazol-2(3H)-one

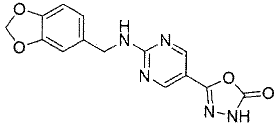

The title compound was prepared by the same method as in Preparation Example 3, except that benzo[d][1,3]dioxol-5-ylmethanamine was used instead of 2,3-dihydro-1H-inden-2-amine.

¹H NMR (400 MHz, DMSO-d₆) δ12.42 (s, 1H), 8.64 (s, 2H), 8.45 (t, J = 6.3 Hz, 1H), 6.92 - 6.73 (m, 3H), 5.97 (s, 2H), 4.47 (d, J = 6.3 Hz, 2H); LCMS m/z 314 [M+H]⁺

### Step 2: (4-(1-(5-(2-((benzo[d][1,3]dioxol-5-ylmethyl)amino)pyrimidin-5-yl)-1,3,4-oxadiazol-2-yl)pyrrolidin-3-yl)-1H-1,2,3-triazol-1-yl)methyl pivalate

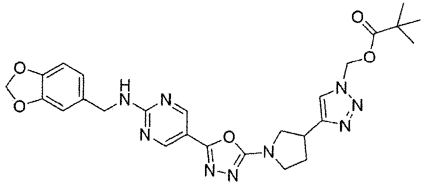

By the same method as in Step 1 of Example 1, 5-(2-((benzo[d][1,3]dioxol-5-ylmethyl)amino)pyrimidin-5-yl)-1,3,4-oxadiazol-2(3H)-one (34 mg, 0.11 mmol), (4-(pyrrolidin-3-yl)-1H-1,2,3-triazol-1-yl)methyl pivalate, 2,2,2-trifluoroacetate (45 mg, 0.13 mmol), DIPEA (91 µL, 0.54 mmol), BOP reagent (57 mg, 0.13 mmol), and DMF (1 mL) were used to obtain the title compound as a white solid (41 mg, 70%).

¹H NMR (400 MHz, DMSO-d₆) δ8.71 (s, 2H), 8.34 (t, *J* = 6.3 Hz, 1H), 8.19 (s, 1H), 6.89 (s, 1H), 6.85 - 6.77 (m, 2H), 6.28 (s, 2H), 5.97 (s, 2H), 4.46 (d, *J=* 6.2 Hz, 2H), 3.95 - 3.89 (m, 1H), 3.74 - 3.67 (m, 1H), 3.64 - 3.56 (m, 3H), 2.44 - 2.36 (m, 1H), 2.22 - 2.13 (m, 1H), 1.11 (s, 9H); LCMS m/z 548 [M+H]⁺

### Example 6. 5-(5-(3-(1H-1,2,3-triazol-4-yl)pyrrolidin-1-yl)-1,3,4-oxadiazol-2-yl)-N-(benzo[d][1,3]dioxol-5-ylmethyl)pyrimidin-2-amine

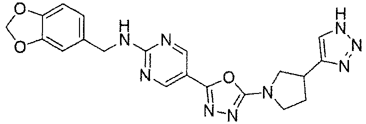

By the same method as in step 2 of Example 1, (4-(1-(5-(2-((benzo[d][1,3]dioxol-5-ylmethyl)amino)pyrimidin-5-yl)-1,3,4-oxadiazol-2-yl)pyrrolidin-3-yl)-1H-1,2,3-triazol-1-yl)methyl pivalate (36 mg, 0.066 mmol), K₂CO₃ (18 mg, 0.13 mmol), and MeOH (1 mL) was used to obtain the title compound as a white solid (22 mg, 76%).

¹H NMR (400 MHz, DMSO-d₆) δ8.71 (s, 2H), 8.35 (t, *J =* 6.3 Hz, 1H), 7.81 (s, 1H), 6.89 (s, 1H), 6.85 - 6.77 (m, 2H), 5.97 (s, 2H), 4.46 (d, *J =* 6.2 Hz, 2H), 3.95 - 3.89 (m, 1H), 3.71 - 3.59 (m, 4H), 2.44 - 2.36 (m, 1H), 2.21 - 2.10 (m, 1H); LCMS m/z 434 [M+H]⁺

### Example 7. N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyrazin-6-amine

### Step 1: 5-(2-((6,7-dihydro-5H-cyclopenta[b]pyrazin-6-yl)amino)pyrimidin-5-yl)-1,3,4-oxadiazol-2(3H)-one

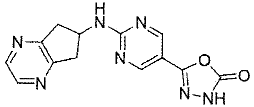

The title compound was prepared by the same method as in Preparation Example 4, except that 6,7-dihydro-5H-cyclopenta[b]pyrazin-6-amine dihydrochloride was used instead of 6,7-dihydro-5H-cyclopenta[b]pyridin-6-amine dihydrochloride.

1H NMR (400 MHz, DMSO-d₆) δ12.03 (br s, 1H), 8.68 (s, 2H), 8.42 (d, J = 6.8 Hz, 1H), 8.36 (s, 2H), 4.88 - 4.76 (m, 1H), 3.46 - 3.41 (m, 2H), 3.05 (dd, J = 17.3, 5.5 Hz, 2H); LCMS m/z 298 [M+H]⁺

### Step 2: (4-(1-(5-(2-((6,7-dihydro-5H-cyclopenta[b]pyrazin-6-yl)amino)pyrimidin-5-yl)-1,3,4-oxadiazol-2-yl)pyrrolidin-3-yl)-1H-1,2,3-triazol-1-yl)methyl pivalate

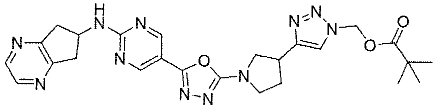

By the same method as in step 1 of example 1, 5-(2-((6,7-dihydro-5H-cyclopenta[b]pyrazin-6-yl)amino)pyrimidin-5-yl)-1,3,4-oxadiazol-2(3H)-one (35.5 mg, 0.119 mmol), (4-(pyrrolidin-3-yl)-1H-1,2,3-triazol-1-yl)methyl pivalate, 2,2,2-trifluoroacetate (71 mg, 0.19 mmol), DIPEA (105 uL, 0.62 mmol), BOP reagent (63.4 mg, 0.14 mmol), and DMF (2.4 mL) were used to obtain the title compound as a yellow solid (15 mg, 19%).

¹H NMR (400 MHz, DMSO-d₆) δ 8.76 (s, 2H), 8.36 (s, 2H), 8.33 (d, J = 6.9 Hz, 1H), 8.20 (s, 1H), 6.28 (s, 2H), 4.88 - 4.74 (m, 1H), 3.93 (dd, J = 9.4, 7.0 Hz, 1H), 3.77 - 3.66 (m, 1H), 3.66 - 3.54 (m, 3H), 3.43 (dd, J = 17.3, 7.9 Hz, 2H), 3.05 (dd, J = 17.3, 5.6 Hz, 2H), 2.44 - 2.35 (m, 1H), 2.24 - 2.13 (m, 1H), 1.12 (s, 9H); LCMS m/z 532 [M+H]⁺

### Step 3: N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyrazin-6-amine

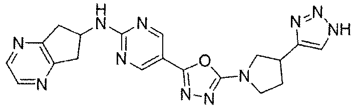

(4-(1-(5-(2-((6,7-Dihydro-5H-cyclopenta[b]pyrazin-6-yl)amino)pyrimidin-5-yl)-1,3,4-oxadiazol-2-yl)pyrrolidin-3-yl)-1H-1,2,3-triazol-1-yl)methyl pivalate (12.6 mg, 0.024 mmol) was dissolved in MeOH (0.47 mL), and K₂CO₃ (6.6 mg, 0.047 mmol) was added, and the mixture was stirred at room temperature for 2 hours. After concentrating the reaction mixture, the obtained residue was purified by column chromatography (0-8% MeOH/DCM) to give the title compound as a white solid (8 mg, 81%).

¹H NMR (400 MHz, DMSO-d₆) δ8.76 (s, 2H), 8.46 - 8.22 (m, 3H), 7.81 (s, 1H), 4.87 - 4.76 (m, 1H), 3.93 (dd, J = 9.2, 7.3 Hz, 1H), 3.77 - 3.52 (m, 4H), 3.43 (dd, J = 17.4, 8.0 Hz, 2H), 3.17 (d, J = 5.1 Hz, 1H), 3.05 (dd, J = 17.2, 5.6 Hz, 2H), 2.45 - 2.35 (m, 1H), 2.22 - 2.10 (m, 1H); LCMS m/z 418 [M+H]⁺

### Example 8. N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyridin-6-amine

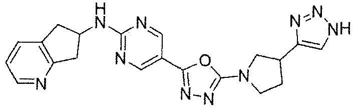

By the same method as in Step 3 of Example 7, (4-(1-(5-(2-((6,7-dihydro-5H-cyclopenta[b]pyridin-6-yl)amino)pyrimidin-5-yl)-1,3,4-oxadiazol-2-yl)pyrrolidin-3-yl)-1H-1,2,3-triazol-1-yl)methyl pivalate (22.8 mg, 0.043 mmol), K₂CO₃ (11.9 mg, 0.086 mmol), and MeOH (0.86 mL) were used to obtain the title compound as a white solid (9 mg, 50%).

¹H NMR (400 MHz, DMSO-d₆) δ8.75 (s, 2H), 8.30 (dd, J = 14.0, 5.8 Hz, 2H), 7.82 (s, 1H), 7.61 (d, J = 7.9 Hz, 1H), 7.15 (dd, J = 7.4, 5.1 Hz, 1H), 4.80 - 4.65 (m, 1H), 3.93 (dd, J = 9.3, 7.4 Hz, 1H), 3.75 - 3.54 (m, 4H), 3.38 - 3.35 (m, 1H), 3.31 - 3.27 (m, J = 9.0 Hz, 1H), 3.07 - 2.89 (m, 2H), 2.44 - 2.35 (m, 1H), 2.21 - 2.09 (m, 1H); LCMS m/z 417 [M+H]+

### Example 9. (E)-1-(3-(1H-1,2,3-triazol-4-yl)pyrrolidin-1-yl)-3-(2-((5,6-difluoro-2,3-dihydro-1H-inden-2-yl)amino)pyrimidin-5-yl)prop-2-en-1-one

### Step 1: ethyl (E)-3-(2-((5,6-difluoro-2,3-dihydro-1H-inden-2-yl)amino)pyrimidin-5-yl)acrylate

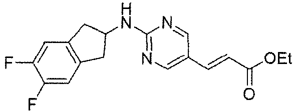

5,6-Difluoro-2,3-dihydro-1H-inden-2-amine (0.507 g, 1.86 mmol), ethyl (E)-3-(2-chloropyrimidin-5-yl)acrylate (0.789 g, 3.71 mmol), DIPEA (6.3 mL, 37.1 mmol) were dissolved in n-BuOH (3.7 mL) and reacted in a microwave reactor at 150 ° C for 2 h. After concentrating the reaction mixture, the obtained residue was purified by column chromatography (EtOAc/Hex) to obtain the title compound as a brown solid (0.348 g, 54.4%).

¹H NMR (400 MHz, DMSO-d₆) *δ* 8.67 (s, 2H), 8.09 (d, J = 6.8 Hz, 1H), 7.48 (d, J = 16.1 Hz, 1H), 7.26 (t, J = 9.3 Hz, 2H), 6.51 (d, J = 16.1 Hz, 1H), 4.66 (dd, J = 13.8, 6.8 Hz, 1H), 4.15 (dd, J = 14.1, 7.0 Hz, 2H), 3.22 (dd, J = 16.1, 7.7 Hz, 2H), 2.86 (dd, J = 15.8, 6.3 Hz, 2H), 1.22 (t, J = 7.1 Hz, 3H); LCMS m/z 346 [M+H]⁺

### Step 2: (E)-3-(2-((5,6-difluoro-2,3-dihydro-1H-inden-2-yl)amino)pyrimidin-5-yl)acrylic acid

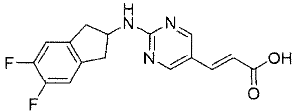

To a mixturer of ethyl (E)-3-(2-((5,6-difluoro-2,3-dihydro-1H-inden-2-yl)amino)pyrimidin-5-yl)acrylate (0.345 g, 0.999 mmol) in MeOH/THF (2 mL/20 mL) was added a solution of LiOH (0.119 g, 4.99 mmol) in DW (2 mL) and the mixture was stirred at room temperature overnight. After concentrating the reaction mixture, DW was added, pH was adjusted to 1-2 with 3 M HCl, and extracted with EtOAc. The organic layer was washed with DW, dried over Na₂SO₄, filtered, and concentrated. The obtained residue was purified by column chromatography (MeOH/DCM) to give the title compound as an off-white solid (0.147 g, 46.4%).

¹H NMR (400 MHz, DMSO-d₆) *δ* 12.17 (s, 1H), 8.64 (s, 1H), 8.05 (d, *J =* 7.2 Hz, 1H), 7.41 (d, *J* = 16.0 Hz, 1H), 7.26 (t, *J* = 9.3 Hz, 2H), 6.41 (d, *J =* 16.2 Hz, 1H), 4.66 (dd, *J* = 14.0, 6.8 Hz, 1H), 3.26 - 3.19 (m, 2H), 2.86 (dd, *J =* 16.1, 6.6 Hz, 2H); LCMS m/z 318 [M+H]⁺

### Step 3: (E)-(4-(1-(3-(2-((5,6-difluoro-2,3-dihydro-1H-inden-2-yl)amino)pyrimidin-5-yl)acryloyl)pyrrolidin-3-yl)-1H-1,2,3-triazol-1-yl)methyl pivalate

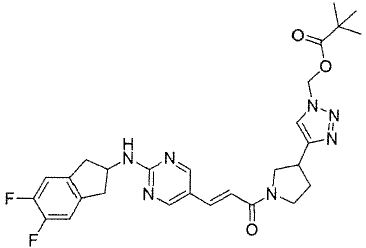

To a mixture of (E)-3-(2-((5,6-difluoro-2,3-dihydro-1H-inden-2-yl)amino)pyrimidin-5-yl)acrylic acid **1** (40 mg, 0.126 mmol), (4-(pyrrolidin-3- yl)-1H-1,2,3-triazol-1-yl)methyl pivalate, 2,2,2-trifluoroacetate ((52.8 mg, 0.151 mmol), DIPEA (64 µL, 0.378 mmol) and DMF (2.5 mL), HBTU (57.4 mg, 0.151 mmol) was added and the mixture was stirred at room temperature for 2 h. After concentrating the reaction mixture, the obtained residue was purified by column chromatography (MeOH/DCM) to give the title compound as a yellow solid (42.7 mg, 61.4%).

LCMS m/z 552 [M+H]⁺

### Step 4: (E)-1-(3-(1H-1,2,3-triazol-4-yl)pyrrolidin-1-yl)-3-(2-((5,6-difluoro-2,3-dihydro-1H-inden-2-yl)amino)pyrimidin-5-yl)prop-2-en-1-one

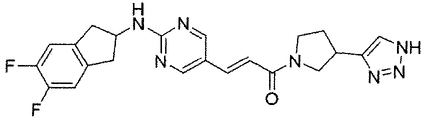

By the same method as in Example 3, (E)-(4-(1-(3-(2-((5,6-difluoro-2,3-dihydro-1H-inden-2-yl)amino)pyrimidin-5-yl)acryloyl)pyrrolidin-3-yl)-1H-1,2,3-triazol-1-yl)methyl pivalate (40 mg, 0.073 mmol), K₂CO₃ (20.0 mg, 0.145 mmol), and MeOH (0.36 mL) were used to obtain the title compound as a white solid (27.2 mg, 85.7%).

¹H NMR (400 MHz, DMSO-d₆) *δ* 8.67 (s, 2H), 7.98 (s, 1H), 7.70 (s, 1H), 7.34 (s, 1H), 7.27 (dd, J = 16.7, 7.6 Hz, 2H), 6.91 (dd, J = 16.0, 9.6 Hz, 1H), 4.65 (dd, J = 13.6, 6.8 Hz, 1H), 4.08 (s, 1H), 3.81 (dd, J = 12.6, 9.8 Hz, 2H), 3.65 (dd, J = 24.1, 15.4 Hz, 2H), 3.48 (s, 2H), 3.22 (dd, J = 15.2, 7.5 Hz, 2H), 2.86 (dd, J = 15.4, 4.6 Hz, 2H); LCMS m/z 438 [M+H]⁺

### Example 10. 1-(3-(1H-1,2,3-triazol-4-yl)pyrrolidin-1-yl)-3-(2-((5,6-difluoro-2,3-dihydro-1H-inden-2-yl)amino)pyrimidin-5-yl)propan-1-one

### Step 1: ethyl 3-(2-((5,6-difluoro-2,3-dihydro-1H-inden-2-yl)amino)pyrimidin-5-yl)propanoate

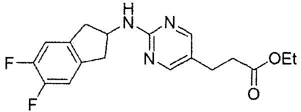

To a mixture of ethyl (E)-3-(2-((5,6-difluoro-2,3-dihydro-1H-inden-2-yl)amino)pyrimidin-5-yl)acrylate (0.3 g, 0.869 mmol) and EA/MeOH/THF (14 mL/28 mL/14 mL), Pd(OH)₂ (0.146 g, 1.04 mmol) was added and the mixture was stirred under an atmosphere of H₂ overnight (room temperature). The reaction mixture was filtered through a pad of Celite and concentrated, and the resulting residue was purified by column chromatography (EtOAc/Hex) to give the title compound as a white solid (0.115 g, 38.2%).

¹H NMR (400 MHz, DMSO-d₆) *δ* 8.17 (s, 2H), 7.31 (d, J = 6.7 Hz, 1H), 7.24 (t, J = 9.2 Hz, 2H), 4.61 - 4.52 (m, 1H), 4.02 (q, J = 7.1 Hz, 2H), 3.18 (dd, J = 15.7, 7.4 Hz, 2H), 2.81 (dd, J = 16.1, 6.8 Hz, 2H), 2.59 (dt, J = 32.2, 7.2 Hz, 4H), 1.14 (t, J = 7.1 Hz, 3H); LCMS m/z 348 [M+H]⁺

### Step 2: 3-(2-((5,6-difluoro-2,3-dihydro-1H-inden-2-yl)amino)pyrimidin-5-yl)propanoic acid

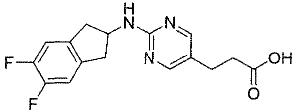

By the same method as in step 2 of example 9, ethyl 3-(2-((5,6-difluoro-2,3-dihydro-1H-inden-2-yl)amino)pyrimidin-5-yl)propanoate (113 mg, 0.325 mmol), LiOH (39 mg, 1.63 mmol), DW (0.7 mL) and MeOH/THF (0.7 mL/7 mL) were used to obtain the title compound as a white solid (75.6 mg, 72.8%).

¹H NMR (400 MHz, DMSO-d₆) *δ* 12.12 (bs, 1H), 8.17 (s, 2H), 7.30 (d, J = 6.6 Hz, 1H), 7.24 (t, J = 9.3 Hz, 2H), 4.61 - 4.51 (m, 1H), 3.19 (dd, J = 15.9, 7.7 Hz, 2H), 2.81 (dd, J = 15.9, 6.8 Hz, 2H), 2.60 (t, J = 7.3 Hz, 2H), 2.46 (d, J = 7.8 Hz, 2H); LCMS m/z 320 [M+H]⁺

### Step 3: (4-(1-(3-(2-((5,6-difluoro-2,3-dihydro-1H-inden-2-yl)amino)pyrimidin-5-yl)propanoyl)pyrrolidin-3-yl)-1H-1,2,3-triazol-1-yl)methyl pivalate

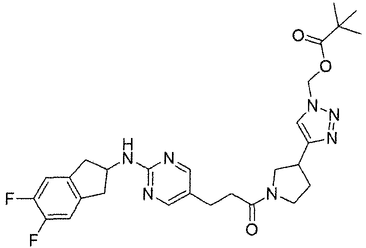

By the same method as in step 3 of Example 9, 3-(2-((5,6-difluoro-2,3-dihydro-1H-inden-2-yl)amino)pyrimidin-5-yl)propanoic acid (16 mg, 0.050 mmol), (4-(pyrrolidin-3-yl)-1H-1,2,3-triazol-1-yl)methyl pivalate, 2,2,2-trifluoroacetate (21 mg, 0.060 mmol), DIPEA (26 µL, 0.150 mmol), HBTU (22.8 mg, 0.060 mmol), and DMF (1 mL) was used to obtain the title compound as a yellow solid (24 mg, 86.5%).

LCMS m/z 554 [M+H]⁺

### Step 4: 1-(3-(1H-1,2,3-triazol-4-yl)pyrrolidin-1-yl)-3-(2-((5,6-difluoro-2,3-dihydro-1H-inden-2-yl)amino)pyrimidin-5-yl)propan-1-one

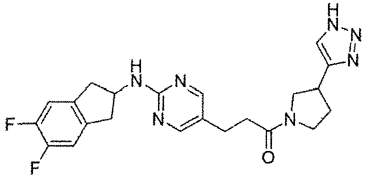

By the same method as in Example 3, (4-(1-(3-(2-((5,6-difluoro-2,3-dihydro-1H-inden-2-yl)amino)pyrimidin-5-yl)propanoyl)pyrrolidin-3-yl)-1H-1,2,3-triazol-1-yl)methyl pivalate (20 mg, 0.036 mmol), K₂CO₃ (10 mg, 0.072 mmol), and MeOH (0.18 mL) were used to obtain the title compound as a yellow solid (8.7 mg, 54.8%).

¹H NMR (400 MHz, DMSO-d₆) *δ* 8.19 (d, J = 1.4 Hz, 2H), 7.72 (s, 1H), 7.29 - 7.22 (m, 2H), 4.56 (dd, J = 13.9, 7.4 Hz, 1H), 3.86 - 3.70 (m, 1H), 3.58 - 3.39 (m, 3H), 3.28 (d, J = 7.8 Hz, 1H), 3.21 - 3.13 (m, 2H), 2.81 (dd, J = 15.9, 6.4 Hz, 2H), 2.61 (t, J = 7.2 Hz, 2H), 2.23 (dd, J = 31.5, 6.1 Hz, 2H), 2.06 - 1.84 (m, 2H); LCMS m/z 440 [M+H]⁺

### Example 11. 5-(5-(3-(1H-1,2,3-triazol-4-yl)pyrrolidin-1-yl)-1,3,4-oxadiazol-2-yl)-N-(3,5-dichlorophenethyl)pyrimidin-2-amine

### Step 1: 5-(2-((3,5-dichlorophenethyl)amino)pyrimidin-5-yl)-1,3,4-oxadiazol-2(3H)-one

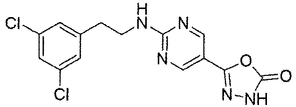

The title compound was prepared by the same method as in Preparation Example 3, except that 2-(3,5-dichlorophenyl)ethan-1-amine was used instead of 2,3-dihydro-1H-inden-2-amine.

¹H NMR (400 MHz, DMSO-d₆) δ12.40 (broad, 1H), 8.62 (d, *J* = 9.6 Hz, 2H), 8.07 (t, *J =* 5.7 Hz, 1H), 7.42 (s, 1H), 7.31 (d, *J =* 1.6 Hz, 2H), 3.58 (dd, *J =* 12.8, 6.6 Hz, 2H), 2.87 (t, *J = 6.8* Hz, 2H); LCMS m/z 352 [M+H]⁺

### Step 2: (4-(1-(5-(2-((3,5-dichlorophenethyl)amino)pyrimidin-5-yl)-1,3,4-oxadiazol-2-yl)pyrrolidin-3-yl)-1H-1,2,3-triazol-1-yl)methyl pivalate

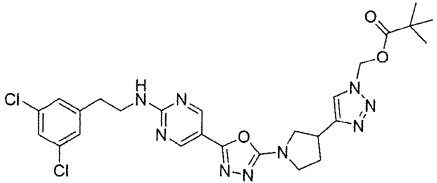

By the same method as in Step 1 of Example 1, 5-(2-((3,5-dichlorophenethyl)amino)pyrimidin-5-yl)-1,3,4-oxadiazol-2(3H)-one (25.6 mg, 0.07 mmol), (4-(pyrrolidin-3-yl)-1H-1,2,3-triazol-1-yl)methyl pivalate, 2,2,2-trifluoroacetate (32 mg, 0.09 mmol), DIPEA (62 uL, 0.36 mmol), BOP reagent (38.7 mg, 0.087 mmol), and DMF (2 mL) were used to obtain the title compound as a white solid (36.8 mg, yield 86.2%).

LCMS m/z 530 [M+H]⁺

### Step 3: 5-(5-(3-(1H-1,2,3-triazol-4-yl)pyrrolidin-1-yl)-1,3,4-oxadiazol-2-yl)-N-(3,5-dichlorophenethyl)pyrimidin-2-amine

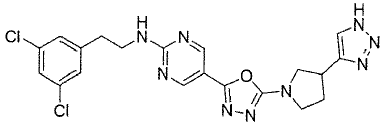

By the same method as in Step 2 of Example 1, (4-(1-(5-(2-((3,5 - Dichlorophenethyl)amino)pyrimidin-5-yl)-1,3,4-oxadiazol-2-yl)pyrrolidin-3-yl)-1H-1,2,3-triazol-1-yl)methyl pivalate (34.7 mg, 0.059 mmol), K₂CO₃ (16.4 mg, 0.12 mmol), and MeOH (2 mL) were used to obtain the title compound as a white solid (11.8 mg, 42.2%).

¹H NMR (400 MHz, DMSO-d₆) *δ* 8.70 (d, J = 7.4 Hz, 2H), 7.98 (t, J = 5.7 Hz, 1H), 7.82 (broad, 1H), 7.43 (s, 1H), 7.32 (d, J = 1.6 Hz, 2H), 3.97 - 3.88 (m, 1H), 3.74 - 3.54 (m, 6H), 2.88 (t, J = 6.8 Hz, 2H), 2.47 - 2.35 (m, 1H), 2.25 - 2.10 (m, 1H); LCMS m/z 472 [M+H]⁺

### Example 12. (4-(1-(5-(2-((5-methoxy-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

### Step 1: 5-(2-((5-methoxy-2,3-dihydro-1H-inden-2-yl)amino)pyrimidin-5-yl)-1,3,4-oxadiazol-2(3H)-one

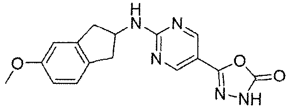

The title compound was prepared by the same method as in Preparation Example 3, except that 5-methoxy-2,3-dihydro-1H-inden-2-amine was used instead of 2,3-dihydro-1H-inden-2-amine.

¹H NMR (400 MHz, DMSO-d₆) δ8.65 (d, *J* = 16.3 Hz, 2H), 8.30 (d, *J* = 7.0 Hz, 1H), 7.10 (d, *J* = 8.2 Hz, 1H), 6.81 (s, 1H), 6.71 (dd, *J* = 8.2, 2.2 Hz, 1H), 4.67 (dd, *J =* 14.3, 7.2 Hz, 1H), 3.71 (s, 3H), 3.21 (dd, *J =* 16.7, 9.0 Hz, 3H), 2.92 - 2.79 (m, 2H); LCMS m/z 326 [M+H]⁺

### Step 2: (4-(1-(5-(2-((5-methoxy-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

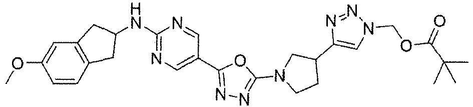

By the same method as in Step 1 of Example 1, 5-(2-((5-methoxy-2,3-dihydro-1H-inden-2-yl)amino)pyrimidin-5-yl)-1,3,4-oxadiazol-2(3H)-one (26 mg, 0.080 mmol), (4-(pyrrolidin-3-yl)-1H-1,2,3-triazol-1-yl)methyl pivalate, 2,2,2-trifluoroacetate (34 mg, 0.096 mmol), DIPEA (68 µL, 0.096 mmol), BOP reagent (42 mg, 0.096 mmol), and DMF (1 mL) were used to obtain the title compound as a white solid (17 mg, 38%).

¹H NMR (400 MHz, DMSO-d₆) δ8.74 (d, *J* = 12.3 Hz, 2H), 8.26 - 8.17 (m, 2H), 7.11 (d, *J* = 8.1 Hz, 1H), 6.81 (s, 1H), 6.72 (d, *J =* 8.2 Hz, 1H), 6.28 (s, 2H), 4.73 - 4.62 (m, 1H), 3.98 - 3.89 (m, 1H), 3.76 - 3.67 (m, 4H), 3.66 - 3.56 (m, 3H), 3.21 (td, *J* = 16.3, 7.5 Hz, 2H), 2.93 - 2.80 (m, 2H), 2.46 - 2.37 (m, 1H), 2.22 - 2.14 (m, 1H), 1.12 (s, 9H); LCMS m/z 560 [M+H]⁺

### Example 13. 5-(5-(3-(1H-1,2,3-triazol-4-yl)pyrrolidin-1-yl)-1,3,4-oxadiazol-2-yl)-N-(5-methoxy-2,3-dihydro-1H-inden-2-yl)pyrimidin-2-amine

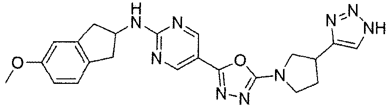

By the same method as in Step 2 of Example 1, (4-(1-(5-(2-((5-methoxy-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (11 mg, 0.020 mmol), K₂CO₃ (5.0 mg, 0.39 mmol), and MeOH (1 mL) were used to obtain the title compound as a white solid (2.7 mg, 31%).

¹H NMR (400 MHz, DMSO-d₆) δ8.73 (d, *J* = 15.3 Hz, 2H), 8.22 (d, *J* = 7.0 Hz, 1H), 7.81 (s, 1H), 7.11 (d, *J =* 8.3 Hz, 1H), 6.81 (s, 1H), 6.72 (d, *J =* 8.2 Hz, 1H), 4.73 - 4.62 (m, 1H), 3.96 - 3.89 (m, 1H), 3.75 - 3.57 (m, 7H), 3.25 - 3.16 (m, 2H), 2.95 - 2.80 (m, 2H), 2.43 - 2.36 (m, 1H), 2.21 - 2.12 (m, 1H) ; LCMS m/z 446 [M+H]⁺

### Example 14. 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrazin-2-amine

### Step 1: 5-(5-((5,6-difluoro-2,3-dihydro-1H-inden-2-yl)amino)pyrazin-2-yl)-1,3,4-oxadiazol-2(3H)-one

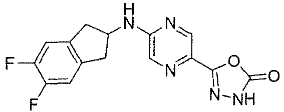

The title compound was prepared by the same method as Preparation Example 3, except that 5,6-difluoro-2,3-dihydro-1H-inden-2-amine was used instead of 2,3-dihydro-1H-inden-2-amine and ethyl 5-chloropyrimidine-5-carboxylate was used instead of ethyl 2-chloropyrimidine-5-carboxylate.

LCMS m/z 332 [M+H]⁺

### Step 2: (4-(1-(5-(5-((5,6-difluoro-2,3-dihydro-1H-inden-2-yl)amino)pyrazin-2-yl)-1,3,4-oxadiazol-2-yl)pyrrolidin-3-yl)-1H-1,2,3-triazol-1-yl)methyl pivalate

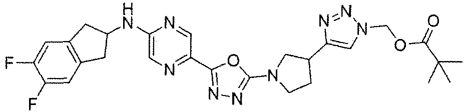

By the same method as in Step 1 of Example 1, 5-(5-((5,6-difluoro-2,3-dihydro-1H-inden-2-yl)amino)pyrazin-2-yl)-1,3,4-oxadiazol-2(3H)-one (24 mg, 0.07 mmol), (4-(pyrrolidin-3-yl)-1H-1,2,3-triazol-1-yl)methyl pivalate, 2,2,2-trifluoroacetate (32 mg, 0.09 mmol), DIPEA (62 uL, 0.36 mmol), BOP reagent (39 mg, 0.087 mmol), and DMF (2 mL) were used to obtain the title compound as a brown solid (28.8 mg, 69.9%).

LCMS m/z 566 [M+H]⁺

### Step 3: 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrazin-2-amine

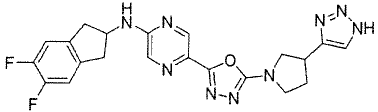

By the same method as in Step 2 of Example 1, (4-(1-(5-(5-((5,6-difluoro-2,3-dihydro-1H-inden-2-yl)amino)pyrazin-2-yl)-1,3,4-oxadiazol-2-yl)pyrrolidin-3-yl)-1H-1,2,3-triazol-1-yl)methyl pivalate (27.5 mg, 0.049 mmol), K₂CO₃ (13.4 mg, 0.097 mmol), and MeOH (2 mL) were used to obtain the title compound as a white solid (3.95 mg, 18%).

¹H NMR (400 MHz, CD₃OD) *δ* 8.59 (s, 1H), 7.94 (s, 1H), 7.74 (s, 1H), 7.11 (t, J = 8.8 Hz, 2H), 4.82 - 4.74 (m, 1H), 4.09 - 4.01 (m, 1H), 3.86 - 3.68 (m, 4H), 3.41 - 3.34 (m, 2H), 2.90 (dd, J = 15.9, 5.4 Hz, 2H), 2.56 - 2.46 (m, 1H), 2.36 - 2.25 (m, 1H); LCMS m/z 452 [M+H]⁺

### Example 15. methyl 1-(5-(2-((5,6-difluoro-2,3-dihydro-1H-inden-2-yl)amino)pyrimidin-5-yl)-1,3,4-oxadiazol-2-yl)pyrrolidine-3-carboxylate

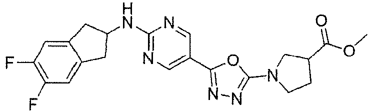

To a solution of 5-(2-((5,6-difluoro-2,3-dihydro-1H-inden-2-yl)amino)pyrimidin-5-yl)-1,3,4-oxadiazol-2(3H)-one (60 mg, 0.181 mmol) in DMF (0.9 mL) was added methyl 3-pyrrolidinecarboxylate hydrochloride (36.0 mg, 0.217 mmol), followed by DIPEA (92 µL, 0.543 mmol) at 0 °C. After stirring at the same temperature for 30 min, BOP reagent (96.1 mg, 0.217 mmol) was added, and the mixture was stirred at room temperature overnight. DW was added to the reaction mixture, extracted with EtOAc, and the organic layer was washed with brine, dried over Na₂SO₄, filtered, and concentrated. The obtained residue was purified by column chromatography (MeOH/DCM) to obtain the title compound as a white solid (45.4 mg, 56.7%).

¹H NMR (400 MHz, DMSO-d₆) *δ* 8.73 (s, 2H), 8.24 (d, J = 6.4 Hz, 1H), 7.27 (t, J = 9.1 Hz, 2H), 4.68 (d, J = 6.6 Hz, 1H), 3.75 - 3.59 (m, 5H), 3.52 (s, 2H), 3.24 (dd, J = 16.0, 7.6 Hz, 2H), 2.87 (dd, J = 16.0, 6.4 Hz, 2H), 2.20 (d, J = 32.5 Hz, 2H). LCMS m/z 443 [M+H]⁺

### Example 16. 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)-4-methylpyrimidin-2-amine

### Step 1: 5-(2-((5,6-difluoro-2,3-dihydro-1H-inden-2-yl)amino)-4-methylpyrimidin-5-yl)-1,3,4-oxadiazol-2(3H)-one

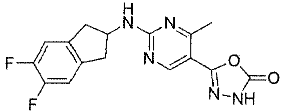

The title compound was prepared by the same method as in Preparation Example 3, except that 5,6-difluoro-2,3-dihydro-1H-inden-2-amine was used instead of 2,3-dihydro-1H-inden-2-amine and ethyl 2-chloro-4-methylpyrimidine-5-carboxylate was used instead of ethyl 2-chloropyrimidine-5-carboxylate.

¹H NMR (400 MHz, DMSO-d₆) δ12.30 (broad, 1H), 8.58 - 8.47 (m, 1H), 8.28 - 8.15 (m, 1H), 7.28 (t, *J* = 9.2 Hz, 6H), 4.71 (s, 4H), 3.29 - 3.19 (m, 2H), 2.88 (dd, *J =* 15.9, 6.6 Hz, 2H), 2.54 (s, 3H); LCMS m/z 346 [M+H]⁺

### Step 2: (4-(1-(5-(2-((5,6-difluoro-2,3-dihydro-1H-inden-2-yl)amino)-4-methylpyrimidin-5-yl)-1,3,4-oxadiazol-2-yl)pyrrolidin-3-yl)-1H-1,2,3-triazol-1-yl)methyl pivalate

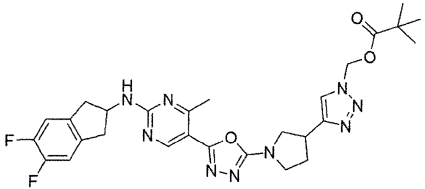

By the same method as in Step 1 of Example 1, 5-(2-((5,6-difluoro-2,3-dihydro-1H-inden-2-yl)amino)-4-methylpyrimidin-5-yl)-1,3,4-oxadiazol-2(3H)-one (25 mg, 0.07 mmol), (4-(pyrrolidin-3-yl)-1H-1,2,3-triazol-1-yl)methyl pivalate, 2,2,2-trifluoroacetate (32 mg, 0.09 mmol), DIPEA (62 uL, 0.36 mmol), BOP reagent (38 mg, 0.087 mmol), and DMF (2 mL) were used to obtain the title compound as a brown solid (30.9 mg, 73.7%).

LCMS m/z 580 [M+H]⁺

### Step 3: 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)-4-methylpyrimidin-2-amine

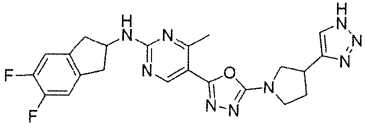

By the same method as in Step 2 of Example 1, (4-(1-(5-(2-((5,6-difluoro-2,3-dihydro-1H-inden-2-yl)amino)-4-methylpyrimidin-5-yl)-1,3,4-oxadiazol-2-yl)pyrrolidin-3-yl)-1H-1,2,3-triazol-1-yl)methyl pivalate (29.5 mg, 0.051 mmol), K₂CO₃ (14.1 mg, 0.10 mmol), and MeOH (2 mL) were used to obtain the title compound as a white solid (17 mg, 71.8%).

¹H NMR (400 MHz, DMSO-d₆) *δ* 8.62 (broad, 1H), 8.10 (s, 1H), 7.82 (s, 1H), 7.28 (t, J = 9.3 Hz, 2H), 4.71 (s, 1H), 3.98 - 3.88 (m, 1H), 3.75 - 3.55 (m, 4H), 3.25 (dd, J = 15.9, 7.2 Hz, 2H), 2.88 (dd, J = 15.9, 6.7 Hz, 2H), 2.57 (s, 3H), 2.46 - 2.35 (m, 1H), 2.24 - 2.10 (m, 1H); LCMS m/z 466 [M+H]⁺

### Example 17. 5-(3-(3-(1H-1,2,3-triazol-4-yl)pyrrolidin-1-yl)-4,5-dihydroisoxazol-5-yl)-N-(5,6-difluoro-2,3-dihydro-1H-inden-2-yl)pyrimidin-2-amine

### Step 1: N-(5,6-difluoro-2,3-dihydro-1H-inden-2-yl)-5-vinylpyrimidin-2-amine

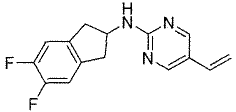

A mixture of 5,6-difluoro-2,3-dihydro-1H-inden-2-amine (0.5 g, 2.96 mmol), 2-chloro-5-vinylpyrimidine (0.831 g, 5.91 mmol), DIPEA (10.1 mL, 59.1 mmol) and n-BuOH (5.9 mL) was reacted in a microwave (160 °C, 1 h). After concentrating the reaction mixture, the obtained residue was purified by column chromatography (EA/Hex) to obtain the title compound as a brown solid (0.322 g, 39.7%).

¹H NMR (400 MHz, DMSO-d₆) *δ* 8.44 (s, 2H), 7.65 (d, J = 6.6 Hz, 1H), 7.25 (t, J = 9.3 Hz, 2H), 6.52 (dd, J = 17.8, 11.3 Hz, 1H), 5.70 (d, J = 17.4 Hz, 1H), 5.09 (d, J = 11.7 Hz, 1H), 4.67 - 4.57 (m, 1H), 3.21 (dd, J = 16.0, 7.5 Hz, 2H), 2.84 (dd, J = 15.8, 6.6 Hz, 2H); LCMS m/z 274 [M+H]⁺

### Step 2: 5-(3-bromo-4,5-dihydroisoxazol-5-yl)-N-(5,6-difluoro-2,3-dihydro-1H-inden-2-yl)pyrimidin-2-amine

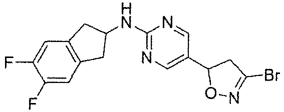

1,1-Dibromoformaldoxime (0.356 g, 1.76 mmol) was dissolved in DMF (2.9 mL) and cooled to -10 °C. N-(5,6-difluoro-2,3-dihydro-1H-inden-2-yl)-5-vinylpyrimidin-2-amine (0.320 g, 1.17 mmol) and KHCO₃ (0.293 g, 2.93 mmol) in DW (2.9 mL) were slowly added dropwise, and the mixture was stirred at room temperature for 1 hour. DW was added to the reaction mixture, extracted with EtOAc, and the organic layer was dried over MgSO₄, filtered and concentrated. The obtained residue was purified by column chromatography (EtOAc/Hex) to obtain the title compound as a brown solid (0.294 g, 63.5%).

¹H NMR (400 MHz, DMSO-d₆) *δ* 8.35 (s, 2H), 7.76 (d, J = 6.9 Hz, 1H), 7.25 (t, J = 9.2 Hz, 2H), 5.54 (t, J = 10.6 Hz, 1H), 4.62 (d, J = 7.0 Hz, 1H), 3.60 (dd, J = 17.3, 10.8 Hz, 1H), 3.47 (dd, J = 17.3, 10.5 Hz, 1H), 3.20 (dd, J = 15.9, 7.1 Hz, 2H), 2.83 (dd, J = 12.5, 4.1 Hz, 2H); LCMS m/z 396 [M+H]⁺

### Step 3: 5-(3-(3-(1H-1,2,3-triazol-4-yl)pyrrolidin-1-yl)-4,5-dihydroisoxazol-5-yl)-N-(5,6-difluoro-2,3-dihydro-1H-inden-2-yl)pyrimidin-2-amine

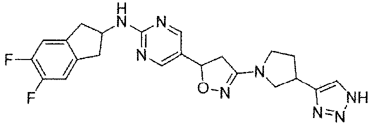

A mixture of 5-(3-bromo-4,5-dihydroisoxazol-5-yl)-N-(5,6-difluoro-2,3-dihydro-1H-inden-2-yl)pyrimidin-2-amine (55 mg, 0.139 mmol), 4-(pyrrolidin-3-yl)-1H-1,2,3-triazole, 2,2,2-trifluoroacetate (39.4 mg, 0.167 mmol), Na₂CO₃ (36.7 mg, 0.348 mmol) and t-BuOH (1.4 mL) was reacted in a microwave (160 °C, 2 h). The reaction mixture was filtered, washed with 10% MeOH/DCM solution, and the filtrate was concentrated. The obtained residue was purified by column chromatography (MeOH/DCM) to give the title compound as an orange solid (15.8 mg, 25.1%).

¹H NMR (400 MHz, CDCl₃) *δ* 8.36 (s, 2H), 7.53 (s, 1H), 7.00 (t, J = 8.7 Hz, 2H), 5.75 (t, J = 6.3 Hz, 1H), 5.39 (t, J = 8.8 Hz, 1H), 4.81 (dd, J = 12.6, 5.7 Hz, 1H), 3.77 (dd, J = 15.6, 6.6 Hz, 1H), 3.67 - 3.50 (m, 3H), 3.43 - 3.29 (m, 3H), 3.06 (dd, J = 14.9, 8.1 Hz, 1H), 2.83 (dd, J = 16.1, 5.0 Hz, 2H), 2.40 (dd, J = 12.4, 5.8 Hz, 1H), 2.17 (dd, J = 13.2, 7.1 Hz, 1H); LCMS m/z 453 [M+H]⁺

### Example 18. 5-(3-(3-(1H-1,2,3-triazol-4-yl)pyrrolidin-1-yl)isoxazol-5-yl)-N-(5,6-difluoro-2,3-dihydro-1H-inden-2-yl)pyrimidin-2-amine

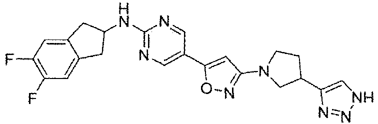

A mixture of 5-(3-(3-(1H-1,2,3-triazol-4-yl)pyrrolidin-1-yl)-4,5-dihydroisoxazol-5-yl)-N-(5,6-difluoro-2,3-dihydro-1H-inden-2-yl)pyrimidin-2-amine (10 mg, 0.022 mmol), I₂ (8.4 mg, 0.033 mmol), imidazole (4.5 mg, 0.066 mmol) and toluene (1.1 mL) was stirred at 110 °C for 3 h. EtOAc and 10% Na₂S₂O₄ solution were added to the reaction mixture, and the mixture was stirred at room temperature for 30 min. The organic layer was separated and extracted with EtOAc. The organic layers were combined, washed with brine, dried over MgSO₄, filtered and concentrated. The obtained residue was purified by column chromatography (MeOH/DCM) to obtain the title compound as a white solid (4.2 mg, 42.2%).

¹H NMR (400 MHz, DMSO-d6) *δ* 8.68 (s, 2H), 8.13 (d, J = 6.7 Hz, 1H), 7.27 (t, J = 9.3 Hz, 2H), 6.51 (s, 1H), 4.67 (dd, J = 13.8, 7.0 Hz, 1H), 3.68 (d, J = 8.4 Hz, 1H), 3.39 (dd, J = 15.0, 7.8 Hz, 2H), 3.23 (dd, J = 16.1, 7.5 Hz, 2H), 2.87 (dd, J = 16.1, 6.5 Hz, 2H), 2.35 (d, J = 6.3 Hz, 2H), 2.05 (d, J = 48.6 Hz, 2H); LCMS m/z 451 [M+H]⁺

### Example 19. 5-(5-(3-(1H-1,2,3-triazol-4-yl)pyrrolidin-1-yl)-1,3,4-oxadiazol-2-yl)-N-(5-bromo-2,3-dihydro-1H-inden-2-yl)pyrimidin-2-amine

### Step 1: 5-(2-((5-bromo-2,3-dihydro-1H-inden-2-yl)amino)pyrimidin-5-yl)-1,3,4-oxadiazol-2(3H)-one

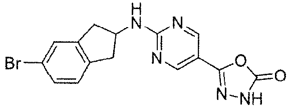

The title compound was prepared by the same method as in Preparation Example 3, except that 5-bromo-2,3-dihydro-1H-inden-2-amine was used instead of 2,3-dihydro-1H-inden-2-amine.

¹H NMR (400 MHz, DMSO-d₆) *δ* 12.46 (s, 1H), 8.66 (d, J = 13.9 Hz, 2H), 8.33 (d, J = 6.8 Hz, 1H), 7.43 (s, 1H), 7.33 (d, J = 8.0 Hz, 1H), 7.19 (d, J = 8.1 Hz, 1H), 4.73 - 4.63 (m, 1H), 3.28 - 3.20 (m, 2H), 2.90 (ddd, J = 22.4, 16.2, 6.5 Hz, 2H); LCMS m/z 374 [M+H]⁺

### Step 2: (4-(1-(5-(2-((5-bromo-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

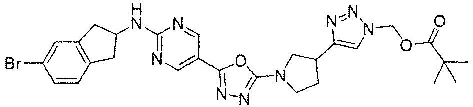

By the same method as in Step 1 of Example 1, 5-(2-((5-bromo-2,3-dihydro-1H-inden-2-yl)amino)pyrimidin-5-yl)-1,3,4-oxadiazol-2(3H)-one (37 mg, 0.1 mmol), (4-(pyrrolidin-3-yl)-1H-1,2,3-triazol-1-yl)methyl pivalate, 2,2,2-trifluoroacetate (45 mg, 0.13 mmol), DIPEA (87 uL, 0.5 mmol), BOP reagent (52 mg, 0.12 mmol), and DMF (2 mL) were used to obtain the title compound as a yellow solid (50 mg, 82%).

LCMS m/z 608, 610 [M+H]⁺

### Step 3: 5-(5-(3-(1H-1,2,3-triazol-4-yl)pyrrolidin-1-yl)-1,3,4-oxadiazol-2-yl)-N-(5-bromo-2,3-dihydro-1H-inden-2-yl)pyrimidin-2-amine

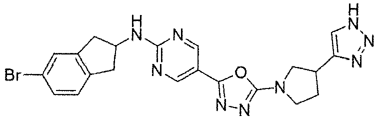

By the same method as in step 2 of example 1, (4-(1-(5-(2-((5-bromo-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (50 mg, 0.08 mmol), K₂CO₃ (22 mg, 0.16 mmol), and MeOH (1 mL) were used to obtain the title compound as a white solid (25 mg, 63%).

¹H NMR (400 MHz, CD₃OD) *δ* 8.71 (s, 2H), 8.23 (d, J = 8.0 Hz, 1H), 7.73 (s, 1H), 7.41 (s, 1H), 7.32 (d, J = 8.0 Hz, 2H), 7.18 (d, J = 8.0 Hz, 2H), 4.68 - 4.63 (m, 1H), 3.91 - 3.88 (m, 1H), 3.69 - 3.59 (m, 4H), 3.28 - 3.18 (m, 2H), 2.94 - 2.83 (m, 2H), 2.39 - 2.37 (m, 1H), 2.16 - 2.14 (m, 1H); LCMS m/z 494, 496 [M+H]⁺

### Example 20. (E)-1-(3-(1H-1,2,3-triazol-4-yl)pyrrolidin-1-yl)-3-(2-((2,3-dihydro-1H-inden-2-yl)amino)pyrimidin-5-yl)prop-2-en-1-one

### Step 1: (E)-3-(2-((2,3-dihydro-1H-inden-2-yl)amino)pyrimidin-5-yl)acrylic acid

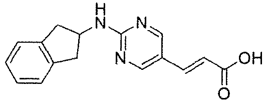

The title compound was prepared by the same method as in Steps 1 and 2 of Example 9, except that 2,3-dihydro-1H-inden-2-amine was used instead of 5,6-difluoro-2,3-dihydro-1H-inden-2-amine in Step 1 of Example 9.

LCMS m/z 282 [M+H]⁺

### Step 2: (E)-(4-(1-(3-(2-((2,3-dihydro-1H-inden-2-yl)amino)pyrimidin-5-yl)acryloyl)pyrrolidin-3-yl)-1H-1,2,3-triazol-1-yl)methyl pivalate

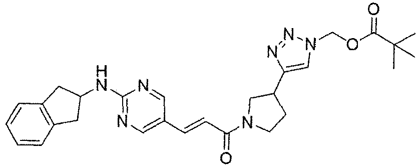

(E)-3-(2-((2,3-Dihydro-1H-inden-2-yl)amino)pyrimidin-5-yl)acrylic acid (27 mg, 0.095 mmol) and (4-(pyrrolidin-3-yl)-1H-1,2,3-triazol-1-yl)methyl pivalate, 2,2,2-trifluoroacetate (41.7 mg, 0.11 mmol) were dissolved in DMF (2 mL), DIPEA (49 uL, 0.28 mmol) and HBTU (43 mg, 0.11 mmol) were added, and the mixture was stirred at room temperature for 5 h. DW was added to the reaction mixture, extracted with EtOAc, and the organic layer was washed with brine. The organic layer was dried over MgSO₄, filtered, concentrated, and the resulting residue was purified by column chromatography (0-100% EtOAc/Hex) to obtain the title compound as a white solid (30.8 mg, 63%).

LCMS m/z 516 [M+H]⁺

### Step 3: (E)-1-(3-(1H-1,2,3-triazol-4-yl)pyrrolidin-1-yl)-3-(2-((2,3-dihydro-1H-inden-2-yl)amino)pyrimidin-5-yl)prop-2-en-1-one

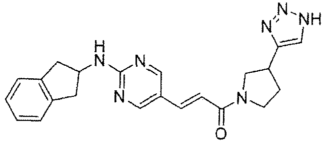

By the same method as in Step 2 of Example 1, (E)-(4-(1-(3-(2-((2,3-dihydro-1H-inden-2-yl)amino)pyrimidin-5-yl)acryloyl)pyrrolidin-3-yl)-1H-1,2,3-triazol-1-yl)methyl pivalate (28.6 mg, 0.056 mmol), K₂CO₃ (15.3 mg, 0.11 mmol) and MeOH (2 mL) were used to obtain the title compound as a white solid (11.4 mg, yield 51.2%).

¹H NMR (400 MHz, DMSO-d₆) *δ* 8.69 (s, 2H), 7.97 (dd, J = 6.4, 3.4 Hz, 1H), 7.78 (broad, 1H), 7.35 (dd, J = 15.5, 3.6 Hz, 1H), 7.26 - 7.07 (m, 4H), 6.92 (dd, J = 15.5, 9.0 Hz, 1H), 4.72 - 4.60 (m, 1H), 4.18 - 4.04 (m, 1H), 3.92 - 3.77 (m, 1H), 3.75 - 3.56 (m, 2H), 3.56 - 3.40 (m, 2H), 3.25 (dd, J = 16.0, 7.8 Hz, 2H), 2.91 (dd, J = 15.7, 4.7 Hz, 2H), 2.41 - 2.22 (m, 1H), 2.15 - 1.94 (m, 1H); LCMS m/z 402 [M+H]⁺

### Example 21. 5-(5-(3-(1H-1,2,3-triazol-4-yl)pyrrolidin-1-yl)-1,3,4-oxadiazol-2-yl)-N-(3,5-difluorobenzyl)pyrimidin-2-amine

### Step 1: 5-(2-((3,5-difluorobenzyl)amino)pyrimidin-5-yl)-1,3,4-oxadiazol-2(3H)-one

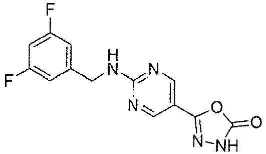

The title compound was prepared by the same method as in Preparation Example 3, except that (3,5-difluorophenyl)methanamine was used instead of 2,3-dihydro-1H-inden-2-amine.

LCMS m/z 306 [M+H]⁺

### Step 2: (4-(1-(5-(2-((3,5-difluorobenzyl)amino)pyrimidin-5-yl)-1,3,4-oxadiazol-2-yl)pyrrolidin-3-yl)-1H-1,2,3-triazol-1-yl)methyl pivalate

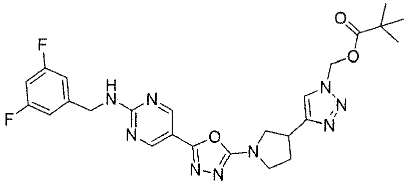

By the same method as in Step 1 of Example 1, 5-(2-((3,5-difluorobenzyl)amino)pyrimidin-5-yl)-1,3,4-oxadiazol-2(3H)-one (20.9 mg, 0.07 mmol), (4-(pyrrolidin-3-yl)-1H-1,2,3-triazol-1-yl)methyl pivalate, 2,2,2-trifluoroacetate (30.1 mg, 0.082 mmol), DIPEA (58 uL, 0.34 mmol), BOP reagent (37 mg, 0.082 mmol), and DMF (2 mL) were used to obtain the title compound as a brown solid (33.6 mg, 90.9%).

LCMS m/z 540 [M+H]⁺

### Step 3: 5-(5-(3-(1H-1,2,3-triazol-4-yl)pyrrolidin-1-yl)-1,3,4-oxadiazol-2-yl)-N-(3,5-difluorobenzyl)pyrimidin-2-amine

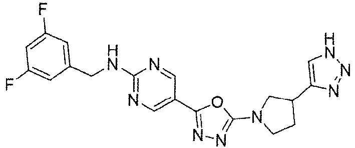

By the same method as in Step 2 of Example 1, using (4-(1-(5-(2-((3,5-difluorobenzyl)amino)pyrimidin-5-yl)-1,3,4-oxadiazol-2-yl)pyrrolidin-3-yl)-1H-1,2,3-triazol-1-yl)methyl pivalate (31.8 mg, 0.059 mmol), K₂CO₃ (16.3 mg, 0.12 mmol), and MeOH (2 mL), the title compound was obtained as a white solid (16.7 mg, 66.7%).

¹H NMR (400 MHz, DMSO-d₆) *δ* 8.73 (broad, 2H), 8.45 (t, J = 6.4 Hz, 1H), 7.81 (s, 1H), 7.09 (t, J = 9.3 Hz, 1H), 7.02 (d, J = 6.9 Hz, 2H), 4.58 (d, J = 6.2 Hz, 2H), 3.98 - 3.87 (m, 1H), 3.75 -3.54 (m, 4H), 2.45 - 2.34 (m, 1H), 2.23 - 2.08 (m, 1H); LCMS m/z 426 [M+H]⁺

### Example 22. 1-(5-(2-((5,6-difluoro-2,3-dihydro-1H-inden-2-yl)amino)pyrimidin-5-yl)-1,3,4-oxadiazol-2-yl)pyrrolidine-3-carboxylic acid

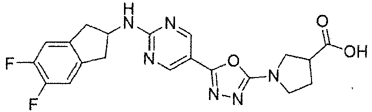

Methyl 1-(5-(2-((5,6-difluoro-2,3-dihydro-1H-inden-2-yl)amino)pyrimidin-5-yl)-1,3,4-oxadiazol-2-yl)pyrrolidine-3-carboxylate (39.4 mg, 0.089 mmol) was dissolved in THF (0.45 mL), 1 N NaOH (223 µL, 0.223 mmol) was added, and the mixture was stirred at room temperature for 1.5 h. After THF was concentrated and removed, EA was added, and the mixture was stirred for 1 h. The resulting solid was filtered to obtain the title compound as a white solid (36 mg, 94.4%).

¹H NMR (400 MHz, DMSO-d₆) *δ* 8.70 (s, 2H), 8.21 (d, J = 6.5 Hz, 1H), 7.27 (t, J = 9.2 Hz, 2H), 4.68 (dd, J = 13.1, 5.9 Hz, 1H), 3.68 - 3.60 (m, 1H), 3.47 - 3.39 (m, 2H), 3.24 (dd, J = 16.2, 7.8 Hz, 2H), 2.87 (dd, J = 16.1, 6.2 Hz, 2H), 2.65 (d, J = 6.5 Hz, 1H), 2.03 (d, J = 55.0 Hz, 3H); LCMS m/z 429 [M+H]⁺

### Example 23. 5-(1-(5-(2-((5,6-difluoro-2,3-dihydro-1H-inden-2-yl)amino)pyrimidin-5-yl)-1,3,4-oxadiazol-2-yl)pyrrolidin-3-yl)-1,3,4-oxadiazol-2(3H)-one

### Step 1: 1-(5-(2-((5,6-difluoro-2,3-dihydro-1H-inden-2-yl)amino)pyrimidin-5-yl)-1,3,4-oxadiazol-2-yl)pyrrolidine-3-carbohydrazide

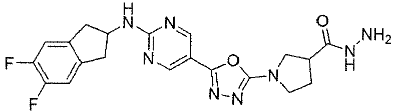

Methyl 1-(5-(2-((5,6-difluoro-2,3-dihydro-1H-inden-2-yl)amino)pyrimidin-5-yl)-1,3,4-oxadiazol-2-yl)pyrrolidine-3-carboxylate (53.5 mg, 0.12 mmol) was dissolved in EtOH (3 mL), hydrazine monohydrate (0.2 mL, 2.4 mmol) was added, and the mixture was stirred at 85 °C overnight. DW was added to the reaction mixture, and the mixture was stirred at 0 °C for 2 h. The resulting solid was filtered and dried to obtain the title compound as a white solid (43.8 mg, 81.9%).

LCMS m/z 443 [M+H]⁺

### Step 2: 5-(1-(5-(2-((5,6-difluoro-2,3-dihydro-1H-inden-2-yl)amino)pyrimidin-5-yl)-1,3,4-oxadiazol-2-yl)pyrrolidin-3-yl)-1,3,4-oxadiazol-2(3H)-one

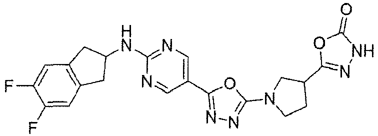

1-(5-(2-((5,6-difluoro-2,3-dihydro-1H-inden-2-yl)amino)pyrimidin-5-yl)-1,3,4-oxadiazol-2-yl)pyrrolidine-3-carbohydrazide (43.8 mg, 0.099 mmol) was dissolved in THF (2 mL), and TEA (14 µL, 0.099 mmol) and CDI (19.3 mg, 0.12 mmol) were slowly added at 0 °C, and the mixture was stirred at the same temperature for 20 min and then at room temperature for 20 min. The reaction mixture was concentrated, DW was added, extracted with EtOAc, and the organic layer was washed with brine. The organic layer was dried over MgSO₄, filtered, concentrated, and the resulting residue was purified by column chromatography (0-10% MeOH/DCM) to obtain the title compound as a white solid (26.4 mg, 56.9%).

¹H NMR (400 MHz, DMSO-d₆) *δ* 12.22 (s, 1H), 8.74 (broad, 2H), 8.27 (d, J = 6.7 Hz, 1H), 7.29 (t, J = 9.3 Hz, 2H), 4.76 - 4.63 (m, 1H), 3.86 - 3.77 (m, 1H), 3.76 - 3.69 (m, 1H), 3.69 - 3.56 (m, 3H), 3.26 (dd, J = 16.0, 7.5 Hz, 2H), 2.90 (dd, J = 16.1, 6.5 Hz, 2H), 2.41 - 2.30 (m, 1H), 2.30 - 2.18 (m, 1H); LCMS m/z 469 [M+H]⁺

### Example 24. methyl 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetate

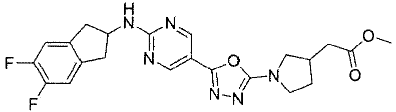

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (60 mg, 0.181 mmol), methyl 2-pyrrolidin-3-yl acetate hydrochloride (39.0 mg, 0.217 mmol), DIPEA (92 µL, 0.543 mmol), BOP reagent (96.1 mg, 0.217 mmol), and DMF (0.9 mL), the title compound was obtained as a yellow solid (22.5 mg, 27.2%).

¹H NMR (400 MHz, DMSO-d₆) *δ* 8.70 (s, 2H), 8.23 (d, J = 6.8 Hz, 1H), 7.27 (t, J = 9.2 Hz, 2H), 4.68 (dd, J = 13.3, 6.6 Hz, 1H), 3.72 - 3.65 (m, 1H), 3.58 (d, J = 16.2 Hz, 4H), 3.44 (d, J = 8.8 Hz, 1H), 3.24 (dd, J = 16.4, 7.4 Hz, 3H), 3.16 - 3.09 (m, 1H), 2.87 (dd, J = 15.9, 6.2 Hz, 2H), 2.72 - 2.53 (m, 2H), 2.23 - 2.03 (m, 2H); LCMS m/z 457 [M+H]⁺

### Example 25. 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetic acid

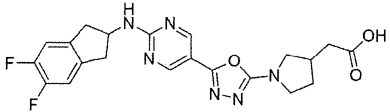

By the same method as in Example 22, using methyl 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetate (10 mg, 0.022 mmol), 1 N NaOH (223 µL, 0.223 mmol), and THF (0.11 mL), the title compound was obtained as a white solid (8 mg, 82.5%).

¹H NMR (400 MHz, D₂O) *δ* 8.54 (s, 2H), 6.99 (t, J = 9.4 Hz, 2H), 3.59 (s, 1H), 3.44 (d, J = 37.6 Hz, 3H), 3.16 (dd, J = 17.6, 7.5 Hz, 2H), 3.07 - 3.02 (m, 1H), 2.76 (d, J = 16.1 Hz, 2H), 2.60 - 2.48 (m, 2H), 2.20 (s, 2H), 2.07 (s, 1H); LCMS m/z 443 [M+H]⁺

### Example 26. 1-(5-(2-((5,6-difluoro-2,3-dihydro-1H-inden-2-yl)amino)pyrimidin-5-yl)-1,3,4-oxadiazol-2-yl)pyrrolidin-3-ol

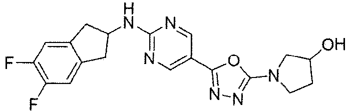

By the same method as in Step 1 of Example 1, using 5-(2-((5,6-difluoro-2,3- dihydro-1H-inden-2-yl)amino)pyrimidin-5-yl)-1,3,4-oxadiazol-2(3H)-one (0.3 g, 0.906 mmol), 3-hydroxypyrrolidine HCl (0.134 g, 1.09 mmol), DIPEA (462 µL, 2.72 mmol), BOP reagent (0.481 g, 1.09 mmol), and DMF (4.5 mL), the title compound was obtained as an off-white solid (0.235 g, 64.8%).

¹H NMR (400 MHz, DMSO-d₆) *δ* 8.72 (s, 2H), 8.23 (d, J = 6.9 Hz, 1H), 7.27 (t, J = 9.3 Hz, 2H), 5.08 (d, J = 3.6 Hz, 1H), 4.68 (dd, J = 13.9, 7.0 Hz, 1H), 4.38 (s, 1H), 3.58 - 3.51 (m, 3H), 3.35 (d, J = 10.9 Hz, 1H), 3.24 (dd, J = 16.2, 7.4 Hz, 2H), 2.88 (dd, J = 16.1, 6.4 Hz, 2H), 2.04 - 1.86 (m, 2H); LCMS m/z 401[M+H]⁺

### Example 27. 1-(5-(2-((5,6-difluoro-2,3-dihydro-1H-inden-2-yl)amino)pyrimidin-5-yl)-1,3,4-oxadiazol-2-yl)pyrrolidin-3-yl methanesulfonate

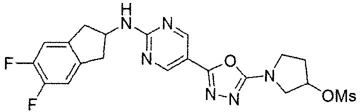

1-(5-(2-((5,6-difluoro-2,3-dihydro-1H-inden-2-yl)amino)pyrimidin-5-yl)-1,3,4-oxadiazol-2-yl)pyrrolidin-3-ol (0.0191 g, 0.477 mmol) and TEA (100 µL, 0.716 mmol) were dissolved in DCM (1.2 mL), MsCl (41 µL, 0.525 mmol) was added at 0 °C, and the mixture was stirred at room temperature for 8 h. DW was added to the reaction mixture, extracted with DCM, dried over Na₂SO₄, filtered, and concentrated. The obtained residue was purified by column chromatography (MeOH/DCM) to give the title compound as an off-white solid (86 mg, 37.7%).

¹H NMR (400 MHz, DMSO-d₆) *δ* 8.72 (s, 2H), 8.25 (d, J = 6.7 Hz, 1H), 7.27 (t, J = 9.3 Hz, 2H), 5.41 (s, 1H), 4.68 (dd, J = 14.0, 6.9 Hz, 1H), 3.83 - 3.71 (m, 2H), 3.66 (d, J = 7.1 Hz, 1H), 3.61 - 3.50 (m, 1H), 3.27 (s, 3H), 3.25 - 3.20 (m, 2H), 2.88 (dd, J = 16.2, 6.5 Hz, 2H), 2.36 - 2.25 (m, 2H); LCMS m/z 479 [M+H]⁺

### Example 28. 5-(5-(3-(1H-1,2,4-triazol-1-yl)pyrrolidin-1-yl)-1,3,4-oxadiazol-2-yl)-N-(5,6-difluoro-2,3-dihydro-1H-inden-2-yl)pyrimidin-2-amine

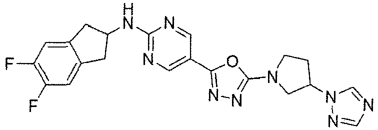

1-(5-(2-((5,6-difluoro-2,3-dihydro-1H-inden-2-yl)amino)pyrimidin-5-yl)-1,3,4-oxadiazol-2-yl)pyrrolidin-3-yl methanesulfonate (35 mg, 0.073 mmol) and 1,2,4-triazole (6.1 mg, 0.088 mmol) were dissolved in DMF (0.73 mL), NaH (57-63% oil dispersion) (3.5 mg, 0.088 mmol) was added, and the mixture was stirred at 60 °C overnight. The reaction mixture was cooled to 0 °C, MeOH was added to terminate the reaction, and DW was added and extracted with EtOAc. The organic layer was dried over Na₂SO₄, filtered and concentrated, and the resulting residue was purified by column chromatography (MeOH/DCM) to obtain the title compound as a white solid (32.3 mg, 97.8%).

¹H NMR (400 MHz, DMSO-d₆) *δ* 8.73 (s, 2H), 8.65 (s, 1H), 8.24 (d, J = 6.6 Hz, 1H), 7.99 (s, 1H), 7.27 (t, J = 9.2 Hz, 2H), 5.32 - 5.24 (m, 1H), 4.68 (dd, J = 13.9, 7.2 Hz, 1H), 3.98 (dd, J = 11.0, 6.4 Hz, 1H), 3.86 - 3.62 (m, 4H), 3.24 (dd, J = 16.1, 7.3 Hz, 2H), 3.15 (d, J = 5.3 Hz, 1H), 2.87 (dd, J = 16.1, 6.3 Hz, 2H); LCMS m/z 452[M+H]⁺

### Example 29. N-(5,6-difluoro-2,3-dihydro-1H-inden-2-yl)-5-(5-(3-(3-methyl-1H-1,2,4-triazol-1-yl)pyrrolidin-1-yl)-1,3,4-oxadiazol-2-yl)pyrimidin-2-amine

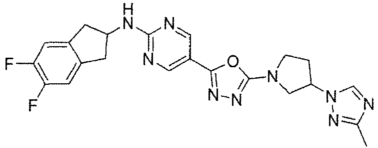

By the same method as in Example 28, using 1-(5-(2-((5,6-difluoro-2,3-dihydro-1H-inden-2-yl)amino)pyrimidin-5-yl)-1,3,4-oxadiazol-2-yl)pyrrolidin-3-yl methanesulfonate (35 mg, 0.073 mmol), 3-methyl-1H-1,2,4-triazole (7.3 mg, 0.088 mmol), NaH (57-63% oil dispersion) (3.5 mg, 0.088 mmol), and DMF (0.73 mL), the title compound was obtained as a white solid (31.4 mg, 92.2%).

¹H NMR (400 MHz, DMSO-d₆) *δ* 8.72 (s, 2H), 8.48 (s, 1H), 8.25 (d, J = 7.0 Hz, 1H), 7.27 (t, J = 9.2 Hz, 2H), 5.20 (d, J = 28.4 Hz, 2H), 4.68 (dd, J = 14.3, 7.4 Hz, 1H), 3.95 (s, 1H), 3.70 (ddd, J = 14.1, 12.3, 5.9 Hz, 4H), 3.24 (dd, J = 16.0, 7.6 Hz, 2H), 2.88 (dd, J = 16.0, 6.5 Hz, 2H), 2.22 (s, 3H); LCMS m/z 466[M+H]⁺

### Example 30. N-(5,6-difluoro-2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine

### Step 1: (4-(1-(5-(2-((5,6-difluoro-2,3-dihydro-1H-inden-2-yl)amino)pyrimidin-5-yl)-1,3,4-oxadiazol-2-yl)-3-methylpyrrolidin-3-yl)-1H-1,2,3-triazol-1-yl)methyl pivalate

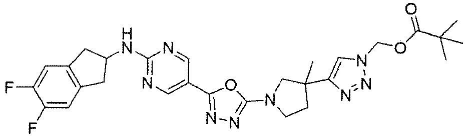

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (23 mg, 0.069 mmol), (4-(3-methylpyrrolidin-3-yl)-1H-1,2,3-triazol-1-yl)methyl pivalate, 2,2,2-trifluoroacetate (31 mg, 0.08 mmol), DIPEA (59 uL, 0.35 mmol), BOP reagent (37 mg, 0.08 mmol), and DMF (2 mL), the title compound was obtained as an off-white solid (25.5 mg, 63.8%).

LCMS m/z 580 [M+H]⁺

### Step 2: N-(5,6-difluoro-2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine

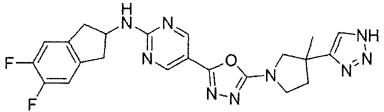

By the same method as in Step 2 of Example 1, using (4-(1-(5-(2-((5,6-difluoro-2,3-dihydro-1H-inden-2-yl)amino)pyrimidin-5-yl)-1,3,4-oxadiazol-2-yl)-3-methylpyrrolidin-3-yl)-1H-1,2,3-triazol-1-yl)methyl pivalate (25.5 mg, 0.044 mmol), K₂CO₃ (12.2 mg, 0.088 mmol), and MeOH (2 mL), the title compound was obtained as a white solid (17.8 mg, 86.9%).

¹H NMR (400 MHz, DMSO-d₆) *δ* 8.74 (broad, 2H), 8.25 (d, J = 6.9 Hz, 2H), 7.79 (s, 1H), 7.29 (t, J = 9.2 Hz, 3H), 4.77 - 4.64 (m, 1H), 3.95 - 3.84 (m, 1H), 3.72 - 3.62 (m, 1H), 3.62 - 3.49 (m, 2H), 3.26 (dd, J = 16.0, 7.6 Hz, 3H), 2.89 (dd, J = 16.1, 6.4 Hz, 3H), 2.44 - 2.31 (m, 1H), 2.20 - 2.06 (m, 2H), 1.45 (s, 3H); LCMS m/z 466 [M+H]⁺

### Example 31. N-(2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine

### Step 1: (4-(1-(5-(2-((2,3-dihydro-1H-inden-2-yl)amino)pyrimidin-5-yl)-1,3,4-oxadiazol-2-yl)-3-methylpyrrolidin-3-yl)-1H-1,2,3-triazol-1-yl)methyl pivalate

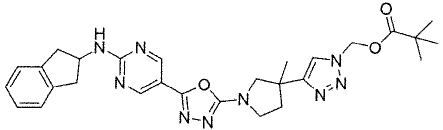

By the same method as in Step 1 of Example 1, using 5-(2-((2,3-dihydro-1H- inden-2-yl)amino)pyrimidin-5-yl)-1,3,4-oxadiazol-2(3H)-one (24.5 mg, 0.083 mmol), (4-(3-methylpyrrolidin-3-yl)-1H-1,2,3-triazol-1-yl)methyl pivalate, 2,2,2-trifluoroacetate (38 mg, 0.10 mmol), DIPEA (71 uL, 0.41 mmol), BOP reagent (44 mg, 0.10 mmol), and DMF (2 mL), the title compound was obtained as an off-white solid (38.3 mg, 84.9%).

LCMS m/z 544 [M+H]⁺

### Step 2: N-(2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine

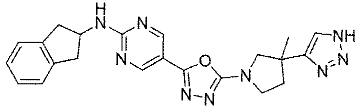

By the same method as in Step 2 of Example 1, using (4-(1-(5-(2-((2,3-dihydro-1H-inden-2-yl)amino)pyrimidin-5-yl)-1,3,4-oxadiazol-2-yl)-3-methylpyrrolidin-3-yl)-1H-1,2,3-triazol-1-yl)methyl pivalate (38.3 mg, 0.07 mmol), K₂CO₃ (19.5 mg, 0.14 mmol), and MeOH (2 mL), the title compound was obtained as a white solid (15.6 mg, 51.7%).

¹H NMR (400 MHz, DMSO-d₆) *δ* 8.71 (broad, 2H), 8.23 (d, J = 6.8 Hz, 1H), 7.79 (s, 1H), 7.23 - 7.07 (m, 4H), 4.77 - 4.61 (m, 1H), 3.88 (d, J = 9.7 Hz, 1H), 3.76 - 3.62 (m, 1H), 3.62 - 3.48 (m, 2H), 3.27 (dd, J = 15.8, 7.6 Hz, 2H), 2.93 (dd, J = 15.8, 6.9 Hz, 2H), 2.44 - 2.31 (m, 1H), 2.21 - 2.06 (m, 1H); LCMS m/z 430 [M+H]⁺

### Example 32. N-(3,5-dichlorophenethyl)-5-(5-(3-methyl-3-(1H-1,2,3-triazol-4-yl)pyrrolidin-1-yl)-1,3,4-oxadiazol-2-yl)pyrimidin-2-amine

### Step 1: (4-(1-(5-(2-((3,5-dichlorophenethyl)amino)pyrimidin-5-yl)-1,3,4-oxadiazol-2-yl)-3-methylpyrrolidin-3-yl)-1H-1,2,3-triazol-1-yl)methyl pivalate

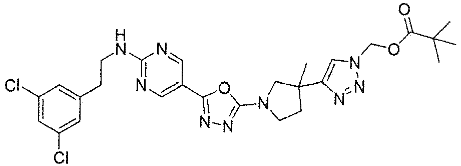

By the same method as in Step 1 of Example 1, using 5-(2-((3,5-dichlorophenethyl)amino)pyrimidin-5-yl)-1,3,4-oxadiazol-2(3H)-one (25.5 mg, 0.072 mmol), (4-(3-methylpyrrolidin-3-yl)-1H-1,2,3-triazol-1-yl)methyl pivalate, 2,2,2-trifluoroacetate (33 mg, 0.086 mmol), DIPEA (62 uL, 0.36 mmol), BOP reagent (38 mg, 0.086 mmol), and DMF (2 mL), the title compound was obtained as a white solid (29.6 mg, 68.1%).

LCMS m/z 600 [M+H]⁺

### Step 2: Preparation of N-(3,5-dichlorophenethyl)-5-(5-(3-methyl-3-(1H-1,2,3-triazol-4-yl)pyrrolidin-1-yl)-1,3,4-oxadiazol-2-yl)pyrimidin-2-amine

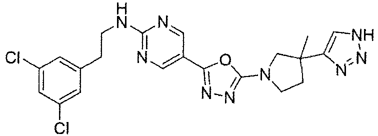

By the same method as in Step 2 of Example 1, using (4-(1-(5-(2-((3,5-dichlorophenethyl)amino)pyrimidin-5-yl)-1,3,4-oxadiazol-2-yl)-3-methylpyrrolidin-3-yl)-1H-1,2,3-triazol-1-yl)methyl pivalate (29.6 mg, 0.05 mmol), K₂CO₃ (13.6 mg, 0.099 mmol), and MeOH (2 mL), the title compound was obtained as a white solid (15.4 mg, 64.2%).

¹H NMR (400 MHz, DMSO-d₆) *δ* 8.69 (d, J = 6.6 Hz, 3H), 7.97 (t, J = 5.8 Hz, 2H), 7.85 (broad, 1H), 7.42 (s, 1H), 7.32 (s, 2H), 3.86 (d, J = 9.7 Hz, 2H), 3.72 - 3.62 (m, 2H), 3.62 - 3.48 (m, 4H), 2.88 (t, J = 6.9 Hz, 2H), 2.42 - 2.31 (m, 1H), 2.18 - 2.05 (m, 1H), 1.45 (s, 3H); LCMS m/z 486 [M+H]⁺

### Example 33. N-(3,5-difluorobenzyl)-5-(5-(3-methyl-3-(1H-1,2,3-triazol-4-yl)pyrrolidin-1-yl)-1,3,4-oxadiazol-2-yl)pyrimidin-2-amine

### Step 1: (4-(1-(5-(2-((3,5-difluorobenzyl)amino)pyrimidin-5-yl)-1,3,4-oxadiazol-2-yl)-3-methylpyrrolidin-3-yl)-1H-1,2,3-triazol-1-yl)methyl pivalate

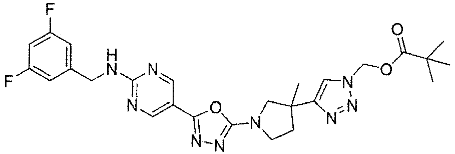

By the same method as in Step 1 of Example 1, using 5-(2-((3,5-difluorobenzyl)amino)pyrimidin-5-yl)-1,3,4-oxadiazol-2(3H)-one (21 mg, 0.068 mmol), (4-(3-methylpyrrolidin-3-yl)-1H-1,2,3-triazol-1-yl)methyl pivalate, 2,2,2-trifluoroacetate (31 mg, 0.082 mmol), DIPEA (58 uL, 0.34 mmol), BOP reagent (36 mg, 0.082 mmol), and DMF (2 mL), the title compound was obtained as a white solid (25.7 mg, 68.4%).

LCMS m/z 554 [M+H]⁺

### Step 2: N-(3,5-difluorobenzyl)-5-(5-(3-methyl-3-(1H-1,2,3-triazol-4-yl)pyrrolidin-1-yl)-1,3,4-oxadiazol-2-yl)pyrimidin-2-amine

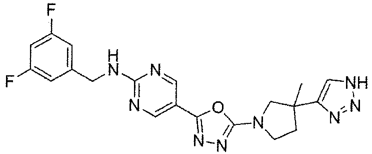

By the same method as in Step 2 of Example 1, using (4-(1-(5-(2-((3,5-difluorobenzyl)amino)pyrimidin-5-yl)-1,3,4-oxadiazol-2-yl)-3-methylpyrrolidin-3-yl)-1H-1,2,3-triazol-1-yl)methyl pivalate (25.7 mg, 0.046 mmol), K₂CO₃ (13 mg, 0.093 mmol), and MeOH (2 mL), the title compound was obtained as an off-white solid (16.2 mg, 79.5%).

¹H NMR (400 MHz, DMSO-d₆) *δ* 8.73 (s, 2H), 8.45 (t, J = 6.4 Hz, 1H), 7.79 (broad, 1H), 7.09 (t, J = 9.1 Hz, 1H), 7.02 (d, J = 6.5 Hz, 2H), 4.58 (d, J = 6.3 Hz, 2H), 3.87 (d, J = 9.5 Hz, 1H), 3.74 - 3.62 (m, 1H), 3.61 - 3.42 (m, 2H), 2.44 - 2.31 (m, 1H), 2.19 - 2.06 (m, 1H), 1.45 (s, 3H); LCMS m/z 440 [M+H]⁺

### Example 34. (E)-3-(2-((2,3-dihydro-1H-inden-2-yl)amino)pyrimidin-5-yl)-1-(3-methyl-3-(1H-1,2,3-triazol-4-yl)pyrrolidin-1-yl)prop-2-en-1-one

### Step 1: (E)-(4-(1-(3-(2-((2,3-dihydro-1H-inden-2-yl)amino)pyrimidin-5-yl)acryloyl)-3-methylpyrrolidin-3-yl)-1H-1,2,3-triazol-1-yl)methyl pivalate

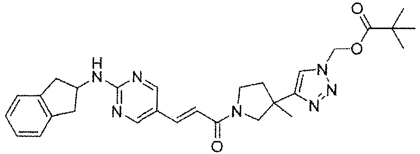

By the same method as in Step 2 of Example 20, using (E)-3-(2-((2,3-dihydro-1H-inden-2-yl)amino)pyrimidin-5-yl)acrylic acid (21.1 mg, 0.075 mmol), (4-(3-methylpyrrolidin-3-yl)-1H-1,2,3-triazol-1-yl)methyl pivalate, 2,2,2-trifluoroacetate (34.2 mg, 0.09 mmol), DIPEA (38 uL, 0.22 mmol), HBTU (34 mg, 0.090 mmol), and DMF (2 mL), the title compound was obtained as a white solid (29.7 mg, 75%).

LCMS m/z 530 [M+H]⁺

### Step 2: (E)-3-(2-((2,3-dihydro-1H-inden-2-yl)amino)pyrimidin-5-yl)-1-(3-methyl-3-(1H-1,2,3-triazol-4-yl)pyrrolidin-1-yl)prop-2-en-1-one

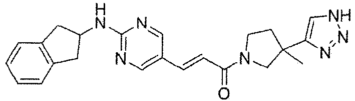

By the same method as in Step 2 of Example 1, using (E)-(4-(1-(3-(2-((2,3-dihydro-1H-inden-2-yl)amino)pyrimidin-5-yl)acryloyl)-3-methylpyrrolidin-3-yl)-1H-1,2,3-triazol-1-yl)methyl pivalate (29.7 mg, 0.056 mmol), K₂CO₃ (15.5 mg, 0.11 mmol), and MeOH (2 mL), the title compound was obtained as a light brown solid (8.6 mg, 36.9%).

¹H NMR (400 MHz, DMSO-d₆) *δ* 8.68 (s, 2H), 7.98 (d, J = 7.0 Hz, 1H), 7.33 (dd, J = 15.6, 2.9 Hz, 1H), 7.27 - 7.08 (m, 4H), 6.90 (dd, J = 20.5, 15.9 Hz, 1H), 4.73 - 4.56 (m, 1H), 4.01 (d, J = 8.2 Hz, 1H), 3.86 - 3.75 (m, 1H), 3.74 - 3.63 (m, 1H), 3.61 - 3.51 (m, 1H), 3.51 - 3.41 (m, 1H), 3.25 (dd, J = 15.7, 7.5 Hz, 6H), 2.91 (dd, J = 15.7, 6.6 Hz, 2H), 1.40 (d, J = 7.9 Hz, 3H); LCMS m/z 416 [M+H]⁺

### Example 35. N-(5,6-difluoro-2,3-dihydro-1H-inden-2-yl)-5-(5-(3-(1-methyl-1H-1,2,3-triazol-4-yl)pyrrolidin-1-yl)-1,3,4-oxadiazol-2-yl)pyrimidin-2-amine

### Step 1: N-(5,6-difluoro-2,3-dihydro-1H-inden-2-yl)-5-(5-(3-ethynylpyrrolidin-1-yl)-1,3,4-oxadiazol-2-yl)pyrimidin-2-amine

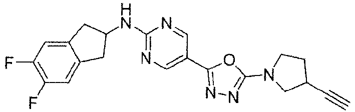

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (70 mg, 0.211 mmol), 3-ethynylpyrrolidine, 2,2,2-trifluoroacetate (49 mg, 0.254 mmol), DIPEA (108 µL, 0.634 mmol), BOP reagent (112 mg, 0.254 mmol), and DMF (1.1 mL), the title compound was obtained as an orange solid (59.5 mg, 68.9%).

¹H NMR (400 MHz, DMSO-d₆) *δ* 8.83 - 8.71 (m, 2H), 8.30 (d, *J =* 6.7 Hz, 1H), 7.32 (t, *J =* 9.2 Hz, 2H), 4.73 (dd, *J =* 13.6, 6.8 Hz, 1H), 3.82 - 3.77 (m, 1H), 3.63 (s, 1H), 3.55 (t, *J* = 8.6 Hz, 1H), 3.48 - 3.42 (m, 1H), 3.37 (s, 2H), 3.29 (dd, *J* = 16.1, 7.5 Hz, 2H), 2.92 (dd, *J* = 15.9, 6.5 Hz, 2H), 2.30 (d, *J* = 6.9 Hz, 1H), 2.04 (dd, *J* = 12.2, 7.5 Hz, 1H); LCMS m/z 409 [M+H]⁺

### Step 2: N-(5,6-difluoro-2,3-dihydro-1H-inden-2-yl)-5-(5-(3-(1-methyl-1H-1,2,3-triazol-4-yl)pyrrolidin-1-yl)-1,3,4-oxadiazol-2-yl)pyrimidin-2-amine

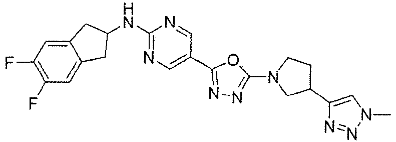

N-(5,6-difluoro-2,3-dihydro-1H-inden-2-yl)-5-(5-(3-ethynylpyrrolidin-1-yl)-1,3,4-oxadiazol-2-yl)pyrimidin-2-amine (20 mg, 0.049 mmol) was dissolved in MeOH (45 µL) and then azidomethane (66 µL), ascorbic acid (1.7 mg, 0.010 mmol) in DW (12 µL) and CuSO₄-5H₂O (1.0 mg, 0.004 mmol) in DW (12 µL) were added, and the mixture was stirred at room temperature overnight. The reaction mixture was concentrated, and the resulting residue was purified by column chromatography (MeOH/DCM) to give the title compound as a yellow solid (5 mg, 21.9%).

¹H NMR (400 MHz, CD₃OD) *δ* 8.77 (s, 2H), 7.89 (s, 1H), 7.12 (t, J = 9.0 Hz, 2H), 4.38 (s, 2H), 4.25 (dd, J = 9.4, 6.2 Hz, 2H), 3.73 (dd, J = 21.0, 7.3 Hz, 4H), 3.36 (s, 5H), 2.97 - 2.89 (m, 2H); LCMS m/z 466 [M+H]⁺

### Example 36. 5-(5-(3-(1H-1,2,3-triazol-4-yl)pyrrolidin-1-yl)-1,3,4-oxadiazol-2-yl)-N-(5-fluoro-2,3-dihydro-1H-inden-2-yl)pyrimidin-2-amine

### Step 1: 5-(2-((5-fluoro-2,3-dihydro-1H-inden-2-yl)amino)pyrimidin-5-yl)-1,3,4-oxadiazol-2(3H)-one

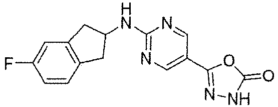

The title compound was prepared by the same method as in Preparation Example 3, except that 5-fluoro-2,3-dihydro-1H-inden-2-amine was used instead of 2,3-dihydro-1H-inden-2-amine.

LCMS m/z 314 [M+H]⁺

### Step 2: (4-(1-(5-(2-((5-fluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

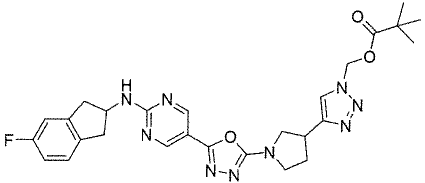

By the same method as in step 1 of Example 1, 5-(2-((5-fluoro-2,3-dihydro-1H-inden-2-yl)amino)pyrimidin-5-yl)-1,3,4-oxadiazol- 2(3H)-one (20.4 mg, 0.065 mmol), (4-(pyrrolidin-3-yl)-1H-1,2,3-triazol-1-yl)methyl pivalate, 2,2,2-trifluoroacetate (32 mg, 0.09 mmol), DIPEA (57.5 uL, 0.34 mmol), BOP reagent (34.5 mg, 0.08 mmol), and DMF (1.3 mL) was used to obtain the title compound as an off-white solid (19.5 mg, 55%).

¹H NMR (400 MHz, CDCl₃) *δ* 8.78 (s, 2H), 7.66 (s, 1H), 7.16 (dd, J = 7.9, 5.4 Hz, 1H), 6.93 (d, J = 8.4 Hz, 1H), 6.87 (t, J = 8.2 Hz, 1H), 6.21 (s, 2H), 5.68 (d, J = 7.5 Hz, 1H), 4.95 - 4.82 (m, 1H), 4.05 (s, 1H), 3.85 - 3.66 (m, 4H), 3.45 - 3.31 (m, 2H), 2.87 (td, J = 16.0, 4.9 Hz, 2H), 2.57 - 2.43 (m, 1H), 2.41 - 2.26 (m, 1H), 1.19 (s, 9H); LCMS m/z 548 [M+H]⁺

### Step 3: 5-(5-(3-(1H-1,2,3-triazol-4-yl)pyrrolidin-1-yl)-1,3,4-oxadiazol-2-yl)-N-(5-fluoro-2,3-dihydro-1H-inden-2-yl)pyrimidin-2-amine

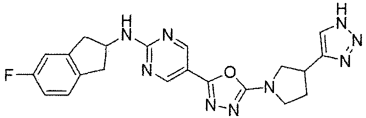

By the same method as in step 2 of example 1, using (4-(1-(5-(2-((5-fluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (17.3 mg, 0.032 mmol), K₂CO₃ (8.8 mg, 0.064 mmol), and MeOH (0.73 mL) was used to obtain the title compound as a yellow solid (13.5 mg, 99%).

¹H NMR (400 MHz, DMSO-d₆) δ 8.73 (s, 2H), 8.24 (d, J = 6.6 Hz, 1H), 7.80 (br s, 1H), 7.28 - 7.19 (m, 1H), 7.06 (d, J = 8.8 Hz, 1H), 6.96 (t, J = 9.0 Hz, 1H), 4.70 (dd, J = 14.3, 6.9 Hz, 1H), 3.93 (t, J = 8.0 Hz, 1H), 3.74 - 3.55 (m, 4H), 3.31 - 3.20 (m, 2H), 2.99 - 2.82 (m, 2H), 2.45 - 2.35 (m, 1H), 2.24 - 2.10 (m, 1H); LCMS m/z 434 [M+H]⁺

### Example 37. 5-(5-(3-(1H-1,2,3-triazol-4-yl)pyrrolidin-1-yl)-1,3,4-oxadiazol-2-yl)-N-(5-chloro-2,3-dihydro-1H-inden-2-yl)pyrimidin-2-amine

### Step 1: 5-(2-((5-chloro-2,3-dihydro-1H-inden-2-yl)amino)pyrimidin-5-yl)-1,3,4-oxadiazol-2(3H)-one

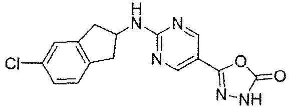

The title compound was prepared by the same method as in Preparation Example 3, except that 5-chloro-2,3-dihydro-1H-inden-2-amine was used instead of 2,3-dihydro-1H-inden-2-amine.

¹H NMR (400 MHz, DMSO-d₆) δ12.44 (br s, 1H), 8.66 (d, J = 17.0 Hz, 2H), 8.34 (d, J = 6.9 Hz, 1H), 7.29 (s, 1H), 7.22 (dd, J = 18.6, 8.0 Hz, 2H), 4.74 - 4.63 (m, 1H), 3.30 - 3.21 (m, 2H), 2.98 - 2.84 (m, 2H); LCMS m/z 330 [M+H]⁺

### Step 2: (4-(1-(5-(2-((5-chloro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate

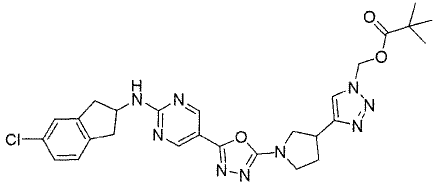

By the same method as in Step 1 of Example 1, using 5-(2-((5-chloro-2,3-dihydro-1H-inden-2-yl)amino)pyrimidin-5-yl)-1,3,4-oxadiazol-2(3H)-one (21.4 mg, 0.065 mmol), (4-(pyrrolidin-3-yl)-1H-1,2,3-triazol-1-yl)methyl pivalate, 2,2,2-trifluoroacetate (32 mg, 0.09 mmol), DIPEA (57.5 uL, 0.34 mmol), BOP reagent (34.5 mg, 0.08 mmol), and DMF (1.3 mL), the title compound was obtained as a white solid (7.5 mg, 20%).

¹H NMR (400 MHz, CDCl₃) δ8.78 (s, 2H), 7.66 (s, 1H), 7.22 (s, 1H), 7.16 (s, 2H), 6.21 (s, 2H), 5.69 (d, J = 7.6 Hz, 1H), 4.92 - 4.82 (m, 1H), 4.05 (s, 1H), 3.84 - 3.67 (m, 4H), 3.44 - 3.32 (m, 2H), 2.88 (ddd, J = 16.4, 11.3, 5.2 Hz, 2H), 2.56 - 2.45 (m, 1H), 2.39 - 2.27 (m, 1H), 1.19 (s, 9H); LCMS m/z 564 [M+H]⁺

### Step 3: 5-(5-(3-(1H-1,2,3-triazol-4-yl)pyrrolidin-1-yl)-1,3,4-oxadiazol-2-yl)-N-(5-chloro-2,3-dihydro-1H-inden-2-yl)pyrimidin-2-amine

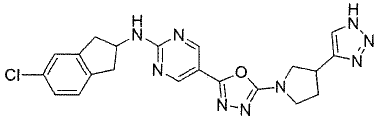

By the same method as in Step 2 of Example 1, using (4-(1-(5-(2-((5-chloro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate (7 mg, 0.012 mmol), K₂CO₃ (3.3 mg, 0.024 mmol), and MeOH (0.45 mL), the title compound was obtained as a white solid (5 mg, 89%).

¹H NMR (400 MHz, DMSO-d₆) δ 8.73 (s, 2H), 8.24 (d, J = 6.6 Hz, 1H), 7.80 (br s, 1H), 7.29 (s, 1H), 7.22 (dd, J = 19.3, 7.8 Hz, 2H), 4.68 (q, J = 6.8 Hz, 1H), 3.92 (dd, J = 9.0, 7.7 Hz, 1H), 3.75 - 3.54 (m, 4H), 3.32 - 3.20 (m, 2H), 2.98 - 2.84 (m, 2H), 2.40 (dt, J = 11.8, 6.2 Hz, 1H), 2.23 - 2.09 (m, 1H); LCMS m/z 450 [M+H]⁺

### Example 38. 5-(5-(3-(1H-imidazol-4-yl)pyrrolidin-1-yl)-1,3,4-oxadiazol-2-yl)-N-(5,6-difluoro-2,3-dihydro-1H-inden-2-yl)pyrimidin-2-amine

### Step 1: Preparation of 4-(pyrrolidin-3-yl)-1H-imidazole, 2,2,2-trifluoroacetate

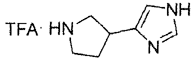

By the same method as in Step 2 of Preparation example 1, using tert-butyl 3-(1H-imidazol-4-yl)pyrrolidine-1-carboxylate (50 mg, 0.21 mmol), TFA (2 mL), and DCM (4 mL), the title compound was obtained (quantitatively) and used in the next reaction without purification (23 mg).

LCMS m/z 138 [M+H]⁺

### Step 2: 5-(5-(3-(1H-imidazol-4-yl)pyrrolidin-1-yl)-1,3,4-oxadiazol-2-yl)-N-(5,6-difluoro-2,3-dihydro-1H-inden-2-yl)pyrimidin-2-amine

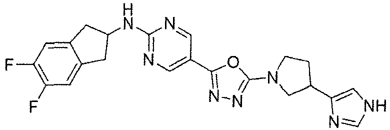

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (30 mg, 0.09 mmol), 4-(pyrrolidin-3-yl)-1H-imidazole, 2,2,2-trifluoroacetate (23 mg, 0.1 mmol), BOP reagent (49 mg, 0.11 mmol), DIPEA (213 µL, 1.22 mmol), and DMF (1 mL), the title compound was obtained as a white solid (3 mg, 7%).

¹H NMR (400 MHz, CD₃OD) *δ* 8.78 (s, 2H), 7.70 (s, 1H), 7.14 (t, J = 8.0 Hz, 2H), 4.87 - 4.84 (m, 1H), 3.79 - 3.64 (m, 4H), 3.38 - 3.36 (m, 2H), 3.26 - 3.24 (m, 1H), 2.97 - 2.92 (m, 2H), 2.44 - 2.40 (m, 1H), 2.28 - 2.24 (m, 1H); LCMS m/z 451 [M+H]⁺

### Example 39. 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrimidin-2-amine

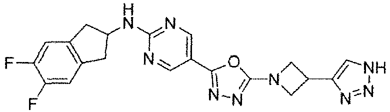

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (30 mg, 0.091 mmol), 4-(azetidin-3-yl)-1H-1,2,3-triazole, 2,2,2-trifluoroacetate (24.1 mg, 0.109 mmol), DIPEA (46 µL, 0.272 mmol), BOP reagent (48.1 mg, 0.109 mmol), and DMF (0.5 mL), the title compound was obtained as a yellow solid (21.3 mg, 53.8%).

¹H NMR (400MHz, DMSO-d₆) *δ* 8.71 (d, *J =* 10.0 Hz, 2H), 8.28 (d, *J* = 6.6 Hz, 1H), 7.91 (s, 1H), 7.27 (t, *J =* 9.4 Hz, 2H), 4.69 (dd, *J* = 13.7, 6.8 Hz, 1H), 4.50 (s, 2H), 4.21 (s, 3H), 3.24 (dd, *J =* 15.9, 7.6 Hz, 2H), 2.88 (dd, *J* = 16.1, 6.7 Hz, 2H); LCMS m/z 438[M+H]⁺

### Example 40. (E)-1-(3-(1H-1,2,3-triazol-4-yl)azetidin-1-yl)-3-(2-((5,6-difluoro-2,3-dihydro-1H-inden-2-yl)amino)pyrimidin-5-yl)prop-2-en-1-one

### Step 1: (E)-(4-(1-(3-(2-((5,6-difluoro-2,3-dihydro-1H-inden-2-yl)amino)pyrimidin-5-yl)acryloyl)azetidin-3-yl)-1H-1,2,3-triazol-1-yl)methyl pivalate

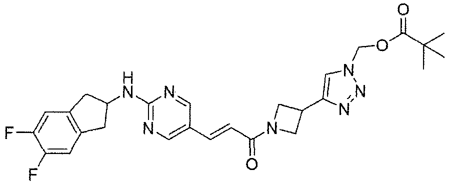

By the same method as in step 3 of Example 9, (E)-3-(2-((5,6-difluoro-2,3-dihydro-1H-inden-2-yl)amino)pyrimidin-5-yl)acrylic acid (60 mg, 0.189 mmol), (4-(azetidin-3-yl)-1H-1,2,3-triazol-1-yl)methyl pivalate (76.1 mg, 0.227 mmol), DIPEA (96 µL, 0.567 mmol), (4-(azetidin-3-yl)-1H-1,2,3-triazol-1-yl)methyl pivalate, HBTU (86.1 mg, 0.227 mmol), and DMF (3.4 mL) were used to obtain the title compound as a white solid (86.1 mg, 84.7%).

¹H NMR (400 MHz, DMSO-d₆) *δ* 8.66 (s, 2H), 8.24 (s, 1H), 8.00 (d, *J =* 6.5 Hz, 1H), 7.27 (dd, *J* = 23.0, 12.7 Hz, 3H), 6.66 (d, *J* = 15.8 Hz, 1H), 6.28 (s, 2H), 4.66 - 4.62 (m, 1H), 4.31 (t, *J* = 6.9 Hz, 2H), 3.99 (t, *J =* 5.6 Hz, 2H), 3.22 (dd, *J* = 16.1, 7.7 Hz, 2H), 3.14 - 3.11 (m, 1H), 2.86 (dd, *J =* 16.2, 6.8 Hz, 2H), 1.11 (s, 9H); LCMS m/z 538 [M+H]⁺

### Step 2: (E)-1-(3-(1H-1,2,3-triazol-4-yl)azetidin-1-yl)-3-(2-((5,6-difluoro-2,3-dihydro-1H-inden-2-yl)amino)pyrimidin-5-yl)prop-2-en-1-one

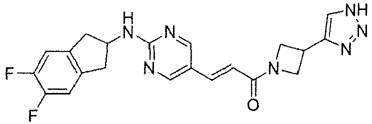

By the same method as in Example 3, using (E)-(4-(1-(3-(2-((5,6-difluoro-2,3-dihydro-1H-inden-2-yl)amino)pyrimidin-5-yl)acryloyl)azetidin-3-yl)-1H-1,2,3-triazol-1-yl)methyl pivalate (80 mg, 0.149 mmol), K₂CO₃ (41.1 mg, 0.298 mmol), and MeOH (0.7 mL), the title compound was obtained as a yellow solid (32 mg, 50.8%).

¹H NMR (400 MHz, DMSO-d₆) *δ* 8.66 (s, 2H), 8.00 (d, *J =* 7.0 Hz, 1H), 7.87 (s, 1H), 7.27 (dd, *J =* 22.4, 12.6 Hz, 3H), 6.66 (d, *J =* 15.7 Hz, 1H), 4.65 (dd, *J =* 14.6, 7.0 Hz, 1H), 4.33 - 4.26 (m, 2H), 4.04 - 3.94 (m, 2H), 3.22 (dd, *J* = 16.0, 7.2 Hz, 2H), 3.15 (d, *J* = 5.1 Hz, 1H), 2.86 (dd, *J* = 16.3, 6.4 Hz, 2H); LCMS m/z 424 [M+H]⁺

### Example 41. N-(5,6-difluoro-2,3-dihydro-1H-inden-2-yl)-5-(5-(3-(1-methyl-1H-1,2,3-triazol-5-yl)azetidin-1-yl)-1,3,4-oxadiazol-2-yl)pyrimidin-2-amine

### Step 1: (4-(1-(5-(2-((5,6-difluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

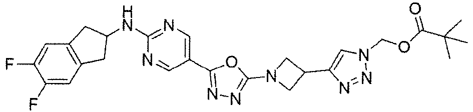

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (0.1 g, 0.302 mmol), (4-(azetidin-3-yl)-1H-1,2,3-triazol-1-yl)methyl pivalate (0.122 g, 0.362 mmol), DIPEA (154 µL, 0.906 mmol), BOP reagent (0.160 g, 0.362 mmol), and DMF (1.5 mL), the title compound was obtained as a white solid (47.8 mg, 28.7%).

¹H NMR (400 MHz, DMSO-d₆) *δ* 8.71 (s, 2H), 8.29 - 8.28 (m, 1H), 7.27 (t, J = 9.2 Hz, 2H), 6.28 (s, 2H), 4.69 (dd, J = 14.3, 7.1 Hz, 1H), 4.50 (s, 2H), 4.22 (d, J = 3.3 Hz, 3H), 3.24 (dd, J = 15.9, 7.8 Hz, 2H), 2.88 (dd, J = 16.1, 6.7 Hz, 2H), 1.14 - 1.08 (m, 9H); LCMS m/z 552 [M+H]⁺

### Step 2: N-(5,6-difluoro-2,3-dihydro-1H-inden-2-yl)-5-(5-(3-(1-methyl-1H-1,2,3-triazol-5-yl)azetidin-1-yl)-1,3,4-oxadiazol-2-yl)pyrimidin-2-amine

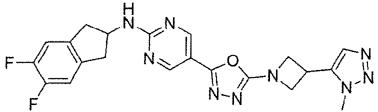

(4-(1-(5-(2-((5,6-difluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (45 mg, 0.082 mmol) and MeOTf (11 µL, 0.098 mmol) were dissolved in a small amount of DCM and stirred at room temperature overnight. MeOH (0.41 mL) was added to the reaction mixture, followed by K₂CO₃ (22.6 mg, 0.163 mmol), and the mixture was stirred at room temperature for 4 h. The reaction mixture was filtered and concentrated, and the obtained residue was purified by column chromatography (MeOH/DCM) to give the title compound as a yellow solid (3.4 mg, 8.9%).

¹H NMR (400 MHz, DMSO-d₆) *δ* 8.70 (d, J = 11.5 Hz, 2H), 8.31 - 8.24 (m, 1H), 7.88 (s, 1H), 7.31 - 7.22 (m, 2H), 4.69 (dd, J = 13.5, 6.7 Hz, 1H), 4.54 (dt, J = 7.5, 3.8 Hz, 2H), 4.28 - 4.19 (m, 2H), 3.94 - 3.81 (m, 3H), 3.27 - 3.21 (m, 2H), 3.15 - 3.08 (m, 1H), 2.92 - 2.83 (m, 2H); LCMS m/z 452 [M+H]⁺

### Example 42. 5-(5-(3-(1H-1,2,3-triazol-4-yl)azetidin-1-yl)-1,3,4-oxadiazol-2-yl)-N-(2,3-dihydro-1H-inden-2-yl)pyrimidin-2-amine

### Step 1: (4-(1-(5-(2-((2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

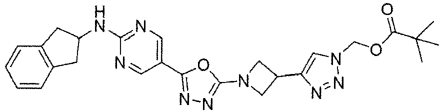

By the same method as in step 1 of Example 1, 5-(2-((2,3-dihydro-1H-inden-2-yl)amino)pyrimidin-5-yl)-1,3,4-oxadiazol-2(3H)-one (29 mg, 0.1 mmol), (4-(azetidin-3-yl)-1H-1,2,3-triazol-1-yl)methyl pivalate (42 mg, 0.1 mmol), DIPEA (84 uL, 0.49 mmol), BOP reagent (52.3 mg, 0.12 mmol), and DMF (2 mL) was used to obtain the title compound as a white solid (42.4 mg, yield 83.4%).

¹H NMR (400 MHz, CDCl₃) *δ* 8.78 (s, 2H), 7.76 (s, 1H), 7.26 - 7.16 (m, 4H), 6.23 (s, 2H), 5.78 (d, J = 7.8 Hz, 1H), 4.92 - 4.84 (m, 1H), 4.60 (t, J = 8.3 Hz, 2H), 4.41 (t, J = 7.6 Hz, 2H), 4.27 - 4.20 (m, 1H), 3.42 (dd, J = 15.9, 6.9 Hz, 2H), 2.91 (dd, J = 15.9, 5.1 Hz, 2H), 1.20 (s, 9H); LCMS m/z 516 [M+H]⁺

### Step 2: 5-(5-(3-(1H-1,2,3-triazol-4-yl)azetidin-1-yl)-1,3,4-oxadiazol-2-yl)-N-(2,3-dihydro-1H-inden-2-yl)pyrimidin-2-amine

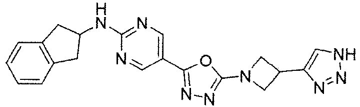

By the same method as in Step 2 of Example 1, using (4-(1-(5-(2-((2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (39 mg, 0.076 mmol), K₂CO₃ (21 mg, 0.15 mmol), and MeOH (1.5 mL), the title compound was obtained as a white solid (19.5 mg, 63.6%).

¹H NMR (400 MHz, DMSO-d₆) *δ* 8.73 (d, J = 16.6 Hz, 2H), 8.27 (d, J = 6.9 Hz, 1H), 7.93 (s, 1H), 7.29 - 7.07 (m, 4H), 4.73 - 4.64 (m 1H), 4.52 (s, 2H), 4.23 (s, 3H), 3.27 (dd, J = 15.9, 7.5 Hz, 2H), 2.93 (dd, J = 15.7, 6.8 Hz, 2H); LCMS m/z 402 [M+H]⁺

### Example 43. 5-(3-(3-(1H-1,2,3-triazol-4-yl)azetidin-1-yl)-4,5-dihydroisoxazol-5-yl)-N-(5,6-difluoro-2,3-dihydro-1H-inden-2-yl)pyrimidin-2-amine

### Step 1: (4-(1-(5-(2-((5,6-difluoro-2,3-dihydro-1H-inden-2-yl)amino)pyrimidin-5-yl)-4,5-dihydroisoxazol-3-yl)azetidin-3-yl)-1H-1,2,3-triazol-1-yl)methyl pivalate

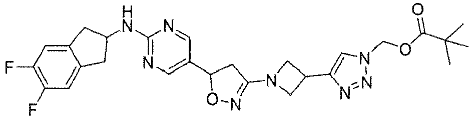

By the same method as in Step 3 of Example 17, using 5-(3-bromo-4,5-dihydroisoxazol-5-yl)-N-(5,6-difluoro-2,3-dihydro-1H-inden-2-yl)pyrimidin-2-amine (0.150 g, 0.380 mmol), (4-(azetidin-3-yl)-1H-1,2,3-triazol-1-yl)methyl pivalate (0.153 g, 0.455 mmol), Na₂CO₃ (0.101 g, 0949 mmol), and t-BuOH (3.4 mL), the title compound was obtained as a yellow solid (43.8 mg, 20.9%).

¹H NMR (400 MHz, DMSO-d₆) *δ* 8.32 (s, 2H), 8.22 (s, 1H), 7.62 (d, J = 7.4 Hz, 1H), 7.25 (s, 2H), 6.28 (s, 2H), 5.27 (d, J = 19.1 Hz, 2H), 4.65 - 4.59 (m, 2H), 4.20 (dd, J = 12.1, 4.7 Hz, 2H), 3.95 (d, J = 6.8 Hz, 1H), 3.19 (s, 4H), 2.83 (d, J = 9.6 Hz, 2H), 1.11 (s, 9H); LCMS m/z 553 [M+H]⁺

### Step 2: 5-(3-(3-(1H-1,2,3-triazol-4-yl)azetidin-1-yl)-4,5-dihydroisoxazol-5-yl)-N-(5,6-difluoro-2,3-dihydro-1H-inden-2-yl)pyrimidin-2-amine

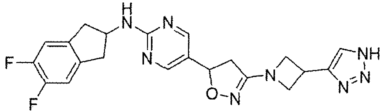

The title compound was obtained as a white solid (10.8 mg, 90.7%) by the same method as in Example 3, using (4-(1-(5-(2-((5,6-difluoro-2,3-dihydro-1H-inden-2-yl)amino)pyrimidin-5-yl)-4,5-dihydroisoxazol-3-yl)azetidin-3-yl)-1H-1,2,3-triazol-1-yl)methyl pivalate (15 mg, 0.027 mmol), K₂CO₃ (7.5 mg, 0.054 mmol), and MeOH (0.14 mL).

¹H NMR (400 MHz, DMSO-d₆) *δ* 8.32 (s, 2H), 7.77 (s, 1H), 7.62 (d, J = 6.8 Hz, 1H), 7.25 (t, J = 9.3 Hz, 2H), 5.27 (t, J = 9.1 Hz, 1H), 4.61 (dd, J = 14.1, 7.2 Hz, 1H), 4.20 (t, J = 7.7 Hz, 2H), 4.03 (dd, J = 12.0, 4.9 Hz, 2H), 3.93 (d, J = 5.8 Hz, 2H), 3.20 (d, J = 8.4 Hz, 2H), 3.02 (dd, J = 15.9, 9.6 Hz, 1H), 2.83 (dd, J = 15.8, 6.5 Hz, 2H); LCMS m/z 439 [M+H]⁺

### Example 44. 1-(3-(1H-1,2,3-triazol-4-yl)azetidin-1-yl)-3-(2-((5,6-difluoro-2,3-dihydro-1H-inden-2-yl)amino)pyrimidin-5-yl)propan-1-one

### Step 1: (4-(1-(3-(2-((5,6-difluoro-2,3-dihydro-1H-inden-2-yl)amino)pyrimidin-5-yl)propanoyl)azetidin-3-yl)-1H-1,2,3-triazol-1-yl)methyl pivalate

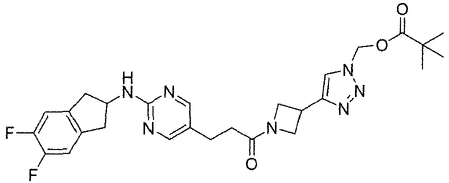

By the same method as in Step 3 of Example 9, using 3-(2-((5,6-difluoro-2,3-dihydro-1H-inden-2-yl)amino)pyrimidin-5-yl)propanoic acid (52 mg, 0.163 mmol), (4-(azetidin-3-yl)-1H-1,2,3-triazol-1-yl)methyl pivalate (65.5 mg, 0.195 mmol), DIPEA (83 µL, 0.489 mmol), HBTU (74.4 mg, 0.195 mmol), and DMF (3.3 mL), the title compound was obtained as a yellow solid (60 mg, 68.3%).

LCMS m/z 540 [M+H]⁺

### Step 2: 1-(3-(1H-1,2,3-triazol-4-yl)azetidin-1-yl)-3-(2-((5,6-difluoro-2,3-dihydro-1H-inden-2-yl)amino)pyrimidin-5-yl)propan-1-one

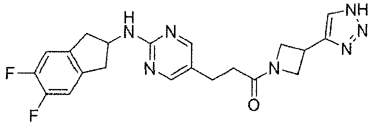

By the same method as in Example 3, (4-(1-(3-(2-((5,6-difluoro-2,3-dihydro-1H-inden-2-yl)amino)pyrimidin-5-yl)propanoyl)azetidin-3-yl)-1H-1,2,3-triazol-1-yl)methyl pivalate (40 mg, 0.074 mmol), K₂CO₃ (20.5 mg, 0.148 mmol), and MeOH (0.4 mL) was used to obtain the title compound as a yellow solid (18.4 mg, 58.3%).

¹H NMR (400 MHz, DMSO-d₆) *δ* 8.17 (s, 2H), 7.28 (d, J = 6.9 Hz, 1H), 7.24 (t, J = 9.4 Hz, 2H), 4.57 (dd, J = 14.0, 6.9 Hz, 1H), 4.44 (t, J = 8.4 Hz, 1H), 4.20 (t, J = 8.5 Hz, 1H), 4.10 (d, J = 6.3 Hz, 1H), 3.96 - 3.84 (m, 2H), 3.19 (dd, J = 16.3, 7.6 Hz, 2H), 2.81 (dd, J = 15.6, 6.7 Hz, 2H), 2.59 (t, J = 7.5 Hz, 2H), 2.32 (t, J = 7.3 Hz, 2H); LCMS m/z 426 [M+H]⁺

### Example 45. 5-(3-(3-(1H-1,2,3-triazol-4-yl)azetidin-1-yl)isoxazol-5-yl)-N-(5,6-difluoro-2,3-dihydro-1H-inden-2-yl)pyrimidin-2-amine

### Step 1: (4-(1-(5-(2-((5,6-difluoro-2,3-dihydro-1H-inden-2-yl)amino)pyrimidin-5-yl)isoxazol-3-yl)azetidin-3-yl)-1H-1,2,3-triazol-1-yl)methyl pivalate

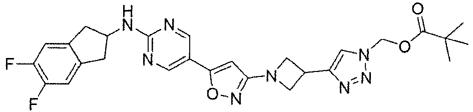

By the same method as in Example 18, using (4-(1-(5-(2-((5,6-difluoro-2,3-dihydro-1H-inden-2-yl)amino)pyrimidin-5-yl)-4,5-dihydroisoxazol-3-yl)azetidin-3-yl)-1H-1,2,3-triazol-1-yl)methyl pivalate (25 mg, 0.045 mmol), I₂ (17.2 mg, 0.068 mmol), imidazole (9.2 mg, 0.136 mmol), and toluene (2.3 mL), the title compound was obtained as a white solid (6.5 mg, 26.1%).

¹H NMR (400 MHz, CD₃OD) *δ* 8.66 (s, 2H), 8.10 (s, 1H), 7.10 (t, J = 9.0 Hz, 2H), 6.30 (s, 2H), 6.29 (s, 1H), 4.83 - 4.77 (m, 3H), 4.39 (t, J = 7.5 Hz, 2H), 4.19 (dd, J = 13.6, 7.7 Hz, 1H), 4.14 - 4.10 (m, 2H), 2.91 (dd, J = 15.8, 6.1 Hz, 2H), 1.18 (s, 8H); LCMS m/z 551 [M+H]⁺

### Step 2: 5-(3-(3-(1H-1,2,3-triazol-4-yl)azetidin-1-yl)isoxazol-5-yl)-N-(5,6-difluoro-2,3-dihydro-1H-inden-2-yl)pyrimidin-2-amine

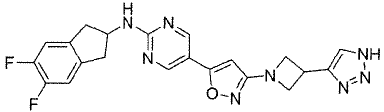

The title compound was obtained as a white solid (0.8 mg, 15.5%) by the same method as in Example 3, using (4-(1-(5-(2-((5,6-difluoro-2,3-dihydro-1H-inden-2-yl)amino)pyrimidin-5-yl)isoxazol-3-yl)azetidin-3-yl)-1H-1,2,3-triazol-1-yl)methyl pivalate (6.5 mg, 0.012 mmol), K₂CO₃ (3.3 mg, 0.024 mmol), and MeOH (0.1 mL).

¹H NMR (400 MHz, CD₃OD) *δ* 8.66 (s, 2H), 7.80 (s, 1H), 7.11 (t, J = 9.0 Hz, 2H), 6.29 (s, 1H), 4.81 (dd, J = 13.8, 7.3 Hz, 3H), 4.40 (t, J = 7.7 Hz, 2H), 4.22 (dt, J = 15.6, 7.8 Hz, 1H), 4.11 (t, J = 6.6 Hz, 2H), 2.91 (dd, J = 15.8, 6.1 Hz, 2H); LCMS m/z 437 [M+H]⁺

### Example 46. 1-(5-(2-((5,6-difluoro-2,3-dihydro-1H-inden-2-yl)amino)pyrimidin-5-yl)-1,3,4-oxadiazol-2-yl)azetidine-3-sulfonamide

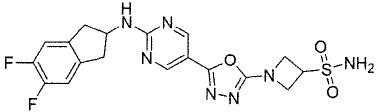

By the same method as in Step 1 of Example 1, a solution of 5-(2-((5,6-difluoro-2,3-dihydro-1H-inden-2-yl)amino)pyrimidin-5-yl)-1,3,4-oxadiazol-2(3H)-one (60 mg, 0.181 mmol), azetidine-3-sulfonamide hydrochloride (37.5 mg, 0.217 mmol), DIPEA (92 µL, 0.543 mmol), BOP reagent (96.1 mg, 0.217 mmol), and DMF (0.9 mL) were used to obtain the title compound as a white solid (31 mg, 38.3%).

¹H NMR (400 MHz, DMSO-d₆) *δ* 8.72 (d, J = 13.8 Hz, 2H), 8.29 (d, J = 6.8 Hz, 1H), 7.30 - 7.24 (m, 4H), 4.68 (dd, J = 13.9, 6.9 Hz, 1H), 4.44 (d, J = 7.6 Hz, 2H), 4.30 - 4.24 (m, 3H), 3.24 (dd, J = 16.0, 7.5 Hz, 2H), 2.88 (dd, J = 16.0, 6.5 Hz, 2H); LCMS m/z 450 [M+H]⁺

### Example 47. methyl 1-(5-(2-((5,6-difluoro-2,3-dihydro-1H-inden-2-yl)amino)pyrimidin-5-yl)-1,3,4-oxadiazol-2-yl)azetidine-3-carboxylate

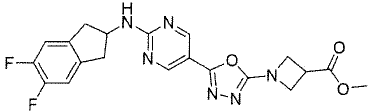

By the same method as in Step 1 of Example 1, 5-(2-((5,6-difluoro-2,3-dihydro-1H-inden-2-yl)amino)pyrimidin-5-yl)-1,3,4-oxadiazol-2(3H)-one (60 mg, 0.18 mmol), methyl 3-pyrrolidinecarboxylate hydrochloride (33 mg, 0.22 mmol), DIPEA (92 µL, 0.54 mmol), BOP reagent (96 mg, 0.22 mmol), and DMF (0.9 mL) were used to obtain the title compound as a white solid (46 mg, 59%).

¹H NMR (400 MHz, CDCl₃) *δ* 8.77 (s, 2H), 7.02 (t, J = 8.8 Hz, 2H), 5.67 (d, J = 7.4 Hz, 1H), 4.93 - 4.84 (m, 1H), 4.41 (s, 2H), 4.40 (s, 2H), 3.79 (s, 3H), 3.71 - 3.63 (m, 1H), 3.36 (dd, J = 16.3, 7.0 Hz, 2H), 2.85 (dd, J = 16.0, 5.4 Hz, 2H); LCMS m/z 429 [M+H]⁺

### Example 48. 5-(5-(3-(1H-1,2,3-triazol-4-yl)azetidin-1-yl)-1,3,4-oxadiazol-2-yl)-N-(3,5-difluorobenzyl)pyrimidin-2-amine

### Step 1: (4-(1-(5-(2-((3,5-difluorobenzyl)amino)pyrimidin-5-yl)-1,3,4-oxadiazol-2-yl)azetidin-3-yl)-1H-1,2,3-triazol-1-yl)methyl pivalate

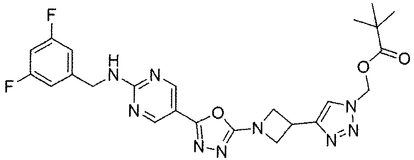

By the same method as in Step 1 of Example 1, using 5-(2-((3,5-difluorobenzyl)amino)pyrimidin-5-yl)-1,3,4-oxadiazol-2(3H)-one (39.7 mg, 0.13 mmol), (4-(azetidin-3-yl)-1H-1,2,3-triazol-1-yl)methyl pivalate (55 mg, 0.16 mmol), DIPEA (111 uL, 0.65 mmol), BOP reagent (69.3 mg, 0.16 mmol) and DMF (1.3 mL), the title compound was obtained as a white solid (48.9 mg, 71.6%).

¹H NMR (400 MHz, CD₃OD) *δ* 8.74 (d, J = 1.5 Hz, 2H), 8.13 (s, 1H), 6.94 (d, J = 6.7 Hz, 2H), 6.79 (t, J = 9.2 Hz, 1H), 6.30 (s, 2H), 4.65 (s, 2H), 4.61 (t, J = 8.2 Hz, 2H), 4.36 (t, J = 6.4 Hz, 2H), 4.32 - 4.24 (m, 1H), 1.17 (s, 9H); LCMS m/z 526 [M+H]⁺

### Step 2: 5-(5-(3-(1H-1,2,3-triazol-4-yl)azetidin-1-yl)-1,3,4-oxadiazol-2-yl)-N-(3,5-difluorobenzyl)pyrimidin-2-amine

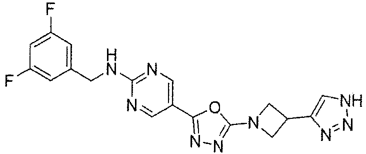

By the same method as in Step 2 of Example 1, using (4-(1-(5-(2-((3,5-difluorobenzyl)amino)pyrimidin-5-yl)-1,3,4-oxadiazol-2-yl)azetidin-3-yl)-1H-1,2,3-triazol-1-yl)methyl pivalate (39 mg, 0.095 mmol), K₂CO₃ (26.2 mg, 0.19 mmol), and MeOH (2 mL), the title compound was obtained as a white solid (31.7 mg, 81.3%).

¹H NMR (400 MHz, DMSO-d₆) *δ* 8.72 (broad, 2H), 8.49 (t, J = 6.3 Hz, 1H), 7.89 (s, 1H), 7.09 (t, J = 9.3 Hz, 1H), 7.02 (d, J = 6.9 Hz, 2H), 4.58 (d, J = 6.2 Hz, 2H), 4.51 (m, 2H), 4.22 (m, 3H); LCMS m/z 412 [M+H]⁺

### Example 49. 1-(5-(2-((5,6-difluoro-2,3-dihydro-1H-inden-2-yl)amino)pyrimidin-5-yl)-1,3,4-oxadiazol-2-yl)azetidin-3-ol

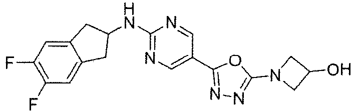

By the same method as in Step 1 of Example 1, using 5-(2-((5,6-difluoro-2,3- dihydro-1H-inden-2-yl)amino)pyrimidin-5-yl)-1,3,4-oxadiazol-2(3H)-one (60 mg, 0.181 mmol), azetidin-3-ol, 2,2,2-trifluoroacetate (37.0 mg, 0.217 mmol), DIPEA (92 µL, 0.543 mmol), BOP reagent (96.1 mg, 0.217 mmol), and DMF (0.9 mL), the title compound was obtained as a white solid (25 mg, 35.7%).

¹H NMR (400 MHz, DMSO-d₆) *δ* 8.70 (s, 2H), 8.26 (d, J = 6.9 Hz, 1H), 7.27 (t, J = 9.3 Hz, 2H), 5.84 (d, J = 6.6 Hz, 1H), 4.68 (dd, J = 13.6, 7.0 Hz, 1H), 4.61 (d, J = 6.8 Hz, 1H), 4.33 - 4.27 (m, 2H), 3.89 (dd, J = 8.6, 4.9 Hz, 2H), 3.24 (dd, J = 16.0, 7.6 Hz, 2H), 2.87 (dd, J = 16.0, 6.4 Hz, 2H); LCMS m/z 387 [M+H]⁺

### Example 50. 1-(5-(2-((5,6-difluoro-2,3-dihydro-1H-inden-2-yl)amino)pyrimidin-5-yl)-1,3,4-oxadiazol-2-yl)azetidine-3-carboxylic acid

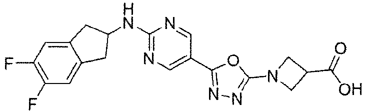

Methyl 1-(5-(2-((5,6-difluoro-2,3-dihydro-1H-inden-2-yl)amino)pyrimidin-5-yl)-1,3,4-oxadiazol-2-yl)azetidine-3-carboxylate (34 mg, 0.08 mmol) was dissolved in THF (0.40 mL), 1 N NaOH (0.2 mL, 0.20 mmol) was added, and the mixture was stirred at room temperature for 3 h. 1 N HCl was added to the reaction mixture to adjust the pH to 2, and extracted with EtOAc. The organic layer was washed with brine, dried over MgSO₄, filtered, and concentrated to give the title compound as a white solid (32 mg, 97%).

¹H NMR (400 MHz, DMSO-d₆) *δ* 12.80 (s, 1H), 8.71 (s, 2H), 8.29 (d, J = 6.8 Hz, 1H), 7.29 (t, J = 9.2 Hz, 2H), 4.75 - 4.64 (m, 1H), 4.32 (t, J = 8.5 Hz, 2H), 4.24 - 4.15 (m, 2H), 3.70 - 3.58 (m, 1H), 3.26 (dd, J = 16.2, 7.6 Hz, 2H), 2.89 (dd, J = 16.1, 6.7 Hz, 2H); LCMS m/z 415 [M+H]⁺

### Example 51. N-(5,6-difluoro-2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine

### Step 1: N-(5,6-difluoro-2,3-dihydro-1H-inden-2-yl)-5-(5-(3-ethynyl-3-methylazetidin-1-yl)-1,3,4-oxadiazol-2-yl)pyrimidin-2-amine

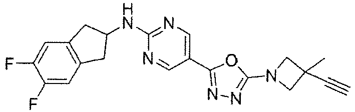

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (70.7 mg, 0.21 mmol), 3-ethynyl-3-methylazetidine, 2,2,2-trifluoroacetate (53.6 mg, 0.26 mmol), DIPEA (182 uL, 1.07 mmol), BOP reagent (113.3 mg, 0.26 mmol), and DMF (2 mL), the title compound was obtained as a yellow solid (60.0 mg, 68.8%).

LCMS m/z 409 [M+H]⁺

### Step 2: N-(5,6-difluoro-2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine

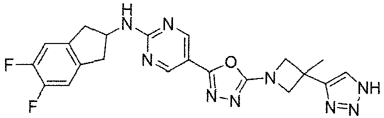

By the same method as in Step 1 of Preparation example 2, using N-(5,6-difluoro-2,3-dihydro-1H-inden-2-yl)-5-(5-(3-ethynyl-3-methylazetidin-1-yl)-1,3,4-oxadiazol-2-yl)pyrimidin-2-amine (22 mg, 0.05 mmol), TMSN₃ (7.5 mg, 0.06 mmol), CuI (0.51 mg, 0.003 mmol), and DMF/MeOH (1.8 mL/0.2 mL), the title compound was obtained as an off-white solid (5.15 mg, yield 21.2%).

¹H NMR (400 MHz, CD₃OD) *δ* 8.74 (broad, 2H), 7.84 (broad, 1H), 7.10 (t, J = 9.0 Hz, 2H), 4.84 - 4.78 (m, 1H), 4.49 (d, J = 7.7 Hz, 2H), 4.23 (d, J = 7.8 Hz, 2H), 3.34 (d, J = 7.3 Hz, 2H), 2.92 (dd, J = 15.9, 6.1 Hz, 2H), 1.81 (s, 3H); LCMS m/z 452 [M+H]⁺

### Example 52. Methyl 2-(1-(5-(2-((5,6-difluoro-2,3-dihydro-1H-inden-2-yl)amino)pyrimidin-5-yl)-1,3,4-oxadiazol-2-yl)azetidin-3-yl)acetate

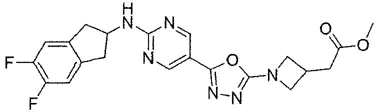

5-(2-((5,6-difluoro-2,3-dihydro-1H-indene-2-yl)amino)pyrimidin-5-yl)-1,3,4-oxadiazol-2(3H)-one (60 mg, 0.18 mmol) was dissolved in DMF (0.9 mL), methyl 2-(azetidin-3-yl)acetate hydrochloride (36 mg, 0.22 mmol) was added, and the mixture was cooled to 0 °C. Then, DIPEA (92 µL, 0.54 mmol) was added, and the mixture was stirred for 30 minutes, after which BOP reagent (96 mg, 0.22 mmol) was added, and the mixture was stirred at room temperature for 3 hours. DW was added to the reaction mixture, and the resulting solid was filtered and washed with DW and hexane. The filtered solid was dried to obtain the title compound as a white solid without further purification (59 mg, 74%).

¹H NMR (400 MHz, CDCl₃) *δ* 8.77 (s, 2H), 7.02 (t, J = 8.8 Hz, 2H), 5.63 (d, J = 7.6 Hz, 1H), 4.93 - 4.84 (m, 1H), 4.39 (t, J = 8.3 Hz, 2H), 3.93 (dd, J = 8.0, 5.8 Hz, 2H), 3.71 (s, 3H), 3.36 (dd, J = 15.8, 7.0 Hz, 2H), 3.26 - 3.17 (m, 1H), 2.85 (dd, J = 16.1, 5.3 Hz, 2H), 2.75 (d, J = 7.7 Hz, 2H); LCMS m/z 443 [M+H]⁺

### Example 53. 2-(1-(5-(2-((5,6-difluoro-2,3-dihydro-1H-inden-2-yl)amino)pyrimidin-5-yl)-1,3,4-oxadiazol-2-yl)azetidin-3-yl)acetic acid

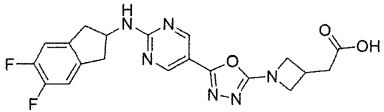

By the same method as in Example 50, methyl 2-(1-(5-(2-((5,6-difluoro-2,3-dihydro-1H-inden-2-yl)amino)pyrimidin-5-yl)-1,3,4-oxadiazol-2-yl)azetidin-3-yl)acetate (39 mg, 0.09 mmol), 1N NaOH (0.22 mL, 0.22 mmol), and THF (0.44 mL) was used to obtain the title compound as a white solid (23 mg, 61%).

¹H NMR (400 MHz, DMSO-d₆) *δ* 12.24 (s, 1H), 8.71 (s, 2H), 8.28 (d, J = 6.7 Hz, 1H), 7.29 (t, J = 9.3 Hz, 2H), 4.75 - 4.65 (m, 1H), 4.23 (t, J = 8.1 Hz, 2H), 3.90 - 3.80 (m, 2H), 3.25 (dd, J = 16.2, 7.4 Hz, 2H), 3.11 - 3.02 (m, 1H), 2.89 (dd, J = 16.1, 6.4 Hz, 2H), 2.67 (d, J = 7.8 Hz, 2H); LCMS m/z 429 [M+H]⁺

### Example 54. 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyridin-2-amine

### Step 1: 5-(6-((5,6-difluoro-2,3-dihydro-1H-inden-2-yl)amino)pyridin-3-yl)-1,3,4-oxadiazol-2(3H)-one

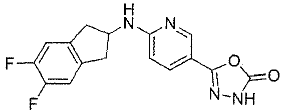

The title compound was prepared by the same method as in Preparation Example 3, except that 5,6-difluoro-2,3-dihydro-1H-inden-2-amine was used instead of 2,3-dihydro-1H-inden-2-amine and ethyl 6-chloronicotinate was used instead of ethyl 2-chloropyrimidine-5-carboxylate.

LCMS m/z 331 [M+H]⁺

### Step 2: (4-(1-(5-(6-((5,6-difluoro-2,3-dihydro-1H-inden-2-yl)amino)pyridin-3-yl)-1,3,4-oxadiazol-2-yl)azetidin-3-yl)-1H-1,2,3-triazol-1-yl)methyl pivalate

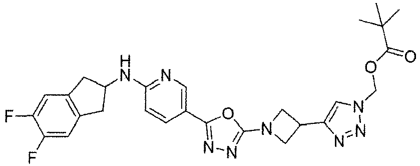

By the same method as in Step 1 of Example 1, using 5-(6-((5,6-difluoro-2,3-dihydro-1H-inden-2-yl)amino)pyridin-3-yl)-1,3,4-oxadiazol-2(3H)-one (30.5 mg, 0.092 mmol), (4-(3-methylpyrrolidin-3-yl)-1H-1,2,3-triazol-1-yl)methyl pivalate, 2,2,2-trifluoroacetate (39 mg, 0.11 mmol), DIPEA (158 uL, 0.92 mmol), BOP reagent (98 mg, 0.22 mmol), and DMF (3 mL), the title compound was obtained as an off-white solid (41.3 mg, 81.2%).

¹H NMR (400 MHz, CDCl₃) *δ* 8.60 (d, J = 1.8 Hz, 1H), 7.96 (dd, J = 8.8, 2.2 Hz, 1H), 7.76 (s, 1H), 7.02 (t, J = 8.8 Hz, 2H), 6.45 (d, J = 8.7 Hz, 1H), 6.23 - 6.22 (m, 2H), 5.08 (d, J = 7.4 Hz, 1H), 4.78 - 4.68 (m, 1H), 4.59 (t, J = 8.3 Hz, 2H), 4.44 - 4.37 (m, 2H), 4.26 - 4.18 (m, 1H), 3.37 (dd, J = 16.0, 6.9 Hz, 2H), 2.85 (dd, J = 16.0, 4.7 Hz, 2H), 1.20 (s, 9H); LCMS m/z 551 [M+H]⁺

### Step 3: 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyridin-2-amine

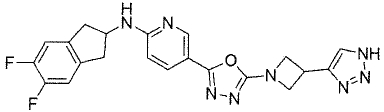

By the same method as in Step 2 of Example 1, using (4-(1-(5-(6-((5,6-difluoro-2,3-dihydro-1H-inden-2-yl)amino)pyridin-3-yl)-1,3,4-oxadiazol-2-yl)azetidin-3-yl)-1H-1,2,3-triazol-1-yl)methyl pivalate (35.1 mg, 0.064 mmol), K₂CO₃ (18 mg, 0.13 mmol), and MeOH (1.3 mL), the title compound was obtained as a white solid (17.8 mg, 63.7%).

¹H NMR (400 MHz, DMSO-d₆) *δ* 8.47 (d, J = 2.0 Hz, 2H), 7.86 (s, 1H), 7.78 (dd, J = 8.8, 2.2 Hz, 2H), 7.57 (d, J = 6.5 Hz, 2H), 7.31 (t, J = 9.3 Hz, 4H), 6.60 (d, J = 8.8 Hz, 2H), 4.74 - 4.63 (m, 1H), 4.56 - 4.46 (m, 2H), 4.21 (s, 3H), 3.28 (dd, J = 16.2, 7.3 Hz, 2H), 2.82 (dd, J = 16.1, 5.4 Hz, 2H); LCMS m/z 437 [M+H]⁺

### Example 55. 1-(5-(2-((5,6-difluoro-2,3-dihydro-1H-inden-2-yl)amino)pyrimidin-5-yl)-1,3,4-oxadiazol-2-yl)azetidin-3-yl methanesulfonate

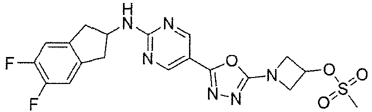

By the same method as in Example 27, using 1-(5-(2-((5,6-difluoro-2,3-dihydro-1H-inden-2-yl)amino)pyrimidin-5-yl)-1,3,4-oxadiazol-2-yl)azetidin-3-ol (21 mg, 0.054 mmol), MsCl (4.6 µL, 0.060 mmol), TEA (11 µL, 0.082 mmol), and DCM (0.14 mL), the title compound was obtained as a white solid (15 mg, 59.4%).

¹H NMR (400 MHz, DMSO-d₆) *δ* 8.71 (d, J = 13.7 Hz, 2H), 8.30 (d, J = 6.7 Hz, 1H), 7.27 (t, J = 9.3 Hz, 2H), 5.45 (ddd, J = 10.7, 6.7, 3.9 Hz, 1H), 4.73 - 4.63 (m, 1H), 4.57 - 4.50 (m, 2H), 4.26 (dd, J = 9.7, 4.3 Hz, 2H), 3.28 (s, 3H), 3.24 (dd, J = 16.1, 7.7 Hz, 2H), 2.87 (dd, J = 16.1, 6.4 Hz, 2H); LCMS m/z 465 [M+H]⁺

### Example 56. N-(5,6-difluoro-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

### Step 1: 3-ethynyl-3-fluoroazetidine, 2,2,2-trifluoroacetate

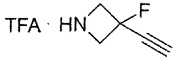

By the same method as in Step 2 of Preparation example 1, using tert-butyl 3-ethynyl-3-fluoroazetidine-1-carboxylate (199 mg, 1.0 mmol), TFA (3.3 mL), and DCM (10 mL), the title compound was obtained (quantitatively) and used in the next reaction without purification (196 mg).

### Step 2: N-(5,6-difluoro-2,3-dihydro-1H-inden-2-yl)-5-(5-(3-ethynyl-3-fluoroazetidin-1-yl)-1,3,4-oxadiazol-2-yl)pyrimidin-2-amine

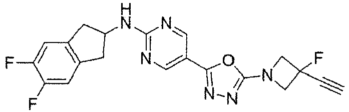

By the same method as in step 1 of example 1, using 5-(2-((5,6-difluoro-2,3-dihydro-1H-inden-2-yl)amino)pyrimidin-5-yl)-1,3,4-oxadiazol-2(3H)-one (232 mg, 0.7 mmol), 3-ethynyl-3-fluoroazetidine, 2,2,2-trifluoroacetate (196 mg, 1.0 mmol), BOP reagent (371 mg, 0.84 mmol), DIPEA (872 µL, 5.0 mmol), and DMF (5 mL) were used to obtain the title compound as a white solid (200 mg, 69%).

¹H NMR (400 MHz, DMSO-d₆) δ 8.73 (s, 2H), 7.27 (t, J = 8.0 Hz, 2H), 4.71 - 4.68 (m, 1H), 4.58 - 4.46 (m, 4H), 3.27 -3.21 (m, 2H), 2.90 - 2.85 (m, 2H); LCMS m/z 413 [M+H]⁺

### Step 3: N-(5,6-difluoro-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

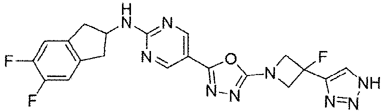

By the same method as in step 1 of Preparation example 2, N-(5,6-difluoro-2,3-dihydro-1H-inden-2-yl)-5-(5-(3-ethynyl-3-fluoroazetidin-1-yl)-1,3,4-oxadiazol-2-yl)pyrimidin-2-amine (100 mg, 0.24 mmol), TMSN₃ (38 uL, 0.29 mmol), CuI (5 mg, 0.0265 mmol), and DMF (3 mL)/MeOH (0.3 mL) were used to obtain the title compound as a white solid (10 mg, 9%).

¹H NMR (400 MHz, DMSO-d₆) δ 8.74 (s, 2H), 8.23 (s, 1H), 7.27 (t, J = 8.0 Hz, 2H), 4.71 - 4.63 (m, 5H), 3.27 -3.21 (m, 2H), 2.90 - 2.85 (m, 2H); LCMS m/z 456 [M+H]⁺

### Example 57. 1-(5-(2-((5,6-difluoro-2,3-dihydro-1H-inden-2-yl)amino)pyrimidin-5-yl)-1,3,4-oxadiazol-2-yl)azetidine-3-carbonitrile

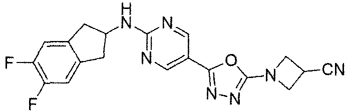

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (100 mg, 0.30 mmol), azetidine-3-carbonitrile, 2,2,2-trifluoroacetate (98 mg, 0.55 mmol), DIPEA (257 *µ* L, 1.51 mmol), BOP reagent (160 mg, 0.36 mmol), and DMF (3 mL), the title compound was obtained as a white solid (53 mg, 44%).

¹H NMR (400 MHz, DMSO-d₆) δ8.72 (d, *J =* 16.8 Hz, 2H), 8.32 (d, *J =* 6.7 Hz, 1H), 7.29 (t, *J =* 9.3 Hz, 2H), 4.76 - 4.64 (m, 1H), 4.45 - 4.37 (m, 2H), 4.36 - 4.29 (m, 2H), 4.05 - 3.96 (m, 1H), 3.25 (dd, *J* = 16.1, 7.5 Hz, 2H), 2.89 (dd, *J* = 16.1, 6.5 Hz, 2H); LCMS m/z 396 [M+H]⁺

### Example 58. N-(5,6-difluoro-2,3-dihydro-1H-inden-2-yl)-5-(5-(3-(5-(trifluoromethyl)-1,3,4-oxadiazol-2-yl)azetidin-1-yl)-1,3,4-oxadiazol-2-yl)pyrimidin-2-amine

### Step 1: tert-butyl 3-(1H-tetrazol-5-yl)azetidin-1-carboxylate

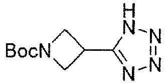

tert-Butyl 3-cyanoazetidine-1-carboxylate (1.0 g, 5.5 mmol) was dissolved in DMF (30 mL), NaN₃ (1.1 g, 18 mmol), NH₄Cl (0.94 g, 18 mmol) were added and stirred at 110 °C overnight. The reaction mixture was concentrated, distilled water was added, pH was adjusted to 1-2 with 1 N HCl, and extracted with EtOAc (3 times). The organic layers were combined, dried over MgSO₄, filtered and concentrated, and the obtained residue was purified by column chromatography (0-3% MeOH in DCM) to obtain the title compound as a color solid (1.2 g, 97%)..

LCMS m/z 224 [M-H]⁻

### Step 2: tert-butyl 3-(5-(trifluoromethyl)-1,3,4-oxadiazol-2-yl)azetidine-1-carboxylate

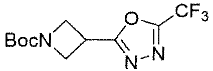

tert-Butyl 3-(1H-tetrazol-5-yl)azetidine-1-carboxylate (30 mg, 0.13 mmol) was dissolved in DCM (2 mL), trifluoroacetic anhydride (28 µL, 0.20 mmol) was added at 0 °C, and the mixture was stirred at room temperature for 1.5 h. The reaction mixture was concentrated to afford the title compound as a colorless oil (quantitatively), which was used in the next reaction without purification (39 mg).

¹H NMR (400 MHz, DMSO-d₆) δ4.29 - 4.10 (m, 4H), 3.85 (s, 1H), 1.40 (s, 9H).

### Step 3: 2-(azetidin-3-yl)-5-(trifluoromethyl)-1,3,4-oxadiazole, 2,2,2-trifluoroacetate

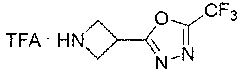

By the same method as in Step 2 of Preparation example 1, using tert-butyl 3-(5-(trifluoromethyl)-1,3,4-oxadiazol-2-yl)azetidine-1-carboxylate (39 mg, 0.13 mmol), TFA (1 mL), and DCM (2 mL), the title compound was obtained as a colorless oil (quantitatively), which was used in the next reaction without purification (41 mg).

### Step 4: N-(5,6-difluoro-2,3-dihydro-1H-inden-2-yl)-5-(5-(3-(5-(trifluoromethyl)-1,3,4-oxadiazol-2-yl)azetidin-1-yl)-1,3,4-oxadiazol-2-yl)pyrimidin-2-amine

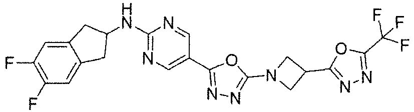

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (37 mg, 0.11 mmol), 2-(azetidin-3-yl)-5-(trifluoromethyl)-1,3,4-oxadiazole, 2,2,2-trifluoroacetate (39 mg, 0.13 mmol), DIPEA (94 µL, 0.56 mmol), BOP reagent (59 mg, 0.13 mmol), and DMF (2 mL), the title compound was obtained as a white solid (35 mg, 62%).

¹H NMR (400 MHz, DMSO-d₆) δ8.74 (d, *J* = 13.6 Hz, 2H), 8.31 (d, *J* = 6.8 Hz, 1H), 7.29 (t, *J =* 9.2 Hz, 2H), 4.76 - 4.66 (m, 1H), 4.62 - 4.41 (m, 5H), 3.26 (dd, *J* = 16.0, 7.5 Hz, 2H), 2.89 (dd, *J* = 16.0, 6.5 Hz, 2H); LCMS m/z 507 [M+H]⁺

### Example 59. 5-(5-(3-(1H-1,2,3-triazol-4-yl)azetidin-1-yl)-1,3,4-oxadiazol-2-yl)-N-(3,5-dichlorophenethyl)pyrimidin-2-amine

### Step 1: (4-(1-(5-(2-((3,5-dichlorophenethyl)amino)pyrimidin-5-yl)-1,3,4-oxadiazol-2-yl)azetidin-3-yl)-1H-1,2,3-triazol-1-yl)methyl pivalate

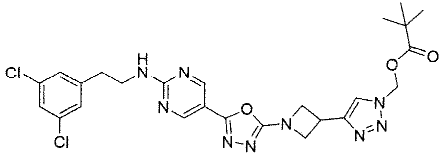

By the same method as in Step 1 of Example 1, using 5-(2-((3,5-dichlorophenethyl)amino)pyrimidin-5-yl)-1,3,4-oxadiazol-2(3H)-one (36.7 mg, 0.1 mmol), (4-(azetidin-3-yl)-1H-1,2,3-triazol-1-yl)methyl pivalate, 2,2,2-trifluoroacetate (44 mg, 0.13 mmol), DIPEA (89 uL, 0.52 mmol), BOP reagent (55.3 mg, 0.13 mmol), and DMF (1.5 mL), the title compound was obtained as a white solid (43.4 mg, 72.8%).

¹H NMR (400 MHz, CDCl₃) *δ* 8.78 (s, 2H), 7.76 (s, 1H), 7.24 (s, 1H), 7.13 (d, J = 1.6 Hz, 2H), 6.23 (s, 2H), 5.62 (t, J = 6.0 Hz, 1H), 4.60 (t, J = 8.3 Hz, 2H), 4.42 (t, J = 7.2 Hz, 2H), 4.28 - 4.19 (m, 1H), 3.74 (q, J = 6.8 Hz, 2H), 2.94 - 2.87 (m, 2H), 1.20 (s, 9H); LCMS m/z 572 [M+H]⁺

### Step 2: 5-(5-(3-(1H-1,2,3-triazol-4-yl)azetidin-1-yl)-1,3,4-oxadiazol-2-yl)-N-(3,5-dichlorophenethyl)pyrimidin-2-amine

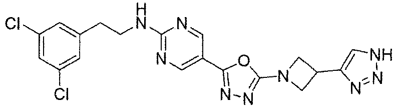

By the same method as in Step 2 of Example 1, using (4-(1-(5-(2-((3,5-dichlorophenethyl)amino)pyrimidin-5-yl)-1,3,4-oxadiazol-2-yl)azetidin-3-yl)-1H-1,2,3-triazol-1-yl)methyl pivalate (33.5 mg, 0.059 mmol), K₂CO₃ (16 mg, 0.12 mmol), and MeOH (2 mL), the title compound was obtained as a white solid (17.8 mg, 63.7%).

¹H NMR (400 MHz, DMSO-d₆) *δ* 8.68 (d, J = 9.2 Hz, 2H), 8.01 (t, J = 5.7 Hz, 1H), 7.92 (s, 1H), 7.42 (s, 1H), 7.32 (d, J = 1.4 Hz, 2H), 4.56 - 4.48 (m, 2H), 4.28 - 4.18 (m, 3H), 3.58 (q, J = 6.8 Hz, 2H), 2.88 (t, J = 6.8 Hz, 2H); LCMS m/z 458 [M+H]⁺

### Example 60. 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrazin-2-amine

### Step 1: (4-(1-(5-(5-((5,6-difluoro-2,3-dihydro-1H-inden-2-yl)amino)pyrazin-2-yl)-1,3,4-oxadiazol-2-yl)azetidin-3-yl)-1H-1,2,3-triazol-1-yl)methyl pivalate

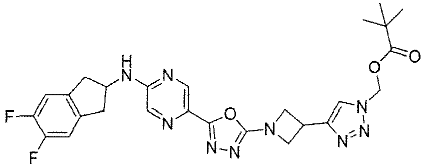

By the same method as in Step 1 of Example 1, using 5-(5-((5,6-difluoro-2,3- dihydro-1H-inden-2-yl)amino)pyrazin-2-yl)-1,3,4-oxadiazol-2(3H)-one (32 mg, 0.097 mmol), (4-(azetidin-3-yl)-1H-1,2,3-triazol-1-yl)methyl pivalate, 2,2,2-trifluoroacetate (41 mg, 0.12 mmol), DIPEA (82 uL, 0.48 mmol), BOP reagent (51.3 mg, 0.12 mmol), and DMF (1.5 mL), the title compound was obtained as a brown oil (49.5 mg, 93%).

LCMS m/z 552 [M+H]⁺

### Step 2: 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrazin-2-amine

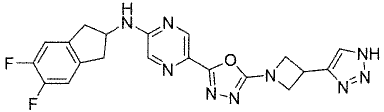

By the same method as in Step 2 of Example 1, using (4-(1-(5-(5-((5,6-difluoro-2,3-dihydro-1H-inden-2-yl)amino)pyrazin-2-yl)-1,3,4-oxadiazol-2-yl)azetidin-3-yl)-1H-1,2,3-triazol-1-yl)methyl pivalate (43.5 mg, 0.079 mmol), K₂CO₃ (22 mg, 0.16 mmol), and MeOH (1.5 mL), the title compound was obtained as a brown solid (11.7 mg, 33.9%).

¹H NMR (400 MHz, DMSO-d₆) *δ* 8.58 (s, 1H), 8.10 (d, J = 6.5 Hz, 1H), 7.99 (s, 1H), 7.94 (broad, 1H), 7.33 (t, J = 9.2 Hz, 2H), 4.72 - 4.61 (m, 1H), 4.58 - 4.46 (m, 2H), 4.22 (s, 3H), 3.28 (dd, J = 7.1 Hz, 2H), 2.84 (dd, J = 16.2, 4.9 Hz, 2H); LCMS m/z 438 [M+H]⁺

### Example 61. N-(5,6-difluoro-2,3-dihydro-1H-inden-2-yl)-5-(5-(3-(5-(difluoromethyl)-1,3,4-oxadiazol-2-yl)azetidin-1-yl)-1,3,4-oxadiazol-2-yl)pyrimidin-2-amine

### Step 1: tert-butyl 3-(5-(difluoromethyl)-1,3,4-oxadiazol-2-yl)azetidine-1-carboxylate

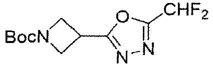

By the same method as in Step 2 of Example 58, tert-butyl 3-(1H-tetrazol-5-yl)azetidine-1-carboxylate (100 mg, 0.44 mmol), 2,2-difluoroacetic anhydride (73 µL, 0.67 mmol), and DCM (5 mL) were used for the reaction, and the residue was purified by column chromatography (0-3% MeOH /DCM) to obtain the title compound as a colorless oil (53 mg, 43%).

¹H NMR (400 MHz, DMSO-d₆) δ7.47 (t, J = 51.3 Hz, 1H), 4.29 - 4.05 (m, 5H), 1.39 (s, 9H)

### Step 2: 2-(azetidin-3-yl)-5-(difluoromethyl)-1,3,4-oxadiazole, 2,2,2-trifluoroacetate

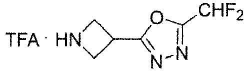

By the same method as in step 2 of Preparation example 1, tert-butyl 3-(5-(difluoromethyl)-1,3,4-oxadiazol-2-yl)azetidine-1-carboxylate (50 mg, 0.18 mmol), TFA (1 mL) and DCM (2 mL) were used to give the title compound as a colorless oil (quantitatively) which was used in the next reaction without purification (58 mg).

LCMS m/z 176 [M+H]⁺

### Step 3: N-(5,6-difluoro-2,3-dihydro-1H-inden-2-yl)-5-(5-(3-(5-(difluoromethyl)-1,3,4-oxadiazol-2-yl)azetidin-1-yl)-1,3,4-oxadiazol-2-yl)pyrimidin-2-amine

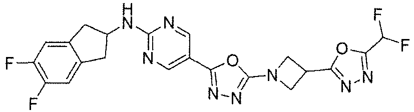

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (35 mg, 0.11 mmol), 2-(azetidin-3-yl)-5-(difluoromethyl)-1,3,4-oxadiazole, 2,2,2-trifluoroacetate (35 mg, 0.13 mmol), DIPEA (91 *µ* L, 0.53 mmol), BOP reagent (56 mg, 0.13 mmol), and DMF (1 mL), the title compound was obtained as a white solid (39 mg, 75%).

¹H NMR (400 MHz, DMSO-d₆) δ8.74 (d, *J* = 14.9 Hz, 2H), 8.31 (d, *J* = 6.7 Hz, 1H), 7.50 (t, *J* = 51.3 Hz, 1H), 7.29 (t, *J* = 9.3 Hz, 2H), 4.75 - 4.65 (m, 1H), 4.64 - 4.55 (m, 2H), 4.54 - 4.45 (m, 1H), 4.45 - 4.37 (m, 2H), 3.25 (dd, *J* = 16.1, 7.5 Hz, 2H), 2.89 (dd, *J* = 16.1, 6.5 Hz, 2H); LCMS m/z 489 [M+H]⁺

### Example 62. (4-(1-(5-(2-((5-bromo-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

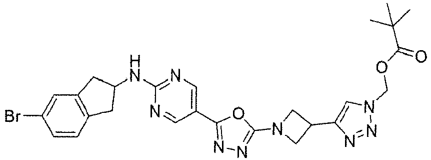

By the same method as in step 1 of Example 1, 5-(2-((5-bromo-2,3-dihydro-1H-inden-2-yl)amino)pyrimidin-5-yl)-1,3,4-oxadiazol-2(3H)-one (68.0 mg, 0.18 mmol), (4-(azetidin-3-yl)-1H-1,2,3-triazol-1-yl)methyl pivalate, 2,2,2-trifluoroacetate (100 mg, 0.28 mmol), DIPEA (158 uL, 0.93 mmol), BOP reagent (96.5 mg, 0.22 mmol), and DMF (3.60 mL) were used to obtain the title compound as a white solid (83 mg, 77%).

¹H NMR (400 MHz, DMSO-d₆) δ 8.80 - 8.65 (m, 2H), 8.32 - 8.24 (m, 2H), 7.43 (s, 1H), 7.33 (d, J = 7.7 Hz, 1H), 7.19 (d, J = 8.3 Hz, 1H), 6.30 (s, 2H), 4.73 - 4.64 (m, 1H), 4.56 - 4.48 (m, 2H), 4.28 - 4.18 (m, 3H), 3.28 - 3.20 (m, 2H), 2.91 (ddd, J = 22.4, 15.7, 6.5 Hz, 2H), 1.13 (s, 9H); LCMS m/z 594 [M+H]⁺

### Example 63. (4-(1-(5-(2-((5-fluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

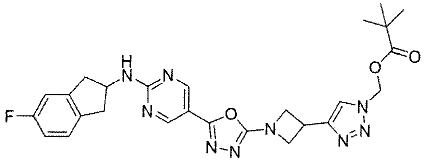

By the same method as in step 1 of Example 1, 5-(2-((5-fluoro-2,3-dihydro-1H-inden-2-yl)amino)pyrimidin-5-yl)-1,3,4-oxadiazol-2(3H)-one (55.0 mg, 0.18 mmol), (4-(azetidin-3-yl)-1H-1,2,3-triazol-1-yl)methyl pivalate, 2,2,2-trifluoroacetate (100 mg, 0.28 mmol), DIPEA (152 uL, 0.89 mmol), BOP reagent (93.0 mg, 0.21 mmol), and DMF (3.50 mL) were used to obtain the title compound as a white solid (66 mg, 71%).

¹H NMR (400 MHz, DMSO-d₆) δ 8.72 (s, 2H), 8.30 (s, 1H), 8.29 (d, J = 6.9 Hz, 1H), 7.23 (dd, J = 8.3, 5.4 Hz, 1H), 7.06 (d, J = 9.0 Hz, 1H), 6.96 (t, J = 8.7 Hz, 1H), 6.30 (s, 2H), 4.75 - 4.66 (m, 1H), 4.52 (s, 2H), 4.24 (d, J = 3.3 Hz, 3H), 3.24 (dd, J = 16.3, 7.3 Hz, 2H), 2.98 - 2.83 (m, 2H), 1.13 (s, 9H); LCMS m/z 534 [M+H]⁺

### Example 64. 5-(5-(3-(1H-1,2,3-triazol-4-yl)azetidin-1-yl)-1,3,4-oxadiazol-2-yl)-N-(5-bromo-2,3-dihydro-1H-inden-2-yl)pyrimidin-2-amine

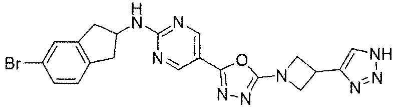

By the same method as in Step 2 of Example 1, using (4-(1-(5-(2-((5-bromo-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (50.0 mg, 0.08 mmol), K₂CO₃ (23.0 mg, 0.17 mmol), and MeOH (1.68 mL), the title compound was obtained as a white solid (33 mg, 82%).

¹H NMR (400 MHz, DMSO-d₆) δ 8.82 - 8.64 (m, 2H), 8.28 (d, J = 6.8 Hz, 1H), 7.92 (s, 1H), 7.43 (s, 1H), 7.33 (d, J = 7.7 Hz, 1H), 7.19 (d, J = 7.8 Hz, 1H), 4.73 - 4.64 (m, 1H), 4.52 (s, 2H), 4.22 (s, 3H), 3.23 (dd, J = 16.0, 6.7 Hz, 2H), 2.91 (ddd, J = 22.1, 16.0, 6.3 Hz, 2H); LCMS m/z 480 [M+H]⁺

### Example 65. 5-(5-(3-(1H-1,2,3-triazol-4-yl)azetidin-1-yl)-1,3,4-oxadiazol-2-yl)-N-(5-fluoro-2,3-dihydro-1H-inden-2-yl)pyrimidin-2-amine

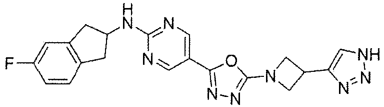

The title compound was obtained as a white solid (30 mg, 86%) by the same method as in Step 2 of Example 1, using (4-(1-(5-(2-((5-fluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (44.8 mg, 0.08 mmol), K₂CO₃ (23.0 mg, 0.17 mmol), and MeOH (1.68 mL).

¹H NMR (400 MHz, DMSO-d₆) δ 8.73 (d, J = 13.3 Hz, 2H), 8.29 (d, J = 6.8 Hz, 1H), 7.93 (s, 1H), 7.23 (dd, J = 8.1, 5.8 Hz, 1H), 7.06 (d, J = 9.1 Hz, 1H), 7.00 - 6.92 (m, 1H), 4.75 - 4.65 (m, 1H), 4.57 - 4.47 (m, 2H), 4.28 - 4.17 (m, 3H), 3.24 (dd, J = 16.0, 7.4 Hz, 2H), 2.99 - 2.83 (m, 2H); LCMS m/z 420 [M+H]⁺

### Example 66. 5-(1-(5-(2-((5,6-difluoro-2,3-dihydro-1H-inden-2-yl)amino)pyrimidin-5-yl)-1,3,4-oxadiazol-2-yl)azetidin-3-yl)-1,3,4-oxadiazol-2(3H)-one

### Step 1: 1-(5-(2-((5,6-difluoro-2,3-dihydro-1H-inden-2-yl)amino)pyrimidin-5-yl)-1,3,4-oxadiazol-2-yl)azetidine-3-carbohydrazide

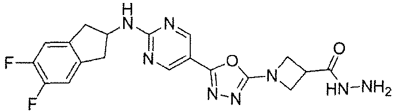

By the same method as in Step 1 of Example 23, using methyl 1-(5-(2-((5,6-difluoro-2,3-dihydro-1H-inden-2-yl)amino)pyrimidin-5-yl)-1,3,4-oxadiazol-2-yl)azetidine-3-carboxylate (62 mg, 0.14 mmol), hydrazine monohydrate (0.3 mL, 2.9 mmol), and EtOH (3 mL), the title compound was obtained as a white solid (44.9 mg, 72.4%).

LCMS m/z 429 [M+H]⁺

### Step 2: 5-(1-(5-(2-((5,6-difluoro-2,3-dihydro-1H-inden-2-yl)amino)pyrimidin-5-yl)-1,3,4-oxadiazol-2-yl)azetidin-3-yl)-1,3,4-oxadiazol-2(3H)-one

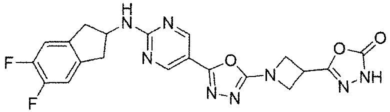

By the same method as in Step 2 of Example 23, using 1-(5-(2-((5,6-difluoro -2,3-dihydro-1H-inden-2-yl)amino)pyrimidin-5-yl)-1,3,4-oxadiazol-2-yl)azetidine-3-carbohydrazide (39 mg, 0.091 mmol), TEA (0.013 mL, 0.091 mmol), CDI (18 mg, 0.11 mmol), and THF (1.5 mL), the title compound was obtained as a white solid (11.4 mg, 27.5%).

¹H NMR (400 MHz, DMSO-d₆) *δ* 12.30 (broad, 1H), 8.73 (d, J = 17.5 Hz, 2H), 8.31 (d, J = 6.7 Hz, 1H), 7.29 (t, J = 9.3 Hz, 2H), 4.70 (m, 1H), 4.52 - 4.41 (m, 2H), 4.37 - 4.24 (m, 2H), 4.12 - 3.98 (m, 1H), 3.26 (dd, J = 16.2, 7.5 Hz, 2H), 2.89 (dd, J = 16.0, 6.6 Hz, 2H); LCMS m/z 455 [M+H]⁺

### Example 67. 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-4-methylpyrimidin-2-amine

### Step 1: (4-(1-(5-(2-((5,6-difluoro-2,3-dihydro-1H-inden-2-yl)amino)-4-methylpyrimidin-5-yl)-1,3,4-oxadiazol-2-yl)azetidin-3-yl)-1H-1,2,3-triazol-1-yl)methyl pivalate

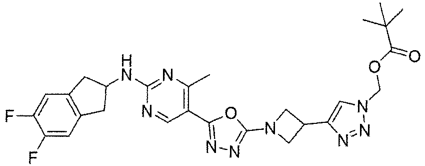

By the same method as in Step 1 of Example 1, using 5-(2-((5,6-difluoro-2,3-dihydro-1H-inden-2-yl)amino)-4-methylpyrimidin-5-yl)-1,3,4-oxadiazol-2(3H)-one (39.2 mg, 0.11 mmol), (4-(azetidin-3-yl)-1H-1,2,3-triazol-1-yl)methyl pivalate, 2,2,2-trifluoroacetate (48 mg, 0.14 mmol), DIPEA (97 uL, 0.57 mmol), BOP reagent (60.3 mg, 0.14 mmol), and DMF (2 mL), the title compound was obtained as a brown oil (34.8 mg, 54.2%).

¹H NMR (400 MHz, CDCl₃) *δ* 8.61 (broad, 1H), 7.76 (s, 1H), 7.02 (t, J = 8.8 Hz, 2H), 6.23 (s, 2H), 5.61 (d, J = 7.8 Hz, 1H), 4.96 - 4.85 (m, 1H), 4.59 (t, J = 8.3 Hz, 2H), 4.41 (t, J = 7.2 Hz, 2H), 4.28 - 4.19 (m, 1H), 3.36 (dd, J = 16.1, 7.0 Hz, 2H), 2.84 (dd, J = 15.9, 5.3 Hz, 2H), 2.69 (s, 3H), 1.20 (s, 9H)

### Step 2: 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-4-methylpyrimidin-2-amine

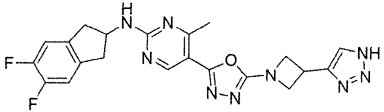

By the same method as in Step 2 of Example 1, using (4-(1-(5-(2-((5,6-difluoro-2,3-dihydro-1H-inden-2-yl)amino)-4-methylpyrimidin-5-yl)-1,3,4-oxadiazol-2-yl)azetidin-3-yl)-1H-1,2,3-triazol-1-yl)methyl pivalate (30.6 mg, 0.054 mmol), K₂CO₃ (15 mg, 0.11 mmol), and MeOH (2 mL), the title compound was obtained as a light brown solid (11.7 mg, 33.9%).

¹H NMR (400 MHz, DMSO-d₆) *δ* 8.59 (d, J = 18.0 Hz, 1H), 8.16 (s, 1H), 7.86 (s, 1H), 7.28 (t, J = 9.3 Hz, 2H), 4.71 (s, 1H), 4.52 (s, 2H), 4.23 (s, 3H), 3.30 - 3.19 (m, 2H), 2.88 (dd, J = 16.0, 6.7 Hz, 2H), 2.65 - 2.53 (m, 3H); LCMS m/z 452 [M+H]⁺

### Example 68. 5-(5-(3-(1H-tetrazol-5-yl)azetidin-1-yl)-1,3,4-oxadiazol-2-yl)-N-(5,6-difluoro-2,3-dihydro-1H-inden-2-yl)pyrimidin-2-amine

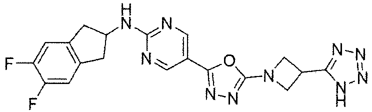

1-(5-(2-((5,6-Difluoro-2,3-dihydro-1H-inden-2-yl)amino)pyrimidin-5-yl)-1,3,4-oxadiazol-2-yl)azetidine-3-carbonitrile (101 mg, 0.25 mmol) was dissolved in DMF (3 mL), NaN₃ (53 mg, 0.81 mmol), NH ₄ Cl (29 mg, 0.81 mmol) were added, and the mixture was stirred at 80 °C overnight. After concentrating the reaction mixture, the obtained residue was purified by column chromatography (0-20% MeOH in DCM) to give the title compound as a white solid (7 mg, 6.3%).

¹H NMR (400 MHz, DMSO-d₆) δ8.70 (s, 5H), 8.30 (d, *J* = 6.7 Hz, 2H), 7.28 (t, *J* = *9.2* Hz, 5H), 4.73 - 4.66 (m, 3H), 4.62 - 4.55 (m, 4H), 4.49 - 4.43 (m, 2H), 4.39 - 4.31 (m, 4H), 3.27 - 3.21 (m, 5H), 2.88 (dd, *J* = 16.1, 6.6 Hz, 5H); LCMS m/z 439 [M+H]⁺

### Example 69. N-(2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine

### Step 1: N-(2,3-dihydro-1H-inden-2-yl)-5-(5-(3-ethynyl-3-methylazetidin-1-yl)-1,3,4-oxadiazol-2-yl)pyrimidin-2-amine

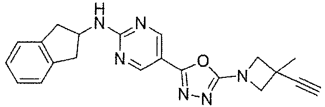

By the same method as in Step 1 of Example 1, 5-(2-((2,3-dihydro-1H-inden-2-yl)amino)pyrimidin-5-yl)-1,3,4-oxadiazol-2(3H)-one (65 mg, 0.22 mmol), 3-ethynyl-3-methylazetidine, 2,2,2-trifluoroacetate (55 mg, crude), DIPEA (190 uL, 1.1 mmol), BOP reagent (116.7 mg, 0.26 mmol), and DMF (2 mL) were used to obtain the title compound as an off-white solid (78.0 mg, 95.3%).

LCMS m/z 373 [M+H]⁺

### Step 2: N-(2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine

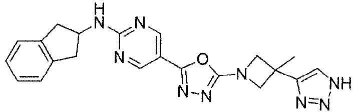

By the same method as in Step 1 of Preparation Example 2, using N-(5,6-difluoro-2,3-dihydro-1H-inden-2-yl)-5-(5-(3-ethynyl-3-methylazetidin-1-yl)-1,3,4-oxadiazol-2-yl)pyrimidin-2-amine (67 mg, 0.18 mmol), TMSN₃ (25 mg, 0.22 mmol), CuI (1.7 mg, 0.01 mmol), and DMF/MeOH (5.4 mL/0.6 mL), the title compound was obtained as a white solid (10.7 mg, 14.3%).

¹H NMR (400 MHz, DMSO-d₆) *δ* 8.73 (d, J = 18.1 Hz, 2H), 8.27 (d, J = 6.9 Hz, 1H), 7.86 (s, 1H), 7.28 - 7.10 (m, 4H), 4.74 - 4.64 (m, 1H), 4.41 - 4.29 (m, 2H), 4.15 (d, J = 7.6 Hz, 2H), 3.27 (dd, J = 15.9, 7.6 Hz, 2H), 2.93 (dd, J = 15.8, 6.8 Hz, 2H), 1.72 (s, 3H); LCMS m/z 416 [M+H]⁺

### Example 70. (4-(1-(5-(2-((5-methoxy-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

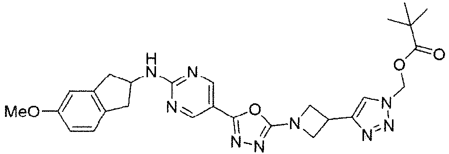

By the same method as in Step 1 of Example 1, using 5-(2-((5-methoxy-2,3-dihydro-1H-inden-2-yl)amino)pyrimidin-5-yl)-1,3,4-oxadiazol-2(3H)-one (30 mg, 0.092 mmol), (4-(azetidin-3-yl)-1H-1,2,3-triazol-1-yl)methyl pivalate, 2,2,2-trifluoroacetate (35 mg, 0.092 mmol), DIPEA (78 µL, 0.46 mmol), BOP reagent (49 mg, 0.11 mmol), and DMF (1 mL), the title compound was obtained as an off-white solid (34 mg, 68%).

¹H NMR (400 MHz, DMSO-d₆) δ8.72 (d, *J* = 18.9 Hz, 2H), 8.30 (s, 1H), 8.26 (d, *J=* 6.9 Hz, 1H), 7.11 (d, *J* = 8.3 Hz, 1H), 6.81 (s, 1H), 6.71 (d, *J* = 8.3 Hz, 1H), 6.30 (s, 2H), 4.71 - 4.62 (m, 1H), 4.52 (s, 2H), 4.28 - 4.19 (m, 3H), 3.71 (s, 3H), 3.26 - 3.16 (m, 2H), 2.92 - 2.80 (m, 2H), 1.12 (s, 9H); LCMS m/z 546[M+H]⁺

### Example 71. 5-(5-(3-(1H-1,2,3-triazol-4-yl)azetidin-1-yl)-1,3,4-oxadiazol-2-yl)-N-(5-methoxy-2,3-dihydro-1H-inden-2-yl)pyrimidin-2-amine

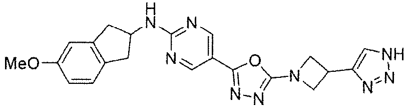

By the same method as in Step 2 of Example 1, using (4-(1-(5-(2-((5-methoxy-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (28 mg, 0.051 mmol), K₂CO₃ (14 mg, 0.10 mmol), and MeOH (1 mL), the title compound was obtained as a white solid (15 mg, 68%).

¹H NMR (400 MHz, DMSO-d₆) δ8.72 (d, *J =* 17.1 Hz, 2H), 8.25 (d, *J* = 6.9 Hz, 1H), 7.93 (s, 1H), 7.10 (d, *J* = 8.2 Hz, 1H), 6.81 (s, 1H), 6.71 (d, *J* = 8.3 Hz, 1H), 4.74 - 4.62 (m, 1H), 4.55 - 4.47 (m, 2H), 4.23 (s, 3H), 3.71 (s, 3H), 3.23 - 3.16 (m, 2H), 2.93 - 2.79 (m, 2H); LCMS m/z 432[M+H]⁺

### Example 72. (4-(1-(5-(2-((6,7-dihydro-5H-cyclopenta[b]pyrazin-6-yl)amino)pyrimidin-5-yl)-1,3,4-oxadiazol-2-yl)azetidin-3-yl)-1H-1,2,3-triazol-1-yl)methyl pivalate

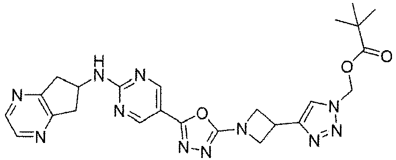

By the same method as in step 1 of Example 1, 5-(2-((6,7-dihydro-5H-cyclopenta[b]pyrazin-6-yl)amino)pyrimidin-5-yl)-1,3,4-oxadiazol-2(3H)-one (50 mg, 0.17 mmol), (4-(azetidin-3-yl)-1H-1,2,3-triazol-1-yl)methyl pivalate, 2,2,2-trifluoroacetate (96 mg, 0.27 mmol), DIPEA (146 uL, 0.86 mmol), BOP reagent (89 mg, 0.20 mmol), and DMF (3.4 mL) were used to obtain the title compound as a white solid (23 mg, 26%).

¹H NMR (400 MHz, DMSO-d₆) *δ* 8.75 (s, 2H), 8.40 - 8.33 (m, 2H), 8.31 (s, 1H), 7.92 (s, 1H), 6.30 (s, 2H), 4.87 - 4.77 (m, 1H), 4.57 - 4.46 (m, 2H), 4.29 - 4.17 (m, 3H), 3.43 (dd, J = 17.4, 7.9 Hz, 2H), 3.05 (dd, J = 17.2, 5.5 Hz, 2H), 1.13 (s, 9H); LCMS m/z 518 [M+H]⁺

### Example 73. (4-(1-(5-(2-((6,7-dihydro-5H-cyclopenta[b]pyridin-6-yl)amino)pyrimidin-5-yl)-1,3,4-oxadiazol-2-yl)azetidin-3-yl)-1H-1,2,3-triazol-1-yl)methyl pivalate

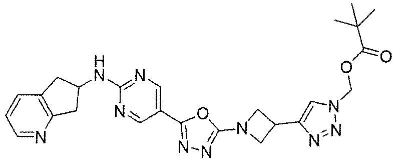

By the same method as in step 1 of Example 1, 5-(2-((6,7-dihydro-5H-cyclopenta[b]pyridin-6-yl)amino)pyrimidin-5-yl)-1,3,4-oxadiazol-2(3H)-one (55 mg, 0.19 mmol), (4-(azetidin-3-yl)-1H-1,2,3-triazol-1-yl)methyl pivalate, 2,2,2-trifluoroacetate (106 mg, 0.30 mmol), DIPEA (161 uL, 0.95 mmol), BOP reagent (99 mg, 0.22 mmol), and DMF (3.7 mL) were used to obtain the title compound as a white solid (54 mg, 56%).

¹H NMR (400 MHz, DMSO-d₆) *δ* 8.73 (s, 2H), 8.37 - 8.25 (m, 3H), 7.61 (d, J = 7.5 Hz, 1H), 7.15 (dd, J = 7.4, 5.0 Hz, 1H), 6.30 (s, 2H), 4.78 - 4.67 (m, 1H), 4.57 - 4.47 (m, 2H), 4.29 - 4.19 (m, 3H), 3.38 - 3.27 (m, 2H), 2.98 (ddd, J = 22.5, 16.3, 6.1 Hz, 2H), 1.13 (s, 9H); LCMS m/z 517 [M+H]⁺

### Example 74. N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyrazin-6-amine

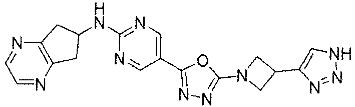

By the same method as in Step 3 of Example 7, using (4-(1-(5-(2-((6,7-dihydro-5H-cyclopenta[b]pyrazin-6-yl)amino)pyrimidin-5-yl)-1,3,4-oxadiazol-2-yl)azetidin-3-yl)-1H-1,2,3-triazol-1-yl)methyl pivalate (18 mg, 0.035 mmol), K₂CO₃ (9.6 mg, 0.07 mmol), and MeOH (0.7 mL), the title compound was obtained as a white solid (9 mg, 64%).

¹H NMR (400 MHz, DMSO-d₆) *δ* 8.75 (s, 2H), 8.45 - 8.28 (m, 3H), 7.92 (s, 1H), 4.87 - 4.77 (m, 1H), 4.58 - 4.47 (m, 2H), 4.29 - 4.17 (m, 3H), 3.43 (dd, J = 17.3, 8.0 Hz, 2H), 3.05 (dd, J = 17.3, 5.5 Hz, 2H); LCMS m/z 404 [M+H]⁺

### Example 75. N-(5,6-difluoro-2,3-dihydro-1H-inden-2-yl)-5-(5-(3-(5-methyl-1H-1,2,3-triazol-4-yl)azetidin-1-yl)-1,3,4-oxadiazol-2-yl)pyrimidin-2-amine

### Step 1: tert-butyl 3-(prop-1-yn-1-yl)azetidine-1-carboxylate

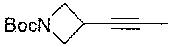

tert-Butyl 3-ethynyl azetidine-1-carboxylate (500 mg, 2.76 mmol) in THF (8 mL) and cooled to -78 °C under an atmosphere of N₂, n-BuLi (2.5 M in hexanes, 1.10 mL, 2.78 mmol) was slowly added dropwise over 10 minutes and stirred for 30 minutes. After warming to room temperature, CH₃I (259 uL, 4.14 mmol) was slowly added dropwise over 5 minutes and stirred for 5 hours. DW was added to the reaction mixture and extracted with EtOAc, and the organic layer was dried over Na₂SO₄, filtered, and concentrated. The obtained residue was purified by column chromatography (0-3% MeOH/DCM) to obtain the labeled compound as a yellow oil (500 mg, 93%).

LCMS m/z 196 [M+H]⁺

### Step 2: tert-butyl 3-(5-methyl-1H-1,2,3-triazol-4-yl)azetidine-1-carboxylate

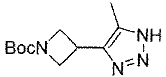

tert-butyl 3-(prop-1-yn-1-yl)azetidine-1-carboxylate (195 mg, 1.0 mmol), TMSN₃ (200 uL, 1.55 mmol) were added to the reaction vessel and reacted in a microwave reactor (200 °C, 2 h). The reaction mixture was purified by column chromatography (0-5% MeOH in DCM) to obtain the title compound as a brown oil. (19 mg, 8%).

¹H NMR (400 MHz, CDCl₃) δ 4.33 - 4.28 (m, 2H), 4.18 - 4.14 (m, 2H), 3.85 - 3.79 (m, 1H), 2.28 (s, 3H), 1.45 (s, 9H)

### Step 3: 4-(azetidin-3-yl)-5-methyl-1H-1,2,3-triazole, 2,2,2-trifluoroacetate

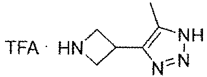

By the same method as step 2 of Preparation example 1, using tert-Butyl 3-(5-methyl-1H-1,2,3-triazol-4-yl)azetidine-1-carboxylate (19 mg, 0.079 mmol), TFA (350 uL), and DCM (1 mL), the title compound was obtained (quantitatively) which was used in the next reaction without purification (19 mg).

### Step 4: N-(5,6-difluoro-2,3-dihydro-1H-inden-2-yl)-5-(5-(3-(5-methyl-1H-1,2,3-triazol-4-yl)azetidin-1-yl)-1,3,4-oxadiazol-2-yl)pyrimidin-2-amine

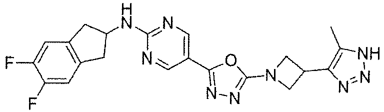

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (26 mg, 0.079 mmol), 4-(azetidin-3-yl)-5-methyl-1H-1,2,3-triazole, 2,2,2-trifluoroacetate (19 mg, 0.079 mmol), BOP reagent (58 mg, 0.13 mmol), DIPEA (77 µL, 0.43 mmol), and DMF (1 mL), the title compound was obtained as a brown solid (7 mg, 20%).

¹H NMR (400 MHz, CD₃OD) δ 8.72 (s, 2H), 7.09 (t, J = 8.0 Hz, 2H), 4.85 - 4.78 (m, 1H), 4.60 (t, J = 8.0 Hz, 2H), 4.39 (t, J = 8.0 Hz, 2H), 4.26 - 4.23 (m, 1H), 3.34 -3.28 (m, 2H), 2.94 - 2.88 (m, 2H) 2.29 (s, 3H); LCMS m/z 452 [M+H]⁺

### Example 76. 5-(5-(3-(1H-imidazol-4-yl)azetidin-1-yl)-1,3,4-oxadiazol-2-yl)-N-(5,6-difluoro-2,3-dihydro-1H-inden-2-yl)pyrimidin-2-amine

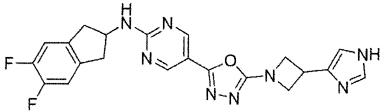

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (30 mg, 0.09 mmol), 4-(azetidin-3-yl)-1H-imidazole, 2,2,2-trifluoroacetate (20 mg, 0.09 mmol), BOP reagent (73 mg, 0.16 mmol), DIPEA (96 µL, 0.54 mmol), and DMF (1 mL), the title compound was obtained as a white solid (3.5 mg, 9%).

¹H NMR (400 MHz, CD₃OD) δ 8.74 (s, 2H), 7.71 (s, 1H), 7.13 -7.08 (m, 3H), 4.86 - 4.83 (m, 1H), 4.54 (t, J = 8.0 Hz, 2H), 4.31 (t, J = 8.0 Hz, 2H), 4.17 - 4.13 (m, 1H), 3.35 -3.30 (m, 2H), 2.94 - 2.89 (m, 2H); LCMS m/z 437 [M+H]⁺

### Example 77. N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyridin-6-amine

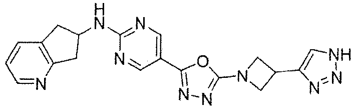

By the same method as in step 3 of Example 7, (4-(1-(5-(2-((6,7-dihydro-5H-cyclopenta[b]pyridin-6-yl)amino)pyrimidin-5-yl)-1,3,4-oxadiazol-2-yl)azetidin-3-yl)-1H-1,2,3-triazol-1-yl)methyl pivalate (42 mg, 0.081 mmol), K₂CO₃ (22.5 mg, 0.16 mmol), and MeOH (1.6 mL) were used to obtain the title compound as a white solid (21 mg, 64%).

¹H NMR (400 MHz, DMSO-d₆) *δ* 8.74 (s, 2H), 8.37 - 8.25 (m, 2H), 7.92 (s, 1H), 7.61 (d, J = 7.3 Hz, 1H), 7.21 - 7.08 (m, 1H), 4.79 - 4.66 (m, 1H), 4.52 (s, 2H), 4.23 (s, 3H), 3.48 - 3.19 (m, 2H), 3.06 - 2.88 (m, 2H); LCMS m/z 403 [M+H]⁺

### Example 78. (E)-3-(2-((5,6-difluoro-2,3-dihydro-1H-inden-2-yl)amino)pyrimidin-5-yl)-1-(3-methyl-3-(1H-1,2,3-triazol-4-yl)azetidin-1-yl)prop-2-en-1-one

### Step 1: (4-(3-methylazetidin-3-yl)-1H-1,2,3-triazol-1-yl)methyl pivalate, 2,2,2-trifluoroacetate

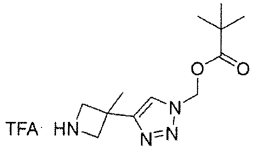

The title compound was prepared by the same method as in Preparation Example 5, except that tert-butyl 3-ethynyl-3-methylazetidine-1-carboxylate was used instead of tert-butyl 3-ethynyl-3-methylpyrrolidine-1-carboxylate.

LCMS m/z 253 [M+H]⁺

### Step 2: (E)-(4-(1-(3-(2-((5,6-difluoro-2,3-dihydro-1H-inden-2-yl)amino)pyrimidin-5-yl)acryloyl)-3-methylazetidin-3-yl)-1H-1,2,3-triazol-1-yl)methyl pivalate

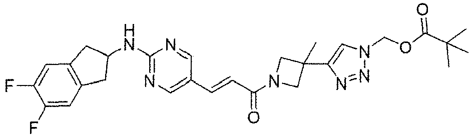

By the same method as in Step 2 of Example 20, using (E)-3-(2-((5,6-difluoro-2,3-dihydro-1H-inden-2-yl)amino)pyrimidin-5-yl)acrylic acid (33 mg, 0.10 mmol), (4-(3-methylazetidin-3-yl)-1H-1,2,3-triazol-1-yl)methyl pivalate, 2,2,2-trifluoroacetate (45 mg, 0.12 mmol), DIPEA (53 uL, 0.31 mmol), HBTU (47 mg, 0.12 mmol), and DMF (1.5 mL), the title compound was obtained as a white solid (51.7 mg, 90.9%).

LCMS m/z 552 [M+H]⁺

### Step 3: (E)-3-(2-((5,6-difluoro-2,3-dihydro-1H-inden-2-yl)amino)pyrimidin-5-yl)-1-(3-methyl-3-(1H-1,2,3-triazol-4-yl)azetidin-1-yl)prop-2-en-1-one

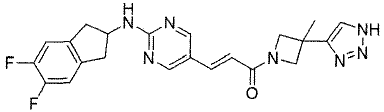

By the same method as in Step 2 of Example 1, using (E)-(4-(1-(3-(2-((5,6-difluoro-2,3-dihydro-1H-inden-2-yl)amino)pyrimidin-5-yl)acryloyl)-3-methylazetidin-3-yl)-1H-1,2,3-triazol-1-yl)methyl pivalate (45.7 mg, 0.083 mmol), K₂CO₃ (23 mg, 0.17 mmol), and MeOH (1.6 mL), the title compound was obtained as a white solid (16.9 mg, 46.6%).

¹H NMR (400 MHz, DMSO-d₆) *δ* 8.67 (s, 2H), 8.01 (d, J = 6.7 Hz, 1H), 7.90 (broad, 1H), 7.36 - 7.23 (m, 3H), 6.67 (d, J = 15.8 Hz, 1H), 4.71 - 4.61 (m, 1H), 4.46 (d, J = 7.6 Hz, 1H), 4.24 (d, J = 8.2 Hz, 1H), 4.14 (d, J = 9.4 Hz, 1H), 3.92 (d, J = 9.2 Hz, 1H), 3.24 (dd, J = 16.1, 7.6 Hz, 2H), 2.87 (dd, J = 16.0, 6.5 Hz, 2H), 1.63 (s, 3H); LCMS m/z 438 [M+H]⁺

### Example 79. 5-(5-(3-(1H-1,2,4-triazol-1-yl)azetidin-1-yl)-1,3,4-oxadiazol-2-yl)-N-(5,6-difluoro-2,3-dihydro-1H-inden-2-yl)pyrimidin-2-amine

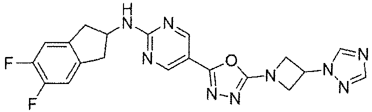

The title compound was obtained as a white solid (6.6 mg, 23.4%) by the same method as in Example 28, using 1-(5-(2-((5,6-difluoro-2,3-dihydro-1H-inden-2-yl)amino)pyrimidin-5-yl)-1,3,4-oxadiazol-2-yl)azetidin-3-yl methanesulfonate (30 mg, 0.065 mmol), 1,2,4-triazole (5.4 mg, 0.078 mmol), NaH (57-63% oil dispersion) (3.1 mg, 0.078 mmol), and DMF (0.6 mL).

¹H NMR (400 MHz, DMSO-d₆) *δ* 8.78 - 8.68 (m, 2H), 8.68 (s, 1H), 8.29 (d, J = 7.7 Hz, 1H), 8.10 (s, 1H), 7.27 (t, J = 9.4 Hz, 2H), 5.63 - 5.55 (m, 1H), 4.73 - 4.66 (m, 1H), 4.63 (t, J = 8.3 Hz, 2H), 4.42 (dd, J = 8.6, 5.6 Hz, 2H), 3.24 (d, J = 8.6 Hz, 2H), 2.88 (dd, J = 15.9, 6.3 Hz, 2H); LCMS m/z 438 [M+H]⁺

### Example 80. N-(5,6-difluoro-2,3-dihydro-1H-inden-2-yl)-5-(5-(3-(3-methyl-1H-1,2,4-triazol-1-yl)azetidin-1-yl)-1,3,4-oxadiazol-2-yl)pyrimidin-2-amine

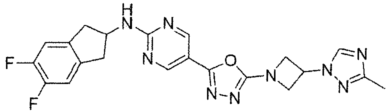

By the same method as in Example 28, using 1-(5-(2-((5,6-difluoro-2,3-dihydro-1H-inden-2-yl)amino)pyrimidin-5-yl)-1,3,4-oxadiazol-2-yl)azetidin-3-yl methanesulfonate (30 mg, 0.065 mmol), 3-methyl-1H-1,2,4-triazole (6.4 mg, 0.078 mmol), NaH (57-63% oil dispersion) (3.1 mg, 0.078 mmol), and DMF (0.6 mL), the title compound was obtained as a white solid (8.6 mg, 29.5%).

¹H NMR (400 MHz, DMSO-d₆) *δ* 8.77 - 8.68 (m, 2H), 8.50 (s, 1H), 8.32 - 8.25 (m, 1H), 7.31 - 7.23 (m, 2H), 5.51 (s, 1H), 4.73 - 4.66 (m, 1H), 4.59 (t, J = 8.9 Hz, 2H), 4.43 - 4.37 (m, 2H), 3.22 (d, J = 7.8 Hz, 2H), 2.88 (dd, J = 15.9, 6.3 Hz, 2H), 2.27 (s, 3H); LCMS m/z 452 [M+H]⁺

### Example 81. N-(5,6-difluoro-2,3-dihydro-1H-inden-2-yl)-5-(5-(3-morpholinoazetidin-1-yl)-1,3,4-oxadiazol-2-yl)pyrimidin-2-amine

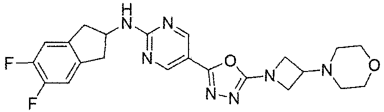

By the same method as in Step 1 of Example 1, using 5-(2-((5,6-difluoro-2,3-dihydro-1H-inden-2-yl)amino)pyrimidin-5-yl)-1,3,4-oxadiazol-2(3H)-one (40 mg, 0.121 mmol), 4-(azetidin-3-yl)morpholine hydrochloride (25.9 mg, 0.145 mmol), DIPEA (64 µL, 0.362 mmol), BOP reagent (64.1 mg, 0.145 mmol), and DMF (0.6 mL), the title compound was obtained as a white solid (45.8 mg, 83.3%).

¹H NMR (400 MHz, DMSO-d₆) *δ* 8.69 (s, 2H), 8.27 (d, J = 6.3 Hz, 1H), 7.27 (t, J = 9.3 Hz, 2H), 4.68 (d, J = 6.5 Hz, 1H), 4.15 (t, J = 7.7 Hz, 2H), 4.03 - 3.94 (m, 2H), 3.58 (s, 3H), 3.29 - 3.21 (m, 3H), 2.87 (dd, J = 16.1, 6.4 Hz, 2H), 2.52 (s, 2H), 2.33 (s, 3H); LCMS m/z 456 [M+H]⁺

### Example 82. (E)-3-(2-((2,3-dihydro-1H-inden-2-yl)amino)pyrimidin-5-yl)-1-(3-methyl-3-(1H-1,2,3-triazol-4-yl)azetidin-1-yl)prop-2-en-1-one

### Step 1: (E)-(4-(1-(3-(2-((2,3-dihydro-1H-inden-2-yl)amino)pyrimidin-5-yl)acryloyl)-3-methylazetidin-3-yl)-1H-1,2,3-triazol-1-yl)methyl pivalate

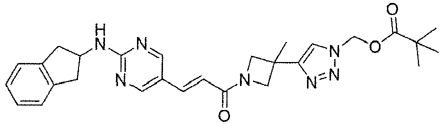

By the same method as in Step 2 of Example 20, using (E)-3-(2-((2,3-dihydro-1H-inden-2-yl)amino)pyrimidin-5-yl)acrylic acid (36.8 mg, 0.13 mmol), (4-(3-methylazetidin-3-yl)-1H-1,2,3-triazol-1-yl)methyl pivalate, 2,2,2-trifluoroacetate (36.8 mg, 0.13 mmol), DIPEA (67 uL, 0.31 mmol), HBTU (60 mg, 0.16 mmol), and DMF (1.5 mL), the title compound was obtained as a pale pink solid (45.7 mg, 67.8%).

LCMS m/z 516 [M+H]⁺

### Step 2: (E)-3-(2-((2,3-dihydro-1H-inden-2-yl)amino)pyrimidin-5-yl)-1-(3-methyl-3-(1H-1,2,3-triazol-4-yl)azetidin-1-yl)prop-2-en-1-one

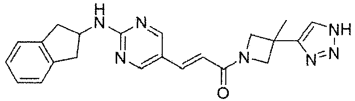

By the same method as in Step 2 of Example 1, using (E)-(4-(1-(3-(2-((2,3-dihydro-1H-inden-2-yl)amino)pyrimidin-5-yl)acryloyl)-3-methylazetidin-3-yl)-1H-1,2,3-triazol-1-yl)methyl pivalate (40.4 mg, 0.078 mmol), K₂CO₃ (63 mg, 0.16 mmol), and MeOH (2 mL), the title compound was obtained as a white solid (13.1 mg, 41.7%).

¹H NMR (400 MHz, DMSO-d₆) *δ* 8.67 (s, 2H), 8.00 (d, J = 6.8 Hz, 1H), 7.33 (d, J = 15.8 Hz, 1H), 7.25 - 7.11 (m, 4H), 6.67 (d, J = 15.8 Hz, 1H), 4.70 - 4.60 (m, 1H), 4.46 (d, J = 8.5 Hz, 1H), 4.25 (d, J = 8.3 Hz, 1H), 4.14 (d, J = 9.4 Hz, 1H), 3.92 (d, J = 9.5 Hz, 1H), 3.25 (dd, J = 15.9, 7.5 Hz, 2H), 2.91 (dd, J = 15.8, 6.9 Hz, 2H), 1.65 (s, 3H); LCMS m/z 402 [M+H]⁺

### Example 83. N-(2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

### Step 1: N-(2,3-dihydro-1H-inden-2-yl)-5-(5-(3-ethynyl-3-fluoroazetidin-1-yl)-1,3,4-oxadiazol-2-yl)pyrimidin-2-amine

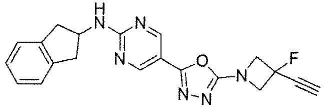

By the same method as in Step 1 of Example 1, using 5-(2-((2,3-dihydro-1H-inden-2-yl)amino)pyrimidin-5-yl)-1,3,4-oxadiazol-2(3H)-one (100 mg, 0.34 mmol), 3-ethynyl-3-fluoroazetidine, 2,2,2-trifluoroacetate (93 mg, 0.47 mmol), BOP reagent (180 mg, 0.41 mmol), DIPEA (423 µL, 2.43 mmol), and DMF (3 mL), the title compound was obtained as a white solid (100 mg, 78%).

¹H NMR (400 MHz, DMSO-d₆) δ 8.73 (s, 2H), 7.20 -7.19 (m, 2H), 7.14 -7.12 (m, 2H), 4.69 - 4.64 (m, 1H), 4.58 - 4.46 (m, 4H), 3.28 -3.22 (m, 2H), 2.93 - 2.88 (m, 2H); LCMS m/z 377 [M+H]⁺

### Step 2: N-(2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

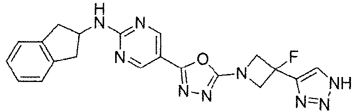

By the same method as step 1 of Preparation example 2, using N-(2,3-dihydro-1H-inden-2-yl)-5-(5-(3-ethynyl-3-fluoroazetidin-1-yl)-1,3,4-oxadiazol-2-yl)pyrimidin-2-amine (55 mg, 0.15 mmol), TMSN₃ (25 uL, 0.19 mmol), CuI (3.3 mg, 0.018 mmol), and DMF (2 mL)/MeOH (0.2 mL), the title compound was obtained as a white solid (10 mg, 16%).

¹H NMR (400 MHz, CD₃OD) δ 8.75 (s, 2H), 8.01 (s, 1H), 7.22 -7.20 (m, 2H), 7.14 - 7.12 (m, 2H), 4.81 - 4.68 (m, 5H), 3.37 -3.30 (m, 2H), 2.97 - 2.90 (m, 2H); LCMS m/z 420 [M+H]⁺

### Example 84. (4-(1-(5-(2-((6,7-dihydro-5H-cyclopenta[c]pyridin-6-yl)amino)pyrimidin-5-yl)-1,3,4-oxadiazol-2-yl)azetidin-3-yl)-1H-1,2,3-triazol-1-yl)methyl pivalate

### Step 1: 5-(2-((6,7-dihydro-5H-cyclopenta[c]pyridin-6-yl)amino)pyrimidin-5-yl)-1,3,4-oxadiazol-2(3H)-one

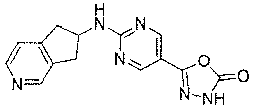

The title compound was prepared by the same method as in Preparation Example 3, except that 6,7-dihydro-5H-cyclopenta[c]pyridin-6-amine dihydrochloride was used instead of 2,3-dihydro-1H-inden-2-amine.

¹H NMR (400 MHz, DMSO-d₆) δ12.14 (s, 1H), 8.67 (d, J = 13.9 Hz, 2H), 8.44 (s, 1H), 8.39 - 8.32 (m, 2H), 7.28 (d, J = 4.7 Hz, 1H), 4.76 - 4.68 (m, 1H), 3.31 - 3.27 (m, 2H), 2.96 (dd, J = 16.5, 6.0 Hz, 2H); LCMS m/z 297 [M+H]⁺

### Step 2: (4-(1-(5-(2-((6,7-dihydro-5H-cyclopenta[c]pyridin-6-yl)amino)pyrimidin-5-yl)-1,3,4-oxadiazol-2-yl)azetidin-3-yl)-1H-1,2,3-triazol-1-yl)methyl pivalate

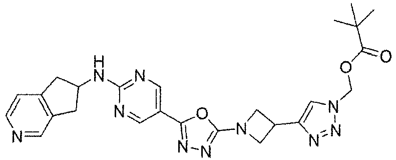

By the same method as in Step 1 of Example 1, using 5-(2-((6,7-dihydro-5H-cyclopenta[c]pyridin-6-yl)amino)pyrimidin-5-yl)-1,3,4-oxadiazol-2(3H)-one (10 mg, 0.034 mmol), (4-(azetidin-3-yl)-1H-1,2,3-triazol-1-yl)methyl pivalate, 2,2,2-trifluoroacetate (11 mg, 0.034 mmol), DIPEA (22 *µ* L, 0.17 mmol), BOP reagent (18 mg, 0.041 mmol), and DMF (1 mL), the title compound was obtained as an off-white solid (4.0 mg, 23%).

¹H NMR (400 MHz, DMSO-d₆) δ8.74 (d, *J* = 17.8 Hz, 2H), 8.44 (s, 1H), 8.36 - 8.28 (m, 3H), 7.28 (s, 1H), 6.30 (s, 2H), 4.75 - 4.69 (m, 1H), 4.54 - 4.49 (m, 2H), 4.28 - 4.21 (m, 3H), 3.30 - 3.27 (m, 2H), 2.96 (dd, *J* = 16.2, 6.6 Hz, 2H), 1.13 (s, 9H); LCMS m/z 517 [M+H]⁺

### Example 85. N-(5-(5-(3-(1H-1,2,3-triazol-4-yl)azetidin-1-yl)-1,3,4-oxadiazol-2-yl)pyrimidin-2-yl)-6,7-dihydro-SH-cyclopenta[c]pyridin-6-amine

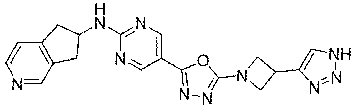

By the same method as in Step 2 of Example 1, using (4-(1-(5-(2-((6,7-dihydro-5H-cyclopenta[c]pyridin-6-yl)amino)pyrimidin-5-yl)-1,3,4-oxadiazol-2-yl)azetidin-3-yl)-1H-1,2,3-triazol-1-yl)methyl pivalate (3.7 mg, 0.0076 mmol), K₂CO₃ (2 mg, 0.014 mmol), and MeOH (1 mL), the title compound was obtained as an off-white solid (2.9 mg, 99%).

¹H NMR (400 MHz, DMSO-d₆) δ8.76 (s, 2H), 8.44 (s, 1H), 8.33 (dd, *J* = 14.9, 5.9 Hz, 2H), 7.93 (s, 1H), 7.29 (d, *J* = 4.6 Hz, 1H), 4.76 - 4.67 (m, 1H), 4.53 (s, 2H), 4.23 (s, 3H), 3.30 (s, 2H), 2.96 (dd, *J* = 16.6, 5.9 Hz, 2H); LCMS m/z 403 [M+H]⁺

### Example 86. (4-(1-(5-(2-((benzo[d][1,3]dioxol-5-ylmethyl)amino)pyrimidin-5-yl)-1,3,4-oxadiazol-2-yl)azetidin-3-yl)-1H-1,2,3-triazol-1-yl)methyl pivalate

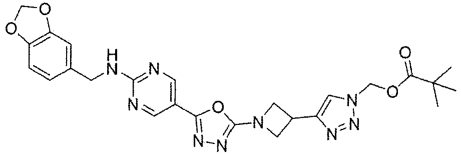

By the same method as in Step 1 of Example 1, using 5-(2-((benzo[d][1,3]dioxol-5-ylmethyl)amino)pyrimidin-5-yl)-1,3,4-oxadiazol-2(3H)-one (25 mg, 0.080 mmol), (4-(azetidin-3-yl)-1H-1,2,3-triazol-1-yl)methyl pivalate, 2,2,2-trifluoroacetate (27 mg, 0.080 mmol), DIPEA (68 µL, 0.40 mmol), BOP reagent (43 mg, 0.096 mmol), and DMF (1 mL), the title compound was obtained as a yellow solid (24 mg, 56%).

¹H NMR (400 MHz, DMSO-d₆) δ8.70 (s, 2H), 8.38 (t, *J* = 6.2 Hz, 1H), 8.30 (s, 1H), 6.88 (s, 1H), 6.85 - 6.78 (m, 2H), 6.30 (s, 2H), 5.97 (s, 2H), 4.52 - 4.45 (m, 4H), 4.26 - 4.21 (m, 3H), 1.13 (s, 9H); LCMS m/z 534 [M+H]⁺

### Example 87. 5-(5-(3-(1H-1,2,3-triazol-4-yl)azetidin-1-yl)-1,3,4-oxadiazol-2-yl)-N-(benzo[d][1,3]dioxol-5-ylmethyl)pyrimidin-2-amine

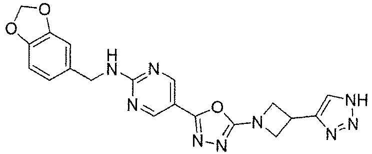

By the same method as in Step 2 of Example 1, using (4-(1-(5-(2-((benzo[d][1,3]dioxol-5-ylmethyl)amino)pyrimidin-5-yl)-1,3,4-oxadiazol-2-yl)azetidin-3-yl)-1H-1,2,3-triazol-1-yl)methyl pivalate (19 mg, 0.035 mmol), K₂CO₃ (10 mg, 0.071 mmol), and MeOH (1 mL), the title compound was obtained as a white solid (8.7 mg, 59%).

¹H NMR (400 MHz, DMSO-d₆) δ8.70 (s, 2H), 8.39 (t, *J* = 6.2 Hz, 1H), 7.92 (s, 1H), 6.88 (s, 1H), 6.85 - 6.78 (m, 2H), 5.97 (s, 2H), 4.54 - 4.50 (m, 2H), 4.47 (d, *J* = 6.3 Hz, 2H), 4.22 (s, 3H); LCMS m/z 420 [M+H]⁺

### Example 88. 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-N-methylpyrimidin-2-amine

### Step 1: ethyl 2-((5,6-difluoro-2,3-dihydro-1H-inden-2-yl)amino)pyrimidine-5-carboxylate

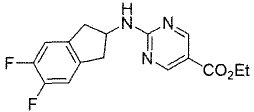

The title compound was prepared by the same method as in Step 1 of Preparation Example 3, except that 5,6-difluoro-2,3-dihydro-1H-inden-2-amine was used instead of 2,3-dihydro-1H-inden-2-amine.

LCMS m/z 320 [M+H]⁺

### Step 2: ethyl 2-((5,6-difluoro-2,3-dihydro-1H-inden-2-yl)(methyl)amino)pyrimidine-5-carboxylate

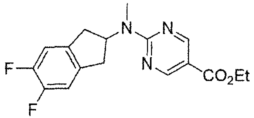

Ethyl 2-((5,6-difluoro-2,3-dihydro-1H-inden-2-yl)amino)pyrimidine-5-carboxylate (50 mg, 0.157 mmol) was dissolved in DMF (1.6 mL) and NaH (57-63% oil dispersion) (9.4 mg, 0.235 mmol) was added at 0 °C and stirred for 30 min. Then, MeI (15 µL, 0.235 mmol) was added at the same temperature and stirred at room temperature for 1 h. DW was added to the reaction mixture, extracted with EtOAc, dried over Na₂SO₄, filtered and concentrated. The obtained residue was purified by column chromatography (EtoAc/Hex) to obtain the title compound as a white solid (22 mg, 42.1%).

¹H NMR (400 MHz, DMSO-d₆) *δ* 8.79 (s, 2H), 7.30 (t, *J* = 9.3 Hz, 2H), 5.78 (dt, *J* = 15.3, 7.7 Hz, 1H), 4.26 (q, *J* = 7.1 Hz, 2H), 3.14 (dd, *J* = 16.4, 8.3 Hz, 2H), 3.02 (q, *J* = 7.0 Hz, 5H), 1.28 (t, *J* = 7.1 Hz, 3H); LCMS m/z 334 [M+H]⁺

### Step 3: 5-(2-((5,6-difluoro-2,3-dihydro-1H-inden-2-yl)(methyl)amino)pyrimidin-5-yl)-1,3,4-oxadiazol-2(3H)-one

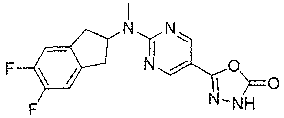

The title compound was prepared by the same method as steps 2 and 3 of Preparation Example 3, except that ethyl 2-((5,6-difluoro-2,3-dihydro-1H-inden-2-yl)(methyl)amino)pyrimidine-5-carboxylate was used instead of ethyl 2-((2,3-dihydro-1H-inden-2-yl)amino)pyrimidine-5-carboxylate.

LCMS m/z 346 [M+H]⁺

### Step 4: 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-N-methylpyrimidin-2-amine

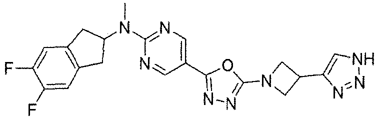

By the same method as in Step 1 of Example 1, using 5-(2-((5,6-difluoro-2,3-dihydro-1H-inden-2-yl)(methyl)amino)pyrimidin-5-yl)-1,3,4-oxadiazol-2(3H)-one (6.5 mg, 0.019 mmol), 4-(azetidin-3-yl)-1H-1,2,3-triazole, 2,2,2-trifluoroacetate (5 mg, 0.023 mmol), DIPEA (9.6 µL, 0.056 mmol), BOP reagent (10 mg, 0.023 mmol), and DMF (0.09 mL), the title compound was obtained as a yellow solid (3.1 mg, 36.5%).

¹H NMR (400 MHz, DMSO-d₆) *δ* 8.77 (s, 2H), 7.31 (t, J = 9.3 Hz, 2H), 5.78 - 5.72 (m, 1H), 4.51 (s, 2H), 4.21 (s, 2H), 3.28 (s, 2H), 3.14 (dd, J = 16.3, 9.1 Hz, 2H), 3.05 (d, J = 7.0 Hz, 2H), 3.00 (s, 3H); LCMS m/z 452 [M+H]⁺

### Example 89. N-(5-bromo-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

### Step 1: N-(5-bromo-2,3-dihydro-1H-inden-2-yl)-5-(5-(3-ethynyl-3-fluoroazetidin-1-yl)-1,3,4-oxadiazol-2-yl)pyrimidin-2-amine

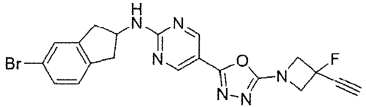

By the same method as in Step 1 of Example 1, using 5-(2-((5-bromo-2,3-dihydro-1H-inden-2-yl)amino)pyrimidin-5-yl)-1,3,4-oxadiazol-2(3H)-one (55 mg, 0.15 mmol), 3-ethynyl-3-fluoroazetidine, 2,2,2-trifluoroacetate (44 mg, 0.22 mmol), BOP reagent (79 mg, 0.18 mmol), DIPEA (186 µL, 0.17 mmol), and DMF (2 mL), the title compound was obtained as a white solid (50 mg, 73%).

¹H NMR (400 MHz, DMSO-d₆) δ 8.73 (s, 2H), 7.41 (s, 1H), 7.32 (d, J = 8.0 Hz, 1H), 7.18 (d , J = 8.0 Hz, 1H), 4.69 - 4.64 (m, 1H), 4.58 - 4.46 (m, 4H), 3.26 -3.19 (m, 2H), 2.89 - 2.87 (m, 2H); LCMS m/z 455, 457 [M+H]⁺

### Step 2: N-(5-bromo-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine

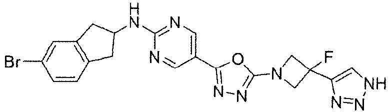

By the same method as in step 1 of Preparation example 2, N-(5-bromo-2,3-dihydro-1H-inden-2-yl)-5-(5-(3-ethynyl-3-fluoroazetidin-1-yl)-1,3,4-oxadiazol-2-yl)pyrimidin-2-amine (90 mg, 0.2 mmol), TMSN₃ (36 uL, 0.28 mmol), CuI (4.8 mg, 0.018 mmol), and DMF (3 mL)/MeOH (0.3 mL) were used to obtain the title compound as a white solid (1.3 mg, 1.3%).

¹H NMR (400 MHz, CD₃OD) δ 8.76 (s, 2H), 8.04 (s, 1H), 7.38 (s, 1H), 7.22 -7.20 (m, 2H), 7.30 (d, J = 8.0 Hz, 1H), 7.15 (d, J = 8.0 Hz, 1H), 4.88 - 4.61 (m, 5H), 3.38 -3.30 (m, 2H), 2.93 - 2.89 (m, 2H); LCMS m/z 498, 500 [M+H]⁺

### Example 90. 1-(3-(1H-1,2,3-triazol-4-yl)azetidin-1-yl)-4-(5-((5,6-difluoro-2,3-dihydro-1H-inden-2-yl)amino)-1,3,4-oxadiazol-2-yl)butan-1-one

### Step 1: methyl 4-(5-((5,6-difluoro-2,3-dihydro-1H-inden-2-yl)amino)-1,3,4-oxadiazol-2-yl)butanoate

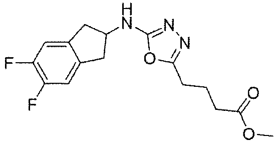

5,6-Difluoro-2,3-dihydro-1H-inden-2-amine (32.8 mg, 0.120 mmol), Methyl 4-(5-oxo-4,5-dihydro-1,3,4-oxadiazol-2-yl)butanoate (18.6 mg, 0.100 mmol), BOP reagent (53 mg, 0.120 mmol), and DIPEA (51 µL, 0.300 mmol) were dissolved in DMF (0.5 mL) and stirred at room temperature for 4 h. DW was added to the reaction mixture, extracted with EtOAc, dried over Na₂SO₄, filtered, and concentrated. The obtained residue was purified by column chromatography (MeOH/DCM) to give the title compound as a brown solid (8.5 mg, 25.2%).

¹H NMR (400 MHz, CD₃OD) *δ* 7.11 (t, *J* = 9.0 Hz, 2H), 4.45 - 4.35 (m, 1H), 3.65 (d, *J* = 2.2 Hz, 3H), 2.91 (dd, *J* = 16.1, 5.2 Hz, 2H), 2.75 (t, *J* = 7.3 Hz, 2H), 2.44 (t, *J* = 7.2 Hz, 2H), 2.06 - 1.85 (m, 4H). LCMS m/z 338[M+H]⁺

### Step 2: 4-(5-((5,6-difluoro-2,3-dihydro-1H-inden-2-yl)amino)-1,3,4-oxadiazol-2-yl)butanoic acid

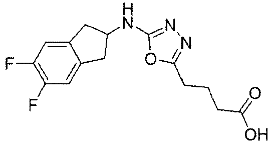

Methyl 4-(5-((5,6-difluoro-2,3-dihydro-1H-inden-2-yl)amino)-1,3,4-oxadiazol-2-yl)butanoate (3.0 mg, 0.025 mmol) was dissolved in MeOH/THF (0.45 mL/0.05 mL), and a solution of LiOH (3.0 mg, 0.126 mmol) in distilled water (0.05 mL) was added. The mixture was stirred at room temperature for 1 h. After concentrating the reaction mixture, distilled water was added, and the mixture was acidified to pH 1-2 with 3 N HCl. The mixture was extracted with EtOAc, washed with distilled water, dried over Na₂SO₄, filtered, and concentrated. The obtained residue was purified by column chromatography (MeOH/DCM) to give the title compound as a white solid (8.0 mg, 98.2%).

¹H NMR (400 MHz, CD₃OD) *δ* 7.11 (t, *J* = 9.0 Hz, 2H), 4.40 (dd, *J* = 11.8, 6.0 Hz, 1H), 2.91 (dd, *J =* 16.1, 5.0 Hz, 2H), 2.76 (t, *J* = 7.3 Hz, 2H), 2.37 (t, *J* = 7.1 Hz, 2H), 2.10 - 1.80 (m, 4H); LCMS m/z 324[M+H]⁺

### Step 3: 1-(3-(1H-1,2,3-triazol-4-yl)azetidin-1-yl)-4-(5-((5,6-difluoro-2,3-dihydro-1H-inden-2-yl)amino)-1,3,4-oxadiazol-2-yl)butan-1-one

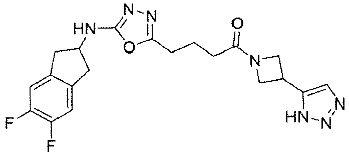

By the same method as in Step 3 of Example 9, using 4-(5-((5,6-difluoro-2,3-dihydro-1H-inden-2-yl)amino)-1,3,4-oxadiazol-2-yl)butanoic acid (8 mg, 0.025 mmol), 4-(azetidin-3-yl)-1H-1,2,3-triazole dihydrochloride (4.8 mg, 0.030 mmol), DIPEA (13 µL, 0.074 mmol), HBTU (11.3 mg, 0.030 mmol), and DMF (0.49 mL), the title compound was obtained as a colorless oil (1.0 mg, 9.4%).

¹H NMR (400 MHz, CD₃OD) *δ* 7.28 (s, 1H), 7.11 (t, *J* = 9.2 Hz, 2H), 4.43 - 4.29 (m, 3H), 4.04 (d, *J* = 6.1 Hz, 2H), 3.76 - 3.68 (m, 1H), 3.22 (d, *J* = 7.6 Hz, 2H), 2.92 (dd, *J* = 16.0, 4.8 Hz, 2H), 2.77 (t, *J* = 7.2 Hz, 2H), 2.28 (t, *J* = 7.1 Hz, 2H), 2.05 - 1.99 (m, 2H); LCMS m/z 430[M+H]⁺

### Example 91. 5-(5-(3-(1H-1,2,3-triazol-4-yl)azetidin-1-yl)-1,3,4-oxadiazol-2-yl)-N-(5-chloro-2,3-dihydro-1H-inden-2-yl)pyrimidin-2-amine

### Step 1: (4-(1-(5-(2-((5-chloro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate

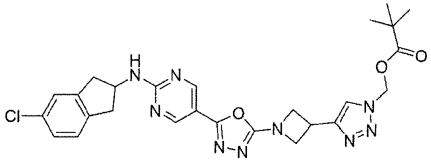

By the same method as in Step 1 of Example 1, using 5-(2-((5-chloro-2,3-dihydro-1H-inden-2-yl)amino)pyrimidin-5-yl)-1,3,4-oxadiazol-2(3H)-one (35 mg, 0.11 mmol), (4-(azetidin-3-yl)-1H-1,2,3-triazol-1-yl)methyl pivalate, 2,2,2-trifluoroacetate (60.5 mg, crude), DIPEA (92 uL, 0.54 mmol), BOP reagent (56.3 mg, 0.13 mmol), and DMF (2 mL), the title compound was obtained as an off-white solid (54 mg, 93%).

¹H NMR (400 MHz, DMSO-d₆) δ 8.73 (d, J = 15.6 Hz, 2H), 8.35 - 8.23 (m, 2H), 7.29 (s, 1H), 7.22 (dd, J = 18.9, 8.0 Hz, 2H), 6.30 (s, 2H), 4.69 (dd, J = 14.1, 6.9 Hz, 1H), 4.52 (s, 2H), 4.31 - 4.17 (m, 3H), 3.30 - 3.21 (m, 2H), 2.98 - 2.85 (m, 2H), 1.12 (s, 9H); LCMS m/z 550 [M+H]⁺

### Step 2: 5-(5-(3-(1H-1,2,3-triazol-4-yl)azetidin-1-yl)-1,3,4-oxadiazol-2-yl)-N-(5-chloro-2,3-dihydro-1H-inden-2-yl)pyrimidin-2-amine

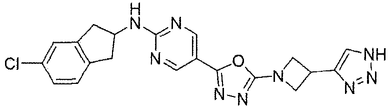

By the same method as in Step 2 of Example 1, using (4-(1-(5-(2-((5-chloro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate (50 mg, 0.09 mmol), K₂CO₃ (25 mg, 0.18 mmol), and MeOH (1.82 mL), the title compound was obtained as a white solid (25 mg, 63%).

¹H NMR (400 MHz, DMSO-d₆) δ8.73 (d, J = 15.2 Hz, 2H), 8.29 (d, J = 6.3 Hz, 1H), 7.92 (s, 1H), 7.29 (s, 1H), 7.22 (dd, J = 19.7, 7.9 Hz, 2H), 4.69 (dd, J = 13.9, 7.0 Hz, 1H), 4.52 (s, 2H), 4.23 (s, 3H), 3.30 - 3.22 (m, 2H), 2.99 - 2.84 (m, 2H); LCMS m/z 436 [M+H]⁺

### Example 92. 5-(5-(3-(1H-1,2,3-triazol-4-yl)pyrrolidin-1-yl)-1,3,4-oxadiazol-2-yl)-N-(4-chlorophenethyl)pyrimidin-2-amine

### Step 1: 5-(2-((4-chlorophenethyl)amino)pyrimidin-5-yl)-1,3,4-oxadiazol-2(3H)-one

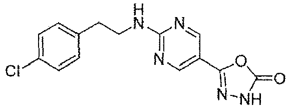

The title compound was prepared by the same method as in Preparation Example 3, except that 2-(4-chlorophenyl)ethylamine was used instead of 2,3-dihydro-1H-inden-2-amine.

¹H NMR (400 MHz, DMSO-d₆) δ12.15 (bs, 1H), 8.60 (d, *J* = 15.6 Hz, 2H), 8.04 (t, *J* = 5.7 Hz, 1H), 7.28 (dd, *J =* 32.0, 8.3 Hz, 4H), 3.53 (dd, *J* = 13.4, 6.7 Hz, 2H), 2.83 (t, *J* = 7.1 Hz, 2H); LCMS m/z 318 [M+H]⁺

### Step 2: 5-(5-(3-(1H-1,2,3-triazol-4-yl)pyrrolidin-1-yl)-1,3,4-oxadiazol-2-yl)-N-(4-chlorophenethyl)pyrimidin-2-amine

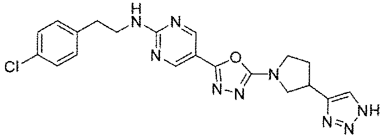

By the same method as in Step 1 of Example 1, using 5-(2-((4-chlorophenethyl)amino)pyrimidin-5-yl)-1,3,4-oxadiazol-2(3H)-one (60 mg, 0.189 mmol), 4-(pyrrolidin-3-yl)-1H-1,2,3-triazole, 2,2,2-trifluoroacetate, (53.3 mg, 0.227 mmol), DIPEA (161 uL, 0.567 mmol), BOP reagent (100 mg, 0.227 mmol), and DMF (3.8 mL), the title compound was obtained as a white solid (49.5 mg, 60%).

¹H NMR (400 MHz, DMSO-d₆) *δ* 8.68 (d, J = 13.4 Hz, 2H), 7.94 (t, J = 5.7 Hz, 1H), 7.39 (s, 1H), 7.33 - 7.23 (m, 4H), 3.94 - 3.87 (m, 1H), 3.66 (d, J = 8.3 Hz, 2H), 3.55 (dd, J = 16.4, 8.8 Hz, 6H), 2.83 (t, J = 7.4 Hz, 2H); LCMS m/z 438 [M+H]⁺

### Example 93. 5-(5-(3-(1H-1,2,3-triazol-4-yl)azetidin-1-yl)-1,3,4-oxadiazol-2-yl)-N-(4-chlorophenethyl)pyrimidin-2-amine

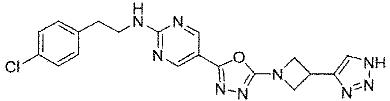

By the same method as in Step 1 of Example 1, using 5-(2-((4-chlorophenethyl)amino)pyrimidin-5-yl)-1,3,4-oxadiazol-2(3H)-one (60 mg, 0.189 mmol), 4-(azetidin-3-yl)-1H-1,2,3-triazole dihydrochloride, (44.6 mg, 0.227 mmol), DIPEA (161 uL, 0.567 mmol), BOP reagent (100 mg, 0.227 mmol), and DMF (3.8 mL), the title compound was obtained as a yellow solid (43 mg, 53.7%).

¹H NMR (400 MHz, DMSO-d₆) *δ* 8.67 (d, J = 16.6 Hz, 2H), 7.98 (t, J = 5.7 Hz, 1H), 7.90 (s, 1H), 7.28 (dd, J = 30.5, 8.5 Hz, 4H), 4.50 (s, 2H), 4.20 (s, 3H), 3.53 (dd, J = 13.0, 6.5 Hz, 2H), 2.84 (dd, J = 14.4, 6.9 Hz, 2H); LCMS m/z 424 [M+H]⁺

### Test Example 1. Evaluation of the inhibitory activity against human Autotaxin protein

### (1) Experimental method

Solutions of each compound synthesized (80 uM, 100% dimethyl sulfoxide) were sequentially diluted 5-fold with dimethyl sulfoxide to prepare 6 concentrations. Each concentration of the compound was diluted 2-fold with 1x test buffer (50 mM Tris-Cl (pH 8.0), 5 mM KCl, 1 mM CaCl₂, 1 mM MgCl₂, 140 mM NaCl, deionized water, 1 mg/mL BSA), and 1 uL (1.25% dimethyl sulfoxide) of this diluted mixture was dispensed into each well of a 96-well clear round-bottom plate. 9 uL of 1x test buffer was added, and 20 uL of 240 nM human autotaxin protein (buffer: 50 mM Tris-HCl, pH 8.0, with 150 mM sodium chloride and 20% glycerol) was added. 10 uL of sonicated 360 uM 18:1 LysoPC (diluted with 1x assay buffer) was added. The reaction was performed for 2 hours in a shaking incubator at 37 °C, and a secondary reaction mixture (choline assay kit, KA1662) (65 uL of 1x assay buffer: 1 uL of choline oxidase: 1 uL of dye probe) was prepared. 60 uL of the secondary reaction mixture was added to the plate where the reaction was performed, and incubated for 30 minutes in a shaker. The absorbance value was measured at a wavelength of 570 nm using a SpectraMax iD3 microplate reader. The percentage inhibition activity value (% inhibition) was calculated by the formula: (1 - absorbance value _{test group} / absorbance value _{control group}) X 100.

### (2) Results

The activity inhibition rates measured as above are shown in Table 1 below.

**[Table 1]**

| Example | Inhibition (%, at 500nM) | Example | Inhibition (%, at 500nM) |
|---|---|---|---|
| 1 | 98 | 48 | 39.2 |
| 2 | 3 | 49 | 11 |
| 3 | 100 | 50 | 14 |
| 4 | 1 | 51 | 91.8 |
| 5 | 7.6 | 52 | 21.9 |
| 6 | 30.4 | 53 | 24.7 |
| 7 | 17 | 54 | 23.4 |
| 8 | 49.5 | 55 | 17.6 |
| 9 | 94.2 | 56 | 85.2 |
| 10 | 18.6 | 57 | 8.6 |
| 11 | 41.6 | 58 | 1 |
| 12 | 4.1 | 59 | 49.5 |
| 13 | 96.8 | 60 | 1 |
| 14 | 23.3 | 61 | 1 |
| 15 | 9.5 | 62 | 1 |
| 16 | 88.1 | 63 | 1 |
| 17 | 39.5 | 64 | 100 |
| 18 | 95.3 | 65 | 100 |
| 19 | 99.7 | 66 | 35.8 |
| 20 | 92.4 | 67 | 74.7 |
| 21 | 72.5 | 68 | 17.6 |
| 22 | 3.6 | 69 | 95.4 |
| 23 | 91.6 | 70 | 9.6 |
| 24 | 1 | 71 | 95.5 |
| 25 | 1.8 | 72 | 3.1 |
| 26 | 1 | 73 | 1.2 |
| 27 | 12.1 | 74 | 7.1 |
| 28 | 10.9 | 75 | 84.6 |
| 29 | 9.2 | 76 | 64 |
| 30 | 96.9 | 77 | 43.7 |
| 31 | 96.8 | 78 | 99.1 |
| 32 | 21.9 | 79 | 24.7 |
| 33 | 29.1 | 80 | 10.4 |
| 34 | 88.4 | 81 | 7 |
| 35 | 1.3 | 82 | 98 |
| 36 | 97.4 | 83 | 81.6 |
| 37 | 97.9 | 84 | 1.1 |
| 38 | 55 | 85 | 19.8 |
| 39 | 98 | 86 | 2.8 |
| 40 | 98.6 | 87 | 18 |
| 41 | 70.6 | 88 | 10.5 |
| 42 | 97.8 | 89 | 84.9 |
| 43 | 35.4 | 90 | 17.9 |
| 44 | 51.5 | 91 | 97.1 |
| 45 | 43 | 92 | 85.5 |
| 46 | 27.1 | 93 | 89.3 |
| 47 | 7.5 | - | - |

All of the synthesized compounds of Examples 1 to 93 exhibited excellent inhibitory activity against human Autotaxin protein.

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. A heterocyclic compound represented by the following Formula 1, a hydrate thereof, a solvate thereof or a pharmaceutically acceptable salt thereof, wherein: 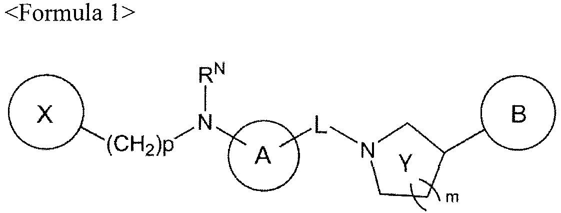
X is aryl; a fused bicyclic ring in which an aryl ring is fused with a non-aromatic cycloalkyl ring; a fused bicyclic ring in which an aryl ring is fused with a non-aromatic heterocyclic ring having 1 to 3 of O; a fused bicyclic ring in which a heteroaryl ring having 1 to 3 of N is fused with a non-aromatic cycloalkyl ring, wherein X is substituted with one or more independent R^{x} or not,
R^{x} is C₁₋₄ alkoxy or halogen,
p is an integer from 0 to 2,
R^{N} is hydrogen or C₁₋₄ alkyl,
A is 5- or 6-membered heteroaryl ring having 1 to 3 heteroatom(s) selected from the group consisting of N and O, wherein A is substituted with R^{a} or not,
R^{a} is C₁₋₄ alkyl,
L is -(CH₂)₁₋₅CO-; -(CHCH)₁₋₂CO-; or a 5-membered aromatic or non-aromatic heterocycle ring having 1 to 3 heteroatoms selected from the group consisting of N and O,
Y ring is substituted or unsubstituted with R^{y},
R^{y} is C₁₋₄ alkyl or halogen,
m is 0 or 1,
B is cyano; OH; COOH; CH₂COOH; sulfonyl; sulfonate(-O-SO₂-); or a 5- to 6-membered aromatic or non-aromatic heterocycle ring having 1 to 4 heteroatoms selected from the group consisting of N and O, wherein B is substituted or unsubstituted with R^{b} or oxo(O),
wherein R^{b} is C₁₋₄ alkyl, C₁₋₄ haloalkyl, amino, or C₁₋₄ alkyl pivalate.

2. The heterocyclic compound, a hydrate thereof, a solvate thereof or a pharmaceutically acceptable salt thereof of Claim 1, **characterized in that** X is selected from the group consisting of phenyl, dihydroindenyl, benzodioxolyl, dihydro-cyclopentapyrazinyl, and dihydro-cyclopentapyridinyl.

3. The heterocyclic compound, a hydrate thereof, a solvate thereof or a pharmaceutically acceptable salt thereof of Claim 1, **characterized in that** R^{x} is selected from the group consisting of methoxy, F, Cl, and Br.

4. The heterocyclic compound, a hydrate thereof, a solvate thereof or a pharmaceutically acceptable salt thereof of Claim 1, **characterized in that** R^{N} is hydrogen or methyl.

5. The heterocyclic compound, a hydrate thereof, a solvate thereof or a pharmaceutically acceptable salt thereof of Claim 1, **characterized in that** A is selected from the group consisting of pyridine, pyrimidine, pyrazine, and oxadiazole.

6. The heterocyclic compound, a hydrate thereof, a solvate thereof or a pharmaceutically acceptable salt thereof of Claim 1, **characterized in that** R^{a} is methyl.

7. The heterocyclic compound, a hydrate thereof, a solvate thereof or a pharmaceutically acceptable salt thereof of Claim 1, **characterized in that** L is selected from the group consisting of -(CH₂)₂CO-, -(CH₂)₃CO-, -(CH)₂CO-, dihydroisoxazolyl, isoxazolyl, and oxadiazolyl.

8. The heterocyclic compound, a hydrate thereof, a solvate thereof or a pharmaceutically acceptable salt thereof of Claim 1, **characterized in that** R^{y} is methyl or F.

9. The heterocyclic compound, a hydrate thereof, a solvate thereof or a pharmaceutically acceptable salt thereof of Claim 1, **characterized in that** B is selected from the group consisting of OH, cyano, carboxyl, carboxymethyl, sulfonyl, sulfonate, imidazolyl, triazolyl, tetrazolyl, oxadiazolyl, oxadiazolone, and morpholino.

10. The heterocyclic compound, a hydrate thereof, a solvate thereof or a pharmaceutically acceptable salt thereof of Claim 1, **characterized in that** R^{b} is selected from the group consisting of methyl, difluoromethyl, trifluoromethyl, amino, and methylpivalate.

11. The heterocyclic compound, a hydrate thereof, a solvate thereof or a pharmaceutically acceptable salt thereof of Claim 1, **characterized in that** the heterocyclic compound is selected from the group consisting of:
[1] 5-(5-(3-(1H-1,2,3-triazol-4-yl)pyrrolidin-1-yl)-1,3,4-oxadiazol-2-yl)-N-(2,3-dihydro-1H-inden-2-yl)pyrimidin-2-amine,
[2] (4-(1-(5-(2-((5,6-difluoro-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate,
[3] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrimidin-2-amine,
[4] (4-(1-(5-(2-((6,7-dihydro-5H-cyclopenta[b]pyridin-6-yl)amino)pyrimidin-5-yl)-1,3,4-oxadiazol-2-yl)pyrrolidin-3-yl)-1H-1,2,3-triazol-1-yl)methyl pivalate,
[5] (4-(1-(5-(2-((benzo[d][1,3]dioxol-5-ylmethyl)amino)pyrimidin-5-yl)-1,3,4-oxadiazol-2-yl)pyrrolidin-3-yl)-1H-1,2,3-triazol-1-yl)methyl pivalate,
[6] 5-(5-(3-(1H-1,2,3-triazol-4-yl)pyrrolidin-1-yl)-1,3,4-oxadiazol-2-yl)-N-(benzo[d][1,3]dioxol-5-ylmethyl)pyrimidin-2-amine,
[7] N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyrazin-6-amine,
[8] N-(5-(5-(3-(1H-1,2,3-triazol-4-yl)pyrrolidin-1-yl)-1,3,4-oxadiazol-2-yl)pyrimidin-2-yl)-6,7-dihydro-5H-cyclopenta[b]pyridin-6-amine,
[9] (E)-1-(3-(1H-1,2,3-triazol-4-yl)pyrrolidin-1-yl)-3-(2-((5,6-difluoro-2,3-dihydro-1H-inden-2-yl)amino)pyrimidin-5-yl)prop-2-en-1-one,
[10] 1-(3-(1H-1,2,3-triazol-4-yl)pyrrolidin-1-yl)-3-(2-((5,6-difluoro-2,3-dihydro-1H-inden-2-yl)amino)pyrimidin-5-yl)propan-1-one,
[11] 5-(5-(3-(1H-1,2,3-triazol-4-yl)pyrrolidin-1-yl)-1,3,4-oxadiazol-2-yl)-N-(3,5-dichlorophenethyl)pyrimidin-2-amine,
[12] (4-(1-(5-(2-((5-methoxy-2,3-dihydro-1H-inden-2-yl)amino)pyrimidin-5-yl)-1,3,4-oxadiazol-2-yl)pyrrolidin-3-yl)-1H-1,2,3-triazol-1-yl)methyl pivalate,
[13] 5-(5-(3-(1H-1,2,3-triazol-4-yl)pyrrolidin-1-yl)-1,3,4-oxadiazol-2-yl)-N-(5-methoxy-2,3-dihydro-1H-inden-2-yl)pyrimidin-2-amine,
[14] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)pyrazin-2-amine,
[15] methyl 1-(5-(2-((5,6-difluoro-2,3-dihydro-1H-inden-2-yl)amino)pyrimidin-5-yl)-1,3,4-oxadiazol-2-yl)pyrrolidine-3-carboxylate,
[16] 5-(5-(3-(1H-1,2,3-triazol-4-yl)pyrrolidin-1-yl)-1,3,4-oxadiazol-2-yl)-N-(5,6-difluoro-2,3-dihydro-1H-inden-2-yl)-4-methylpyrimidin-2-amine,
[17] 5-(3-(3-(1H-1,2,3-triazol-4-yl)pyrrolidin-1-yl)-4,5-dihydroisoxazol-5-yl)-N-(5,6-difluoro-2,3-dihydro-1H-inden-2-yl)pyrimidin-2-amine,
[18] 5-(3-(3-(1H-1,2,3-triazol-4-yl)pyrrolidin-1-yl)isoxazol-5-yl)-N-(5,6-difluoro-2,3-dihydro-1H-inden-2-yl)pyrimidin-2-amine,
[19] 5-(5-(3-(1H-1,2,3-triazol-4-yl)pyrrolidin-1-yl)-1,3,4-oxadiazol-2-yl)-N-(5-bromo-2,3-dihydro-1H-inden-2-yl)pyrimidin-2-amine,
[20] (E)-1-(3-(1H-1,2,3-triazol-4-yl)pyrrolidin-1-yl)-3-(2-((2,3-dihydro-1H-inden-2-yl)amino)pyrimidin-5-yl)prop-2-en-1-one,
[21] 5-(5-(3-(1H-1,2,3-triazol-4-yl)pyrrolidin-1-yl)-1,3,4-oxadiazol-2-yl)-N-(3,5-difluorobenzyl)pyrimidin-2-amine,
[22] 1-(5-(2-((5,6-difluoro-2,3-dihydro-1H-inden-2-yl)amino)pyrimidin-5-yl)-1,3,4-oxadiazol-2-yl)pyrrolidine-3-carboxylic acid,
[23] 5-(1-(5-(2-((5,6-difluoro-2,3-dihydro-1H-inden-2-yl)amino)pyrimidin-5-yl)-1,3,4-oxadiazol-2-yl)pyrrolidin-3-yl)-1,3,4-oxadiazol-2(3H)-one,
[24] methyl 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetate,
[25] 2-(1-(5-(2-((5,6-difluoro-2,3-dihydro-1H-inden-2-yl)amino)pyrimidin-5-yl)-1,3,4-oxadiazol-2-yl)pyrrolidin-3-yl)acetic acid,
[26] 1-(5-(2-((5,6-difluoro-2,3-dihydro-1H-inden-2-yl)amino)pyrimidin-5-yl)-1,3,4-oxadiazol-2-yl)pyrrolidin-3-ol,
[27] 1-(5-(2-((5,6-difluoro-2,3-dihydro-1H-inden-2-yl)amino)pyrimidin-5-yl)-1,3,4-oxadiazol-2-yl)pyrrolidin-3-yl methanesulfonate,
[28] 5-(5-(3-(1H-1,2,4-triazol-1-yl)pyrrolidin-1-yl)-1,3,4-oxadiazol-2-yl)-N-(5,6-difluoro-2,3-dihydro-1H-inden-2-yl)pyrimidin-2-amine,
[29] N-(5,6-difluoro-2,3-dihydro-1H-inden-2-yl)-5-(5-(3-(3-methyl-1H-1,2,4-triazol-1-yl)pyrrolidin-1-yl)-1,3,4-oxadiazol-2-yl)pyrimidin-2-amine,
[30] N-(5,6-difluoro-2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine,
[31] N-(2,3-dihydro-1H-inden-2-yl)-5-(5-(3-methyl-3-(1H-1,2,3-triazol-4-yl)pyrrolidin-1-yl)-1,3,4-oxadiazol-2-yl)pyrimidin-2-amine,
[32] N-(3,5-dichlorophenethyl)-5-(5-(3-methyl-3-(1H-1,2,3-triazol-4-yl)pyrrolidin-1-yl)-1,3,4-oxadiazol-2-yl)pyrimidin-2-amine,
[33] N-(3,5-difluorobenzyl)-5-(5-(3-methyl-3-(1H-1,2,3-triazol-4-yl)pyrrolidin-1-yl)-1,3,4-oxadiazol-2-yl)pyrimidin-2-amine,
[34] (E)-3-(2-((2,3-dihydro-1H-inden-2-yl)amino)pyrimidin-5-yl)-1-(3-methyl-3-(1H-1,2,3-triazol-4-yl)pyrrolidin-1-yl)prop-2-en-1-one,
[35] N-(5,6-difluoro-2,3-dihydro-1H-inden-2-yl)-5-(5-(3-(1-methyl-1H-1,2,3-triazol-4-yl)pyrrolidin-1-yl)-1,3,4-oxadiazol-2-yl)pyrimidin-2-amine,
[36] 5-(5-(3-(1H-1,2,3-triazol-4-yl)pyrrolidin-1-yl)-1,3,4-oxadiazol-2-yl)-N-(5-fluoro-2,3-dihydro-1H-inden-2-yl)pyrimidin-2-amine,
[37] 5-(5-(3-(1H-1,2,3-triazol-4-yl)pyrrolidin-1-yl)-1,3,4-oxadiazol-2-yl)-N-(5-chloro-2,3-dihydro-1H-inden-2-yl)pyrimidin-2-amine,
[38] 5-(5-(3-(1H-imidazol-4-yl)pyrrolidin-1-yl)-1,3,4-oxadiazol-2-yl)-N-(5,6-difluoro-2,3-dihydro-1H-inden-2-yl)pyrimidin-2-amine,
[39] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrimidin-2-amine,
[40] (E)-1-(3-(1H-1,2,3-triazol-4-yl)azetidin-1-yl)-3-(2-((5,6-difluoro-2,3-dihydro-1H-inden-2-yl)amino)pyrimidin-5-yl)prop-2-en-1-one,
[41] N-(5,6-difluoro-2,3-dihydro-1H-inden-2-yl)-5-(5-(3-(1-methyl-1H-1,2,3-triazol-5-yl)azetidin-1-yl)-1,3,4-oxadiazol-2-yl)pyrimidin-2-amine,
[42] 5-(5-(3-(1H-1,2,3-triazol-4-yl)azetidin-1-yl)-1,3,4-oxadiazol-2-yl)-N-(2,3-dihydro-1H-inden-2-yl)pyrimidin-2-amine,
[43] 5-(3-(3-(1H-1,2,3-triazol-4-yl)azetidin-1-yl)-4,5-dihydroisoxazol-5-yl)-N-(5,6-difluoro-2,3-dihydro-1H-inden-2-yl)pyrimidin-2-amine,
[44] 1-(3-(1H-1,2,3-triazol-4-yl)azetidin-1-yl)-3-(2-((5,6-difluoro-2,3-dihydro-1H-inden-2-yl)amino)pyrimidin-5-yl)propan-1-one,
[45] 5-(3-(3-(1H-1,2,3-triazol-4-yl)azetidin-1-yl)isoxazol-5-yl)-N-(5,6-difluoro-2,3-dihydro-1H-inden-2-yl)pyrimidin-2-amine,
[46] 1-(5-(2-((5,6-difluoro-2,3-dihydro-1H-inden-2-yl)amino)pyrimidin-5-yl)-1,3,4-oxadiazol-2-yl)azetidine-3-sulfonamide,
[47] methyl 1-(5-(2-((5,6-difluoro-2,3-dihydro-1H-inden-2-yl)amino)pyrimidin-5-yl)-1,3,4-oxadiazol-2-yl)azetidine-3-carboxylate,
[48] 5-(5-(3-(1H-1,2,3-triazol-4-yl)azetidin-1-yl)-1,3,4-oxadiazol-2-yl)-N-(3,5-difluorobenzyl)pyrimidin-2-amine,
[49] 1-(5-(2-((5,6-difluoro-2,3-dihydro-1H-inden-2-yl)amino)pyrimidin-5-yl)-1,3,4-oxadiazol-2-yl)azetidin-3-ol,
[50] 1-(5-(2-((5,6-difluoro-2,3-dihydro-1H-inden-2-yl)amino)pyrimidin-5-yl)-1,3,4-oxadiazol-2-yl)azetidine-3-carboxylic acid,
[51] N-(5,6-difluoro-2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine,
[52] methyl 2-(1-(5-(2-((5,6-difluoro-2,3-dihydro-1H-inden-2-yl)amino)pyrimidin-5-yl)-1,3,4-oxadiazol-2-yl)azetidin-3-yl)acetate,
[53] 2-(1-(5-(2-((5,6-difluoro-2,3-dihydro-1H-inden-2-yl)amino)pyrimidin-5-yl)-1,3,4-oxadiazol-2-yl)azetidin-3-yl)acetic acid,
[54] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyridin-2-amine,
[55] 1-(5-(2-((5,6-difluoro-2,3-dihydro-1H-inden-2-yl)amino)pyrimidin-5-yl)-1,3,4-oxadiazol-2-yl)azetidin-3-yl methanesulfonate,
[56] N-(5,6-difluoro-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[57] 1-(5-(2-((5,6-difluoro-2,3-dihydro-1H-inden-2-yl)amino)pyrimidin-5-yl)-1,3,4-oxadiazol-2-yl)azetidine-3-carbonitrile,
[58] N-(5,6-difluoro-2,3-dihydro-1H-inden-2-yl)-5-(5-(3-(5-(trifluoromethyl)-1,3,4-oxadiazol-2-yl)azetidin-1-yl)-1,3,4-oxadiazol-2-yl)pyrimidin-2-amine,
[59] 5-(5-(3-(1H-1,2,3-triazol-4-yl)azetidin-1-yl)-1,3,4-oxadiazol-2-yl)-N-(3,5-dichlorophenethyl)pyrimidin-2-amine,
[60] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)pyrazin-2-amine,
[61] N-(5,6-difluoro-2,3-dihydro-1H-inden-2-yl)-5-(5-(3-(5-(difluoromethyl)-1,3,4-oxadiazol-2-yl)azetidin-1-yl)-1,3,4-oxadiazol-2-yl)pyrimidin-2-amine,
[62] (4-(1-(5-(2-((5-bromo-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[63] (4-(1-(5-(2-((5-fluoro-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[64] 5-(5-(3-(1H-1,2,3-triazol-4-yl)azetidin-1-yl)-1,3,4-oxadiazol-2-yl)-N-(5-bromo-2,3-dihydro-1H-inden-2-yl)pyrimidin-2-amine,
[65] 5-(5-(3-(1H-1,2,3-triazol-4-yl)azetidin-1-yl)-1,3,4-oxadiazol-2-yl)-N-(5-fluoro-2,3-dihydro-1H-inden-2-yl)pyrimidin-2-amine,
[66] 5-(1-(5-(2-((5,6-difluoro-2,3-dihydro-1H-inden-2-yl)amino)pyrimidin-5-yl)-1,3,4-oxadiazol-2-yl)azetidin-3-yl)-1,3,4-oxadiazol-2(3H)-one,
[67] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-4-methylpyrimidin-2-amine,
[68] 5-(5-(3-(1H-tetrazol-5-yl)azetidin-1-yl)-1,3,4-oxadiazol-2-yl)-N-(5,6-difluoro-2,3-dihydro-1H-inden-2-yl)pyrimidin-2-amine,
[69] N-(2,3-dihydro-1H-inden-2-yl)-5-(5-(3-methyl-3-(1H-1,2,3-triazol-4-yl)azetidin-1-yl)-1,3,4-oxadiazol-2-yl)pyrimidin-2-amine,
[70] (4-(1-(5-(2-((5-methoxy-2,3-dihydro-1H-inden-2-yl)amino)pyrimidin-5-yl)-1,3,4-oxadiazol-2-yl)azetidin-3-yl)-1H-1,2,3-triazol-1-yl)methyl pivalate,
[71] 5-(5-(3-(1H-1,2,3-triazol-4-yl)azetidin-1-yl)-1,3,4-oxadiazol-2-yl)-N-(5-methoxy-2,3-dihydro-1H-inden-2-yl)pyrimidin-2-amine,
[72] (4-(1-(5-(2-((6,7-dihydro-5H-cyclopenta[b]pyrazin-6-yl)amino)pyrimidin-5-yl)-1,3,4-oxadiazol-2-yl)azetidin-3-yl)-1H-1,2,3-triazol-1-yl)methyl pivalate,
[73] (4-(1-(5-(2-((6,7-dihydro-5H-cyclopenta[b]pyridin-6-yl)amino)pyrimidin-5-yl)-1,3,4-oxadiazol-2-yl)azetidin-3-yl)-1H-1,2,3-triazol-1-yl)methyl pivalate,
[74] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyrazin-6-amine,
[75] N-(5,6-difluoro-2,3-dihydro-1H-inden-2-yl)-5-(5-(3-(5-methyl-1H-1,2,3-triazol-4-yl)azetidin-1-yl)-1,3,4-oxadiazol-2-yl)pyrimidin-2-amine,
[76] 5-(5-(3-(1H-imidazol-4-yl)azetidin-1-yl)-1,3,4-oxadiazol-2-yl)-N-(5,6-difluoro-2,3-dihydro-1H-inden-2-yl)pyrimidin-2-amine,
[77] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[b]pyridin-6-amine,
[78] (E)-3-(2-((5,6-difluoro-2,3-dihydro-1H-inden-2-yl)amino)pyrimidin-5-yl)-1-(3-methyl-3-(1H-1,2,3-triazol-4-yl)azetidin-1-yl)prop-2-en-1-one,
[79] 5-(5-(3-(1H-1,2,4-triazol-1-yl)azetidin-1-yl)-1,3,4-oxadiazol-2-yl)-N-(5,6-difluoro-2,3-dihydro-1H-inden-2-yl)pyrimidin-2-amine,
[80] N-(5,6-difluoro-2,3-dihydro-1H-inden-2-yl)-5-(5-(3-(3-methyl-1H-1,2,4-triazol-1-yl)azetidin-1-yl)-1,3,4-oxadiazol-2-yl)pyrimidin-2-amine,
[81] N-(5,6-difluoro-2,3-dihydro-1H-inden-2-yl)-5-(5-(3-morpholinoazetidin-1-yl)-1,3,4-oxadiazol-2-yl)pyrimidin-2-amine,
[82] (E)-3-(2-((2,3-dihydro-1H-inden-2-yl)amino)pyrimidin-5-yl)-1-(3-methyl-3-(1H-1,2,3-triazol-4-yl)azetidin-1-yl)prop-2-en-1-one,
[83] N-(2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[84] (4-(1-(5-(2-((6,7-dihydro-5H-cyclopenta[c]pyridin-6-yl)amino)pyrimidin-5-yl)-1,3,4-oxadiazol-2-yl)azetidin-3-yl)-1H-1,2,3-triazol-1-yl)methyl pivalate,
[85] N-(5-(5-(3-(1H-1,2,3-triazol-4-yl)azetidin-1-yl)-1,3,4-oxadiazol-2-yl)pyrimidin-2-yl)-6,7-dihydro-5H-cyclopenta[c]pyridin-6-amine,
[86] (4-(1-(5-(2-((benzo[d][1,3]dioxol-5-ylmethyl)amino)pyrimidin-5-yl)-1,3,4-oxadiazol-2-yl)azetidin-3-yl)-1H-1,2,3-triazol-1-yl)methyl pivalate,
[87] 5-(5-(3-(1H-1,2,3-triazol-4-yl)azetidin-1-yl)-1,3,4-oxadiazol-2-yl)-N-(benzo[d][1,3]dioxol-5-ylmethyl)pyrimidin-2-amine,
[88] 5-(5-(3-(1H-1,2,3-triazol-4-yl)azetidin-1-yl)-1,3,4-oxadiazol-2-yl)-N-(5,6-difluoro-2,3-dihydro-1H-inden-2-yl)-N-methylpyrimidin-2-amine,
[89] N-(5-bromo-2,3-dihydro-1H-inden-2-yl)-5-(5-(3-fluoro-3-(1H-1,2,3-triazol-4-yl)azetidin-1-yl)-1,3,4-oxadiazol-2-yl)pyrimidin-2-amine,
[90] 1-(3-(1H-1,2,3-triazol-4-yl)azetidin-1-yl)-4-(5-((5,6-difluoro-2,3-dihydro-1H-inden-2-yl)amino)-1,3,4-oxadiazol-2-yl)butan-1-one,
[91] 5-(5-(3-(1H-1,2,3-triazol-4-yl)azetidin-1-yl)-1,3,4-oxadiazol-2-yl)-N-(5-chloro-2,3-dihydro-1H-inden-2-yl)pyrimidin-2-amine,
[92] 5-(5-(3-(1H-1,2,3-triazol-4-yl)pyrrolidin-1-yl)-1,3,4-oxadiazol-2-yl)-N-(4-chlorophenethyl)pyrimidin-2-amine, and
[93] 5-(5-(3-(1H-1,2,3-triazol-4-yl)azetidin-1-yl)-1,3,4-oxadiazol-2-yl)-N-(4-chlorophenethyl)pyrimidin-2-amine.

12. A pharmaceutical composition for preventing or treating a disease associated with autotaxin activity, comprising the heterocyclic compound, a hydrate thereof, a solvate thereof or a pharmaceutically acceptable salt thereof of any one of Claims 1-11 as an active ingredient.

13. The pharmaceutical composition of Claim 12, **characterized in that** the disease associated with autotaxin activity is selected from the group consisting of fibrotic diseases, inflammatory diseases, autoimmune diseases, respiratory diseases, cardiovascular diseases, metabolic diseases, cancer and cancer metastasis, ocular diseases, cholestatic form and other forms of chronic pruritus, and acute or chronic organ transplant rejection.

14. A pharmaceutical composition comprising the heterocyclic compound, a hydrate thereof, a solvate thereof or a pharmaceutically acceptable salt thereof of any one of Claims 1-11, **characterized in that** is selected from the group consisting of a fibrotic disease selected from idiopathic pulmonary fibrosis (IPF), interstitial lung disease, liver fibrosis, liver cirrhosis, non-alcoholic steatohepatitis, radiation-induced fibrosis, renal fibrosis, cutaneous fibrosis, glomerulosclerosis, myocardial and vascular fibrosis; an inflammatory disease selected from rheumatoid arthritis, osteoarthritis, atopic dermatitis, inflammatory bowel disease, inflammatory airway disease, chronic obstructive pulmonary disease (COPD) and asthma; an autoimmune disease selected from multiple sclerosis and scleroderma; a respiratory disease selected from asbestos-induced pulmonary fibrosis and acute respiratory distress syndrome (ARDS); a cardiovascular disease selected from arteriosclerosis, myocardial infarction, arterial and pulmonary hypertension, cardiac arrhythmia, stroke and other vascular damage; a metabolic disease selected from obesity and diabetes; cancer and cancer metastasis selected from breast cancer, ovarian cancer, lung cancer, prostate cancer, mesothelioma, glioma, liver carcinoma, gastrointestinal cancer, pancreatic cancer, and its progression and metastatic invasion; an ocular disease selected from proliferative and non-proliferative (diabetic) retinopathy, dry and wet age-related macular degeneration (AMD), macular edema, central artery/venous occlusion, traumatic injury, and glaucoma; cholestatic form and other forms of chronic pruritus; and acute or chronic organ transplant rejection.

15. A pharmaceutical composition comprising the heterocyclic compound, a hydrate thereof, a solvate thereof or a pharmaceutically acceptable salt thereof of any one of Claims 1-11, and a pharmaceutically acceptable additive.
